# EUROPEAN PATENT SPECIFICATION

(11) **EP 2 382 235 B1**
(45) Date of publication and mention of the grant of the patent: **24.02.2016**
(21) Application number: 09801699.1
(22) Date of filing: 18.12.2009
(51) Int. Cl.: C07K 14/47, A61K 38/17

(54) **NOVEL CAVIIDAE ALLERGEN AND USES THEREOF**
NEUES CAVIIDAE-ALLERGEN UND ANWENDUNGEN DAVON
NOUVEL ALLERGÈNE DE CAVIIDAE ET SES UTILISATIONS

(30) Priority: 19.12.2008 EP 08022207
(43) Date of publication of application: 02.11.2011
(73) Proprietor: Hilger, Christiane, 7246 Helmsange (LU); Hentges, François, 8009 Strassen (LU)
(72) Inventor: Hilger, Christiane, 7246 Helmsange (LU); Hentges, François, 8009 Strassen (LU)
(74) Representative: Watterson, Peer Marten John
(86) International application number: PCT/EP2009/067604
(87) International publication number: WO 2010/070136

(56) References cited:
- FAHLBUSCH B ET AL: "Further characterization of IgE-binding antigens from guinea pig hair as new members of the lipocalin family." ALLERGY (COPENHAGEN), vol. 58, no. 7, July 2003 (2003-07), pages 629-634, XP002580949 ISSN: 0105-4538 cited in the application
- FAHLBUSCH B ET AL: "Purification and partial characterization of the major allergen, Cav p 1, from guinea pig Cavia porcellus" ALLERGY (COPENHAGEN), vol. 57, no. 5, May 2002 (2002-05), pages 417-422, XP002580950 ISSN: 0105-4538 cited in the application
- HENZGEN M ET AL: "Rodents as source of allergens /// Nagetiere als Allergenquelle" ALLERGOLOGIE, DUSTRI VERLAG, MUENCHEN-DEISENHOFEN, DE, vol. 31, no. 9, 1 September 2008 (2008-09-01), pages 383-388, XP009136947 ISSN: 0344-5062

## Description

The present invention relates to the provision of a nucleic acid molecule encoding a Caviidae allergen comprising a polynucleotide selected from the group consisting of (a) a polynucleotide sequence as shown in SEQ ID NO: 19 *(Cavia porcellus* allergen 1 a); (b) a polynucleotide sequence encoding a polypeptide as shown in SEQ ID NO: 20 *(Cavia porcellus* allergen 1a); (c) a polynucleotide sequence which has at least 80% identity to the polynucleotides as defined in (a) or (b) encoding a Caviidae allergen; (d) a polynucleotide sequence encoding a polypeptide which has at least 85% identity to the polypeptide as shown in SEQ ID NO:20 *(Cavia porcellus* allergen 1a); and (e) a polynucleotide sequence being degenerate as a result of the generic code to the polynucleotide sequence as defined in any one of (a) to (d). The present invention also provides for a method for producing the polypeptides (*Cavia porcellus* allergen 1 a) encoded by said polynucleotides. Moreover, the use of such allergens in a medical setting (e.g. in form of a pharmaceutical and/or diagnostic composition), an *in vitro* method for determining the allergenicity to Caviidae as well as an method for identifying potential antagonists or agonists of the polypeptides are also provided in the present invention.

One in four children and young adults in western countries produces IgE antibodies to common environmental antigens and is prone to develop allergic diseases (Aberg (1995), Clin Exp Allergy 25, 815-9; Sears (1997), Lancet 350, 1015-1020; Beasly (1998), Lancet 351, 1225-1232). This trend to develop allergies is gaining worldwide relevance, affecting also "third-world -nations" hitherto supposed to be hardly allergy-prone. Besides allergens of plant, invertebrate or mould origin, allergens of vertebrate origin are major triggers of type I allergic disorders. From a respiratory point of view allergens linked to animal dander are of major importance. Up to 40% of atopic individuals have IgE to mammalian proteins (Ledford (1994), J Allergy Clin Immunol 94, 327-334. Animals are important sources of inhalant allergens not only at home but also in public places and in working environments. One third to one half of households have mammalian pets. Cats and dogs, the most popular pets, are the most common cause of allergies to animals, followed by guinea-pigs and rabbits. But also hamsters, rats, and mice, or ferrets are kept as pets and can elicit allergies. Rodents are also frequent allergy triggers in laboratory workers (Bush (1998), J Allergy Clin Immunol 102, 99-108). Occupational allergy to cow- and horse- derived allergens affects mainly individuals, who are in direct contact with these animals: farmers, horse-owners, veterinarians.

Guinea pigs have a high sensitisation power provoking symptoms in 31% of exposed laboratory workers in a Japanese study (Aoyama (1992) Br J Ind Med 49, 41-47). In western countries most persons who become sensitized to guinea-pigs become sensitized in their house-hold.

Conventional animal allergen extracts have been prepared from pelt, hair, dander, saliva, serum, urine. Although much work has been done on the biochemical and immunochemical characterisation of these native allergen extracts, they are difficult to standardize in respect of the content of major and minor allergens and also the non-allergic components. The quality and composition of the extracts are variable (Focke (2008), Clin Exp Allergy; 38(8):1400-8).

Commercial animal allergen extracts are generally prepared from hair or dander. They contain multiple components, allergenic as well as non-allergenic molecules. They are difficult to standardize in respect to the content of major and minor allergens.

Prick test solutions may be contaminated with products from other sources and give false positive results (Van der Veen (1996), J Allergy Clin Immunol.;98(6 Pt 1):1028-34). Today, standardization of allergen extracts for diagnosis and immunotherapy is based on biological standardization like skin tests and competitive IgE binding assays. In addition, allergen manufacturers express potencies of their extracts in company-specific units. This does not allow product comparison between companies (Eichler (1988) Allergy; 43:458-63). For the diagnosis of allergy to guinea pig, no purified native or recombinant molecules are available yet.

Allergic reaction to guinea pig has been recognized as a problem in domestic settings and work environments for many years. In the study of Fahlbusch (2002), Allergy 57, 417-422, one allergen of guinea pig, Cav p 1, was characterized and the N-terminal amino-acid sequence was determined.

Fahlbusch (2003), Allergy 58, 629-634 isolated and characterized a further 17 kDa allergen from guinea pig hair which was termed Cav p 2. Furthermore, Fahlbusch eludicated allergenic as well as cross-reactive properties of the guinea pig allergens Cav p 1 and Cav p 2.

It was found in Fahlbusch (2003; loc. cit.) that Cav p 2 and Cav p 1 contained several isoforms with pi values ranging from 3.6 to 5.3. Moreover, Fahlbusch (2003; loc. cit.) disclosed cross-reactive IgE epitopes on the allergens Cav p 2 and Cav p 1. However, guinea pig allergens are not characterized enough to allow standardization of guinea pig extracts in terms of allergen content. There are no tools available to quantify guinea pig allergens. Changing sources of primary material as well as different methods for extract preparation might result in allergen preparations with variable allergen content. However extracts with low amount of major allergens or lack of minor allergens will loose sensitivity for diagnosis of guinea pig allergic patients. The isolation of pure molecules from guinea pig hair is an alternative to using whole extracts. However this is laborious and time-consuming.

The technical problem underlying the present invention is the provision of means and methods for diagnostic assessment and therapeutic intervention of allergenicity to Caviidae.

The technical problem is solved by provision of the embodiments characterized in the claims.

In a first aspect, the present invention relates to a nucleic acid molecule encoding a Caviidae allergen comprising a polynucleotide selected from the group consisting of (a) a polynucleotide sequence as shown in SEQ ID NO: 19 *(Cavia porcellus* allergen 1 a); (b) a polynucleotide sequence encoding a polypeptide as shown in SEQ ID NO: 20 *(Cavia porcellus* allergen 1a); (c) a polynucleotide sequence which has at least 80% identity to the polynucleotides as defined in (a) or (b) encoding a Caviidae allergen; (d) a polynucleotide sequence encoding a polypeptide which has at least 85% identity to the polypeptide as shown in SEQ ID NO:20 *(Cavia porcellus* allergen 1 a); ; and (e) a polynucleotide sequence being degenerate as a result of the generic code to the polynucleotide sequence as defined in any one of (a) to (d).

The present application provides for novel antigens/allergens from guinea pig Caviidae. Within the context the present invention, it has been surprisingly found that purified native or recombinant guinea pig allergens/antigens as provided herein are particularly advantageous in the diagnostic assessment and therapeutic intervention of allergenicity, in particular to Caviidae, as compared to corresponding conventional extracts, like guinea pig extracts. Purified native guinea pig allergens/antigens as used herein and provided in this invention refer preferably to (a) polypeptide(s) that is/are isolated from a cellular context (like the cellular context of a host cell expression in a recombinant setting). In context of the appended examples it was in particular found that the submaxillary gland as well as the harderian gland of guinea pig contain allergens, like Cav p 2, Cav p 3, Cav p6 and *Cavia porcellus* allergen 1a. The novel allergens/antigens are defined herein above and also described herein in the sequences provided herein. Fragments are in particular useful in the preparation of e.g. antibody molecules directed against the novel and inventive guinea pig allergens/antigens, like in the preparation of monoclonal or polyclonal antibodies. In this context in particular fragments of at least 5 amino acids are useful. A further advantageous use of fragments of the polypeptides is in the use in a medical setting, for example in immunization and sensitivisation approaches of (allergenic) patients. Obviously, also full length polypeptides can be used for diagnostic or medical approaches.

In context of feature (c) herein above, it is envisaged that polynucleotides encoding for example a N-terminus of the corresponding *Cavia porcellus* allergen 1 a show an identity of at least 95%, 96%, 97% or 98% on DNA level. On the amino acid level an identity of at least 85% of said N-terminal fragment is within the scope of the present invention. Such (a) N-terminal fragment(s) comprise(s) at least 5 amino acids and up to 15 amino acids or up to 18 amino acids with at least an identity of 95%, 96%, 97% or 98%.

A particular preferred embodiment of the present invention is the provision of a diagnostic tool in the determination of the immunoglobulin level/titer (like the IgE-level/IgE-titer) in a subject prone to suffer from an allergy or already suffering from an allergy. In particular, the determination of corresponding IgE levels/titers in a subject suffering from or prone to suffer from an allergy to Caviidae is envisaged in context of the present invention. However, also other Ig may be determined, like IgG₁ or IgG₄. Corresponding working examples have been provided herein below and are also illustrated in the appended examples. In particular, the novel guinea pig antigens/allergens provided herein and described in this invention constitute a major advantage in diagnostics settings over the "guinea pig extracts" that are currently used in particular diagnostic settings. The present invention provides for highly specific diagnostic tools and specific compounds to be used in medical settings, like immunization approaches and sensitivisation approaches of human patients. However, the present invention is not limited to medical intervention or diagnosis in humans but also to other species like, for example, horses and other animals. As documented in the appended examples, a particular use of the polypeptides of the present invention (or fragments thereof) is their use in diagnostic approaches which allow the discrimination between a specific sensitization to a specific animal species (for examples Caviidae) versus a cross-sensitization to other animals or animal parts. With the present invention it is now possible to produce isolated highly purified proteins/allergens/antigens which can be obtained by for example recombinant methods known in the art. Furthermore, such highly purified or isolated proteins can be obtained routinely from animal extracts, like guinea pig extracts by the use of now available specific antibodies directed against the herein disclosed novel and inventive guinea pig allergens/antigens. Protein purification methods from extract, either recombinant extracts like recombinant extracts from host cells or corresponding cell cultures or from guinea pig extracts are well known in the art and can easily be applied by the person skilled in the art. Within the means and methods provided herein is also a diagnostic method which allows the specific determination of IgE cross-reactivity, for example in biological samples from allergy patients. Such a biological sample may, for example be a blood sample, like a serum sample or a full blood sample. However, also skin samples, mucosal samples and the like may be used.

The gist of the present invention lies in the provision of novel Caviidae allergens. Moreover, it was surprisingly found that these novel allergens are particularly useful for diagnostic assessment and therapeutic intervention of allergenicity to Caviidae, in particular guinea pigs. Furthermore, the use of combinations of said allergens is particularly advantageous in the diagnostic assessment and therapeutic intervention of allergenicity to Caviidae, and accordingly envisaged in the context of the present invention. The identification of Cav p 3, Cav p 6 and *Cavia porcellus* allergen 1 a was not obvious as these proteins have similar MW and pi and are difficult to separate by chromatography. In addition the amount of Cav p 3 and Cav p 6 protein found on hair is low. Proteins are more easily detected in the different gland extracts. The choice of the tissue was essential for isolation of Cav p 3, Cav p 6 and *Cavia porcellus* allergen 1a. Yet, obtaining the allergens/antigens of the present invention is not a routine course of action which comes within the scope of the customary practice for the following reason: N-terminal amino acid sequence information allows the skilled person the design of degenerate primers which may be used in combination with an oligo(dT) primer to amplify the target mRNA by reverse transcription and polymerase chain reaction. However, this approach failed for Cavia porcellus allergens as provided herein and in particular for the Cavia porcellus allergen 1a. Thus, a new strategy had to be developed.This strategy is provided herein and is illustrated in the appended Examples, in particular Examples 8 and 9.

In one embodiment of the present invention a complementary strand of the nucleic acid molecule encoding a Caviidae allergen as defined herein is also envisaged.

The meaning of the terms "nucleic acid molecule", "polynucleotide sequence" and "polypeptide" is well known in the art, and the terms are, if not otherwise defined herein, used accordingly in the context of the present invention. For example, "polynucleotide sequence" as used herein refers to all forms of naturally occurring or recombinantly generated types of nucleic acids and/or nucleotide sequences as well as to chemically synthesized nucleic acids/nucleotide sequences. This term also encompasses nucleic acid analogs and nucleic acid derivatives such as, e. g., locked DNA, PNA, oligonucleotide thiophosphates and substituted ribo-oligonucleotides. Furthermore, the term "polynucleotide sequence" also refers to any molecule that comprises nucleotides or nucleotide analogs.

Preferably, the term "polynucleotide sequence" refers to a nucleic acid molecule, i.e. deoxyribonucleic acid (DNA) and/ or ribonucleic acid (RNA). The "polynucleotide sequence" in the context of the present invention may be made by synthetic chemical methodology known to one of ordinary skill in the art, or by the use of recombinant technology, or may be isolated from natural sources, or by a combination thereof. The DNA and RNA may optionally comprise unnatural nucleotides and may be single or double stranded. "Polynucleotide sequence" also refers to sense and anti-sense DNA and RNA, that is, a polynucleotide sequence which is complementary to a specific sequence of nucleotides in DNA and/or RNA. Furthermore, the term "polynucleotide sequence" may refer to DNA or RNA or hybrids thereof or any modification thereof that is known in the state of the art (see, e.g., US 5525711, US 4711955, US 5792608 or EP 302175 for examples of modifications). The polynucleotide sequence may be single- or double-stranded, linear or circular, natural or synthetic, and without any size limitation. For instance, the polynucleotide sequence may be genomic DNA, cDNA, mRNA, antisense RNA, ribozymal or a DNA encoding such RNAs or chimeroplasts (Gamper, Nucleic Acids Research, 2000, 28, 4332 - 4339). Said polynucleotide sequence may be in the form of a plasmid or of viral DNA or RNA. "Polynucleotide sequence" may also refer to (an) oligonucleotide(s), wherein any of the state of the art modifications such as phosphothioates or peptide nucleic acids (PNA) are included.

The term "allergen" is well known in the art. In context of the present invention, it refers in particular to a nonparasitic antigen capable of stimulating a type-I hypersensitivity reaction in individuals with atopic syndrome. In individuals with atopic syndrome, non-parasitic antigens stimulate inappropriate IgE production, leading to type I hypersensitivity. Sensitivities vary from one individual, preferably human to another and it is possible to be allergic to an extraordinary range of substances. The type of allergens of the present invention comprises (an) allergen(s) derived /obtained from the class of mammalia, preferably rodentia, more preferably Caviidae, more preferably Caviinae, and even more preferably *Cavia.* Non-limiting examples of species belonging to the genus Cavia are most preferably *Cavia porcellus* (guinea pig(s)), *Cavia anolaimae, Cavia guianae, Cavia aperea, Cavia fulgida, Cavia intermedia, Cavia magna, Cavia nana* or *Cavia tschudii. Cavia porcellus* is, in context of the present invention, a preferred species of the family Caviidae. In a preferred embodiment, the allergen is a polypeptide as described and defined herein below, in particular a polypeptide as shown in SEQ ID NO: 20 *(Cavia porcellus* allergen 1a). Accordingly, a polypeptide is disclosed herein, wherein said polypeptide is selected from a group consisting of (a) a polypeptide comprising an amino acid sequence encoded by a nucleic acid molecule having the nucleic acid sequence as depicted in SEQ ID NO: 19 *(Cavia porcellus* allergen 1a); (b) a polypeptide having an amino acid sequence as depicted in SEQ ID NO: 20 *(Cavia porcellus* allergen 1a); (c) a polypeptide comprising an amino acid encoded by a nucleic acid molecule encoding a peptide having an amino acid sequence as depicted in SEQ ID NO: 20 (*Cavia porcellus* allergen 1a).; (d) a polypeptide having at least 85 % identity to the polypeptide of any one of (a) to (c) and encoding a Caviidae allergen.

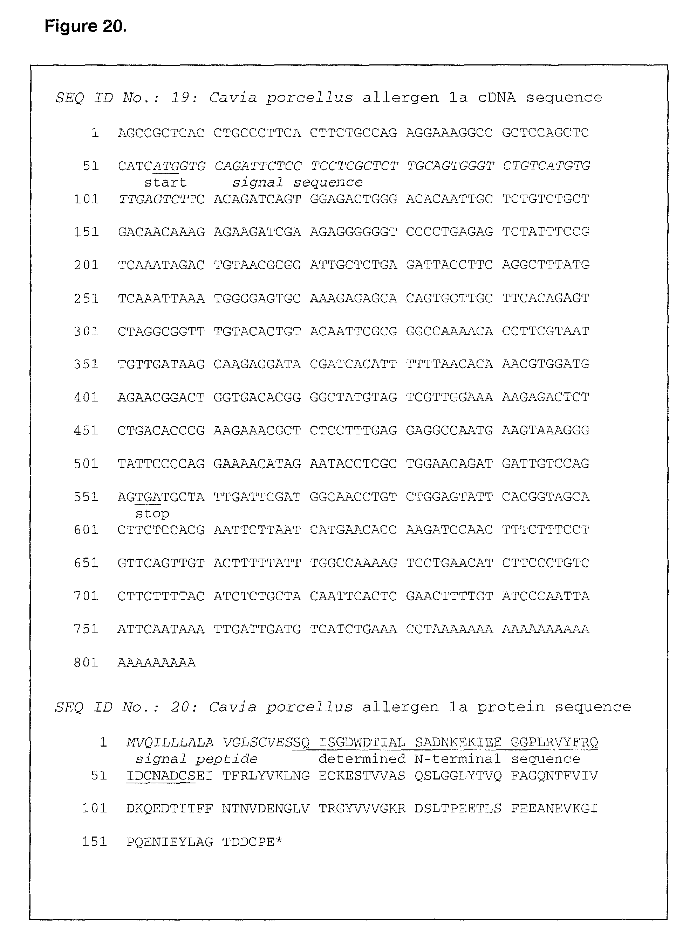

The term "fragment thereof" as used herein in context of polypeptides, refers to a functional fragment which has essentially the same (biological) activity as the polypeptides defined herein (e.g. as shown in Seq ID NOs 2, 4, 6 or 20, respectively) which may be) encoded by the polynucleotides (e.g. Seq ID NOs 1, 3, 5 or 19, respectively). With respect to a nucleotide sequence of Caviidae allergen, the term "fragment" as used herein means a nucleotide sequence being at least 7, at least 10, at least 15, at least 20, at least 30, at least 50, at least 100, at least 150, at least 200 or at least 250 nucleotides in length. As discussed herein above, an inventive and illustrative N-terminal fragment of the polypeptide provided in SEQ ID NO: 20 is a fragment that shows at least an identity of 95% to the first 15 amino acids as provided in SEQ ID NO: 20. Further sequence identity also in context of the other polypeptides, were provided herein above. With respect to an amino acid sequence of Caviidae allergen, the term "fragment" as used herein exemplary means an amino acid sequence being of at least 5, at least 6, at least 7, at least 8, at least 9, at least 10, at least 11, at least 12, at least 13, at least 14, at least 15, at least 16, at least 17, at least 18, at least 19, at least 20 or at least 25 amino acid residues in length. One fragment comprises at least 5 amino acids and ranges up to the first 15 amino acids or up to at least 18 amino acids of the N-terminal-region of *Cavia porcellus* allergen 1 a as also disclosed in Figure 20.

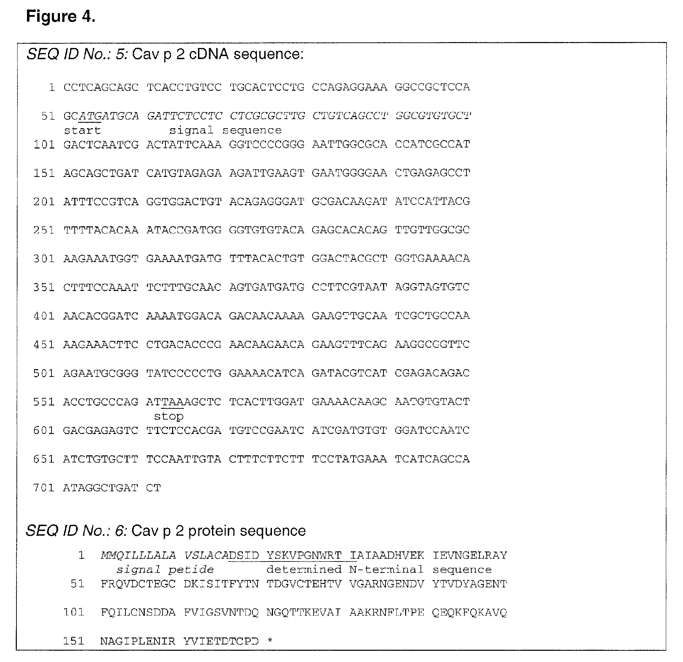

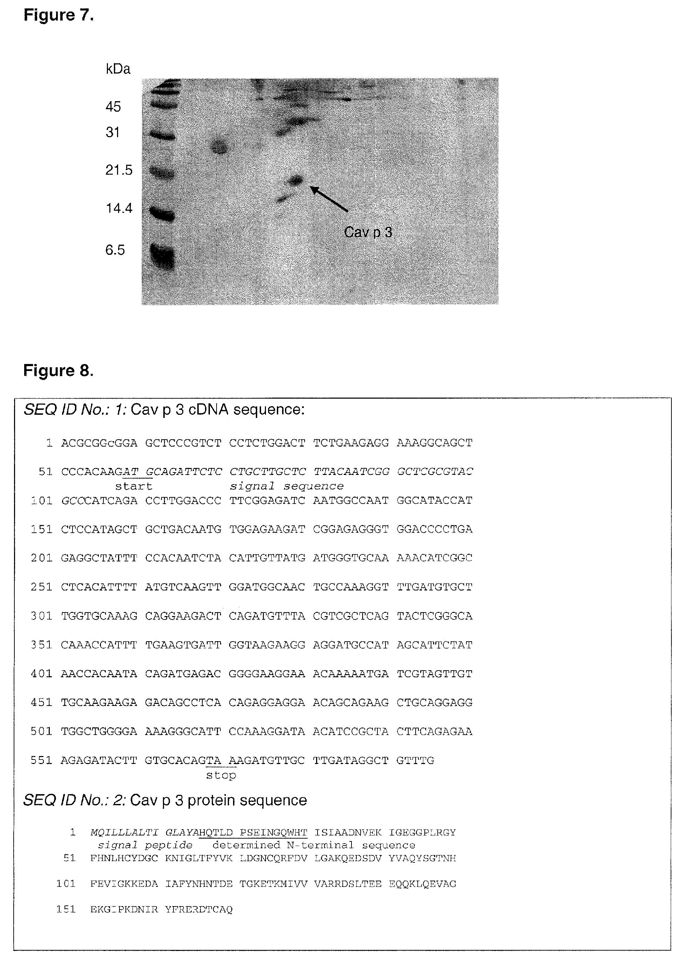

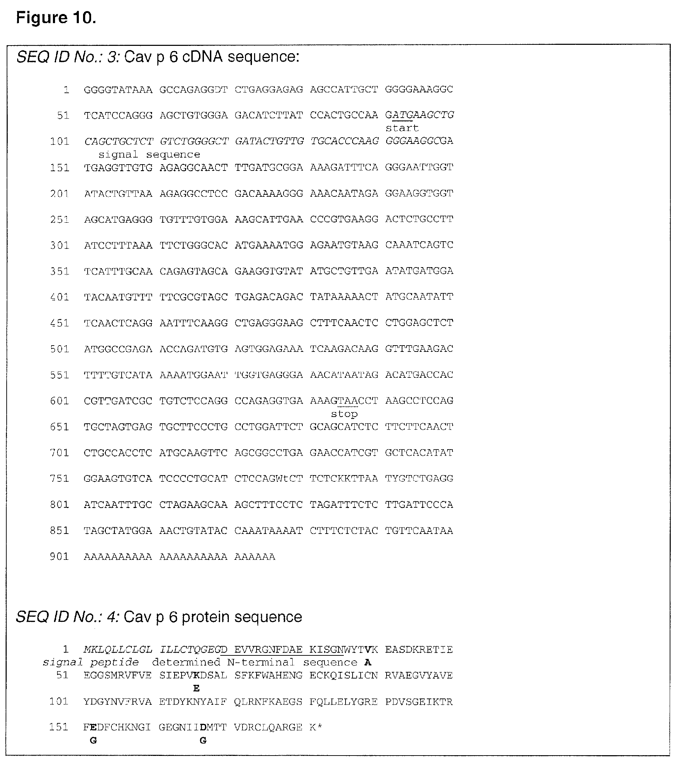

The person skilled in the art is in the position to deduce the coding sequences of the novel Caviidae allergens provided herein. The coding regions as well as the proteinous structures/ amino acids are provided herein and, for example, for *Cavia porcellus* allergen 1 a in Figure 20, the start codon is at nt 55-57, the stop codon is at nt 553-555; for Cav p 2 in Figure 4 start codon is at nt 53-55 and stop codon is at nt 563-565; for Cav p 3 in Figure 8 start codon is at nt 59-61 and stop codon is at nt 569-571; for Cav p 6 in Figure 10 start codon is at nt 92-94 and stop codon is at nt 635-637.

The term "activity" as used herein refers in particular to the capability of (a) polypeptide(s) to elicit an allergic response. In other words, the polypeptide(s) provided in the present invention are (an) allergen(s) as described herein above, preferably Caviidae allergen(s), more preferably (a) Cavia allergen(s) and most preferably (a) Cavia porcellus (guinea pig) allergen. It is to be understood that (an) allergen(s) (e.g. (a) guinea pig allergen(s)) is capable of eliciting an allergic response to said allergen not only in a human, but also in other animals, in particular in animals in which the allergen(s) are not/ cannot be derived/ obtained from (for example, in non-rodents, such as dogs, cats and the like). The definitions and explanations given herein below in context of diagnosis and treatment of patients suffering from (or suspected of suffering from) an allergy to (an) Caviidae allergen(s) also apply, mutatis mutandis, to the animal(s) described above. Furthermore, also envisaged in the present invention is an animal model which can be used for experimental immunotherapy.

A person skilled in the art will be aware that the (biological) activity as described herein often correlates with the expression level (e.g. protein/mRNA). If not mentioned otherwise, the term "expression" used herein refers to the expression of a nucleic acid molecule encoding a polypeptide/protein of the invention, whereas "activity" refers to activity of said polypeptide/protein. Methods/assays for determining the activity of polypeptides (e.g. the polypeptides shown in Seq ID Nos 2, 4, 6 or 20) are well known in the art. Exemplary methods for assessing the activity of polypeptides are shown in Example 6 (e.g. testing for IgE binding in immunoblots, ELISA for IgE reactivity).

In context of polynucleotide sequences the term "fragment thereof" refers in particular to (a) fragment(s) of nucleic acid molecules, wherein said nucleic acid molecules preferably encode (a) Caviidae allergen(s) (or a fragment thereof) as defined above. A "fragment of a polynucleotide" may, for example, encode a polypeptide (e.g. a polypeptide as shown in SEQ ID NOs 2, 4, 6 or 20) having at least one amino acid deletion and, optionally, at least one amino acid substitution, whereby said polypeptide preferably is a Caviidae allergen. Such a shortened polypeptide may be considered as a functional fragment of a polypeptide (e.g. as shown in SEQ ID NOs 2, 4, 6 or 20).

The term "hybridizes" used herein may refer to hybridization under conventional hybridization conditions, preferably under stringent conditions, as for instance described in Sambrook and Russell (2001), Molecular Cloning: A Laboratory Manual, CSH Press, Cold Spring Harbor, NY, USA. If not further specified, the conditions are preferably non-stringent. Said hybridization conditions may be established according to conventional protocols described, e.g., in Sambrook (2001) loc. cit. The setting of conditions is well within the skill of the artisan and can be determined according to protocols described in the art. Thus, the detection of only specifically hybridizing sequences will usually require stringent hybridization and washing conditions. As a non-limiting example, highly stringent hybridization may occur under the following conditions:
Hybridization buffer: 2 x SSC; 10 x Denhardt solution (Fikoll 400 + PEG + BSA;
ratio 1:1:1); 0.1% SDS; 5 mM EDTA; 50 mM Na₂HPO₄;
   250 µg/ml of herring sperm DNA; 50 µg/ml of tRNA; or
   0.25 M of sodium phosphate buffer, pH 7.2;
   1 mM EDTA
   7% SDS
Hybridization temperature T = 60°C
Washing buffer: 2 x SSC; 0.1% SDS
Washing temperature T = 60°C.

Low stringent hybridization conditions for the detection of homologous or not exactly complementary sequences may, for example, be set at 6 x SSC, 1% SDS at 65°C. As is well known, the length of the probe and the composition of the nucleic acid to be determined constitute further parameters of the hybridization conditions.

Polynucleotide sequences which are capable of hybridizing with the polynucleotide sequences provided herein can for instance be isolated from genomic libraries or cDNA libraries of animals. Preferably, such polynucleotides are from animal origin, particularly preferred from an animal belonging to the the class of mammalia, preferably rodentia, more preferably Caviidae, more preferably Caviinae, more preferably *Cavia,* most preferably *Cavia porcellus* (guinea pig(s)), *Cavia anolaimae, Cavia guianae, Cavia aperea, Cavia fulgida, Cavia intermedia, Cavia magna, Cavia nana* or *Cavia tschudii.* Alternatively, such nucleotide sequences can be prepared by genetic engineering or chemical synthesis.

Such polynucleotide sequences being capable of hybridizing may be identified and isolated by using the polynucleotide sequences described herein or parts or reverse complements thereof, for instance by hybridization according to standard methods (see for instance Sambrook and Russell (2001), Molecular Cloning: A Laboratory Manual, CSH Press, Cold Spring Harbor, NY, USA). Nucleotide sequences comprising the same or substantially the same nucleotide sequences as indicated in the listed SEQ ID NOs, or parts/fragments thereof, can, for instance, be used as hybridization probes. The fragments used as hybridization probes can also be synthetic fragments which are prepared by usual synthesis techniques, the sequence of which is substantially identical with that of a nucleotide sequence.

The meaning of the term "identity" and "homology", respectively, particularly with respect to two nucleotide sequences or amino acid sequences/polypeptides to be compared, is known in the art and used herein accordingly. For example, this term is used herein in the context of a nucleotide sequence or amino acid sequence/polypeptide which has a homology, that is to say a sequence identity, of at least 50%, 55%, 60%, preferably of at least 70%, 75% more preferably of at least 80%, 85%, 86%, 87%, 88%, 89%, 90%, 91%, 92%, 93%, 94, 95%, 96%, 97%, 98%, and even most preferably of at least 99% to another, preferably entire, nucleotide sequence or amino acid sequence, respectively. In other words, a polypeptide (being a Caviidae allergen or fragment thereof) has at least 50%, 55% 60% preferably at least 70%, 75% more preferably at least 80%, 85%, 86%, 87%, 88%, 89%, 90%, 91%, 92%, 93%, 94, 95%, 96%, 97%, 98%, and most preferably at least 99% identity/ homology to the polypeptide shown in Seq ID No 2, 4, 6 and 20, respectively.

If the two nucleotide sequences to be compared by sequence comparisons differ in identity refers to the shorter sequence and that part of the longer sequence that matches the shorter sequence. In other words, when the sequences which are compared do not have the same length, the degree of identity preferably either refers to the percentage of nucleotide residues in the shorter sequence which are identical to nucleotide residues in the longer sequence or to the percentage of nucleotides in the longer sequence which are identical to nucleotide sequence in the shorter sequence. In this context, the skilled person is readily in the position to determine that part of a longer sequence that "matches" the shorter sequence.

The search for sequence identity or homology on DNA and protein level can be done according to the present invention by using the publically available program Blast. Further, pairwise sequence comparisons may be done using Gap on the Heidelberg Unix Sequence Analysis Resources (HUSAR) server using the alignment method of Needleman and Wunsch (J. Mol. Biol. 48; 443-453 (1970).

In general, the person skilled in the art knows how nucleic acid molecules can be obtained, for instance, from natural sources (e.g. animals belonging to the family "Caviidae") or may also be produced synthetically or by recombinant techniques, such as PCR. These nucleic acid molecules and include modified or derivatized, nucleic acid molecules as can be obtained by applying techniques described in the pertinent literature. Primers as provided herein are useful in diagnostic as well as scientific (research) settings. Accordingly, primers are defined as shown in SEQ ID NOS: 7 to 11 for the amplification of the Cav p 2 gene, SEQ ID NOS: 12 to 16 for the amplification of the Cav p 2 gene, SEQ ID NOS: 17 to 18 for the amplification of the Cav p 6 gene and SEQ ID NOS: 21 to 42 for the amplification of the *Cavia porcellus* allergen 1 a gene or a fragment thereof as shown in SEQ ID NO:1 (Cav p 3), SEQ ID NO:5 (Cav p 2), SEQ ID NO:3 (Cav p 6) or SEQ ID NO: 19 *(Cavia porcellus* allergen 1 a). These primers can be also used as primer pairs.

The primers being represented by SEQ ID NOS: 21 to 32 (Cavp-MQ1 to Cavp-MQ 12) and SEQ ID NOS: 41 to 42 (Cavp-11 for (3'RACE) and Cavp-b_rev for (5'RACE)) bind to both isoforms of the Caviidae allergens *Cavia porcellus* allergen 1 a gene and Cav p 1. However, the primers being represented by SEQ ID NOS: 33 to 40 (Cavp-MQ1rev to Cavp-MQ8rev) bind specifically to the *Cavia porcellus* allergen 1a gene. These primers can be further used in a kit according. The primer used as shown in SEQ ID NOS: 7 to 11, 12 to 16, 17 to 18 and 21 to 42 may also comprise a shorter sequence starting from AAR to TGG with 18 nucleotides. However, the skilled person is in a position to modify and amend this primers, primer sequences and primer pairs, for example, by elongation and/or shortening of the sequences or distinct nucleotide exchanges. Such an exchange needs to lead to a complementary strand sequence that is still capable to specifically hybridize under conditions disclosed herein (for example stringent hybridization conditions) to its corresponding sequence on the corresponding complementary strand.

Identity, moreover, means that there is a functional and/or structural equivalence between the corresponding nucleotide sequence or polypeptides, respectively (e.g. polypeptides encoded thereby). Nucleotide/amino acid sequences which have at least 50%, 55%, 60%, preferably of at least 70%, 75% more preferably of at least 80%, 85%, 86%, 87%, 88%, 89%, 90%, 91%, 92%, 93%, 94, 95%, 96%, 97%, 98%, and even most preferably of at least 99% identity to the herein-described particular nucleotide/amino acid sequences may represent derivatives/variants of these sequences which, preferably, have the same biological function. They may be either naturally occurring variations, for instance sequences from other ecotypes, varieties, species, etc., or mutations, and said mutations may have formed naturally or may have been produced by deliberate mutagenesis. Furthermore, the variations may be synthetically produced sequences. The allelic variants may be naturally occurring variants or synthetically produced variants or variants produced by recombinant DNA techniques. Deviations from the above-described polynucleotides may have been produced, e.g., by deletion, substitution, addition, insertion and/or recombination. The term "addition" refers to adding at least one nucleic acid residue /amino acid to the end of the given sequence, whereas "insertion" refers to inserting at least one nucleic acid residue /amino acid within a given sequence.

The variant polypeptides and, in particular, the polypeptides encoded by the different variants of the nucleotide sequences exhibit certain characteristics they have in common. These include, for instance, biological activity, molecular weight, immunological reactivity, conformation, etc., and physical properties, such as for instance the migration behavior in gel electrophoreses, chromatographic behavior, sedimentation coefficients, solubility, spectroscopic properties, stability, pH optimum, temperature optimum etc.

The present invention provides for antigens/allergens from Caviidea which are particularly useful in their isolated and purified form for diagnostic purposes disclosed herein as well as in pharmaceutical compositions. The term "isolated" as used herein with reference to a nucleic acid molecule refers to a nucleic acid molecule that is separated from at least one other nucleic acid molecule with which it is ordinarily associated, for example, in its natural environment. An isolated nucleic acid molecule further includes a nucleic acid molecule contained in cells that ordinarily express the nucleic acid molecule, but the nucleic acid molecule is present extrachromosomally or at a chromosomal location that is different from its natural chromosomal location. Isolated nucleic acid molecules also refer to molecules that are substantially free of material from the environment of production. For example, isolated nucleic molecules refer to molecules free of cellular material and culture medium/products when produced by recombinant methods, or chemical precursors or other reaction chemicals when synthesized.

The term "isolated" as used herein with reference to a protein refers to a protein that is separated from at least one other protein with which it is ordinarily associated, for example, in its natural environment. Isolated proteins also refer to molecules that are substantially free of material from the environment of production. For example, isolated proteins are substantially free of cellular material and culture medium/products when produced by recombinant methods, or chemical precursors or other reaction chemicals when synthesized. "Purified" as used herein references that a molecule (including the herein disclosed polypeptides or fragments thereof) is present in a sample at a concentration of at least 90% by weight, preferably at least 95% by weight, and more preferably at least 98% by weight of the sample in which it is contained.

Accordingly, and in a further aspect, the present invention relates to a vector comprising the nucleic acid molecules described herein and a recombinant host cell comprising the nucleic acid molecules and/or the vector.

The term "vector" as used herein particularly refers to plasmids, cosmids, viruses, bacteriophages and other vectors commonly used in genetic engineering. In a preferred embodiment, the vectors of the invention are suitable for the transformation of cells, like fungal cells, cells of microorganisms such as yeast or bacterial cells or animal cells. The vectors as well as the host cells of the present invention are particularly useful in the recombinant expression of the polypeptides (Caviidae allergens/antigens) of the present invention.

In a further aspect, the recombinant host cell of the present invention is capable of expressing or expresses the polypeptide encoded by the polynucleotide sequence of this invention. In a specific embodiment, the "polypeptide" comprised in the host cell may be a heterologous with respect to the origin of the host cell. An overview of examples of different expression systems to be used for generating the host cell of the present invention, for example the above-described particular one, is for instance contained in Glorioso et al. (1999), Expression of Recombinant Genes in Eukaryotic Systems, Academic Press Inc., Burlington, USA, Paulina Balbas und Argelia Lorence (2004), Recombinant Gene Expression: Reviews and Protocols, Second Edition: Reviews and Protocols (Methods in Molecular Biology), Humana Press, USA.

The transformation or genetically engineering of the host cell with a nucleotide sequence or the vector according to the invention can be carried out by standard methods, as for instance described in Sambrook and Russell (2001), Molecular Cloning: A Laboratory Manual, CSH Press, Cold Spring Harbor, NY, USA. Moreover, the host cell of the present invention is cultured in nutrient media meeting the requirements of the particular host cell used, in particular in respect of the pH value, temperature, salt concentration, aeration, antibiotics, vitamins, trace elements etc. The term "recombinant host cell", as used herein, relates to a host cell, genetically engineered with the nucleotide sequence of the present invention or comprising the vector or the polypeptide of the present invention.

Generally, the host cell of the present invention may be a prokaryotic or eukaryotic cell comprising the nucleotide sequence, the vector and/or the polypeptide of the invention or a cell derived from such a cell and containing the nucleotide sequence, the vector and/or the polypeptide of the invention. In a preferred embodiment, the host cell comprises, for example due to genetic engineering, the nucleotide sequence or the vector of the invention in such a way that it contains the nucleotide sequences of the present invention integrated into the genome. Non-limiting examples of such a host cell of the invention (but also the host cell of the invention in general) may be a bacterial, yeast, fungus, plant, animal or human cell.

In accordance with the above, the invention relates in a further embodiment to a method for producing a polypeptide provided herein, comprising culturing the recombinant host cell under such conditions that the polypeptide is expressed, and recovering the polypeptide.

The term "such conditions", as used herein, refers to culture conditions of recombinant host cells in order to express and recover polypeptides, preferably heterologous polypeptides. These conditions are well known to a person skilled in the art, as for instance described in Sambrook and Russell (2001), Molecular Cloning: A Laboratory Manual, CSH Press, Cold Spring Harbor, NY, USA.

In general, the polypeptide of this invention may, in addition to the Caviidae allergen of the invention and as defined herein, comprise (a) further polypeptide(s), i.e. (a) polypeptide(s) being heterologous with respect to the polypeptides of the present invention. One skilled in the art will be aware that the heterologous polypeptide can be expressed by prokaryotic (e.g. bacteria) or eukaryotic cell (e.g. 293 cells or CHO cells). The present invention also relates to a fusion protein (and a nucleic acid molecule encoding the fusion protein). For example, the further/ heterologous polypeptide(s) may particularly be suitable for a potentiated or increased production for the polypeptide of the present invention (for example, in a cell). The further/heterologous polypeptide(s) may, for example, comprise a protein fragment or peptide, an entire functional moiety, or an entire protein sequence which can be designed to be used in purifying the fusion protein, for example either with antibodies or with affinity purification specific for the further/heterologous polypeptide. Likewise, physical properties of the additional polypeptide, protein fragment, peptide (and the like) can be exploited to allow selective purification of the heterologous polypeptide and, hence, also the polypeptide of the present invention.

As a fusion protein, the polypeptides as shown in Seq ID No 2, 4, 6 and 20, respectively, may also include a reporter or reporter construct being expressible in a cell, a tissue, a cell culture, tissue culture, animals or plants. Thus, the polypetide of the present invention can be easily identified and measured by the skilled person, in order to determine whether the polypeptide has been expressed in the cell, animals or plants

Commonly used reporter genes that induce visually identifiable characteristics usually involve fluorescent and luminescent proteins; examples include the gene that encodes jellyfish green fluorescent protein (GFP), which causes cells that express it to glow green under blue light, the enzyme luciferase, which catalyzes a reaction with luciferin to produce light, and the red fluorescent protein from the gene dsRed. Another common reporter in bacteria is the *lacZ* gene, which encodes the protein β-galactosidase. This enzyme causes bacteria expressing the gene to appear blue when grown on a medium that contains the substrate analog X-gal (an inducer molecule such as Isopropyl β-D-1-thiogalactopyranoside (IPTG) is also needed under the native promoter).

One of skill in the art will recognize that the particular peptide/protein fragment etc. is chosen with the purification scheme in mind. As a non-limiting example, His tags, GST, and maltose-binding protein represent peptides that have readily available affinity columns to which they can be bound and eluted. Thus, where the peptide is an N-terminal His tag such as hexahistidine (His.sub.6 tag), the heterologous protein can be purified using a matrix comprising a metal-chelating resin, for example, nickel nitrilotriacetic acid (Ni-NTA), nickel iminodiacetic acid (Ni-IDA), and cobalt-containing resin (Co-resin). See, for example, Steinert et al. (1997) QIAGEN News 4:11-15. Where the peptide is GST, the heterologous protein can be purified using a matrix comprising glutathione-agarose beads (Sigma or Pharmacia Biotech); where the protein fragment is a maltose-binding protein (MBP), the heterologous protein can be purified using a matrix comprising an agarose resin derivatized with amylose.

In a further embodiment, the present invention refers to a polypeptide having the amino acid sequence encoded by one of the nucleic acid molecules which encodes a Caviidae allergen. Furthermore, the polypeptide is obtainable by the above-mentioned method. Polypeptides of the present invention have been described in detail herein above.

Disclosed herein are molecules which bind to the nucleic acid molecules and/or polypeptides of the present invention and which can, accordingly used in the herein described uses and methods. Binding molecules according to the invention can, *inter alia,* be used for detecting the presence, absence or amount of the nucleic acid molecules/ polypeptides of the invention in a sample, in particular in the framework of methods and uses described herein. The binding molecules may furthermore be used for isolating the nucleic acid molecules/ polypeptides from a biological source material. Moreover, the binding molecules to be used/ to be identified that may be used to inhibit/antagonize Cav p 2, Cav p 3, Cav p 6 and/or *Cavia porcellus* allergen 1 a or fragments thereof, which may, for example, result in a reduction of the amount or inhibition of the activity of the molecules/polypeptides as defined herein. Non-limiting examples of suitable binding molecules may be selected from aptamers (Klussmann (2006), The Aptamer Handbook: Functional Oligonucleotides and their applications, Wiley-VCH, USA), RNAi, shRNA (Mclntyre and Fanning (2006), BMC Biotechnology 6:1), ribozymes, antisense nucleotide sequences (like antisense DNAs or antisense RNAs), siRNA (Hannon (2003), Rnai: A Guide to Gene Silencing, Cold Spring Harbor Laboratory Press, USA), PNAs, antibodies (Howard and Bethell (2000) Basic Methods in Antibody Production and Characterization, Crc. Pr. Inc), (Hansson, Immunotechnology 4 (1999), 237-252; Henning, Hum Gene Ther. 13 (2000), 1427-1439), affibodies, lectins, trinectins (Phylos Inc., Lexington, Massachusetts, USA; Xu, Chem. Biol. 9 (2002), 933), anticalins (EPB1 1 017 814) and the like.

In a particular embodiment, the present invention relates to an antibody and the use thereof that specifically binds to the polypeptide as shown in Seq ID 20, respectively, and as further described and defined herein, in particular a Caviidae allergen. Moreover, said antibody can be used for the purification of said polypeptide. The term "antibody" is well known in the art.

In context of the present invention, the term "antibody" as used herein relates in particular to full immunoglobulin molecules as well as to parts of such immunoglobulin molecules substantially retaining binding specificity. Furthermore, the term relates to modified and/or altered antibody molecules, like chimeric and humanized antibodies, CDR-grafted antibodies, recombinantly or synthetically generated/synthesized antibodies and to intact antibodies as well as to antibody fragments thereof, like, separated light and heavy chains, Fab, Fab/c, Fv, Fab', F(ab')₂. The term "antibody" also comprises bifunctional antibodies, trifunctional antibodies and antibody constructs, like single chain Fvs (scFv) or antibody-fusion proteins. Further "antibody" constructs are known in the art and comprised in the present invention.

Techniques for the production of antibodies are well known in the art and described, e.g. in Howard and Bethell (2000) Basic Methods in Antibody Production and Characterization, Crc. Pr. Inc. Antibodies directed against a polypeptide according to the present invention can be obtained, e.g., by direct injection of the polypeptide (or a fragment thereof) into an animal or by administering the polypeptide (or a fragment thereof) to an animal. The antibody so obtained will then bind polypeptide (or a fragment thereof) itself. In this manner, even a fragment of the polypeptide can be used to generate antibodies binding the whole polypeptide, as long as said binding is "specific" as defined above.

These polypeptides are particularly useful in the preparation of specific antibodies and are provided herein for illustrative purposes.

Also in the appended examples (e.g. Example 2) the generation of specific antibodies against a polypeptide of the present invention are shown. In case of *Cavia porcellus* allergen 1 a, potential epitopes for production of specific antibodies to *Cavia porcellus* allergen 1 a are, for example, shown in SEQ 20 at positions 78-86, 106-110 and/or 139-153.

With the normal skill of the person skilled in the art and by routine methods, the person skilled in the art can easily deduce from the sequences provided herein relevant epitopes (also functional fragments) of the polypeptides of the present invention which are useful in the generation of antibodies like polyclonal and monoclonal antibodies. However, the person skilled in the art is readily in a position to also provide for engineered antibodies like CDR-grafted antibodies or also humanized and fully human antibodies and the like.

Particularly preferred in the context of the present invention are monoclonal antibodies. For the preparation of monoclonal antibodies, any technique which provides antibodies produced by continuous cell line cultures can be used. Examples for such techniques include the hybridoma technique, the trioma technique, the human B-cell hybridoma technique and the EBV-hybridoma technique to produce human monoclonal antibodies (Shepherd and Dean (2000), Monoclonal Antibodies: A Practical Approach, Oxford University Press, Goding and Goding (1996), Monoclonal Antibodies: Principles and Practice - Production and Application of Monoclonal Antibodies in Cell Biology, Biochemistry and Immunology, Academic Pr Inc, USA).

The antibody derivatives can also be produced by peptidomimetics. Further, techniques described for the production of single chain antibodies (see, inter alia, US Patent 4,946,778) can be adapted to produce single chain antibodies specifically recognizing the polypeptide of the invention. Also, transgenic animals may be used to express humanized antibodies to the polypeptide of the invention.

As shown in the appended examples, the present invention also envisages the production of specific antibody against native polypeptides and recombinant polypeptides according to the invention. This production is based, for example, on the immunization of animals, like mice. However, also other animals for the production of antibody/antisera is envisaged within the present invention. For example, monoclonal and polyclonal antibodies can be produced by rabbit, mice, goats, donkeys and the like. The polynucleotide according to the invention as shown SEQ ID NO: 19 *(Cavia porcellus* allergen 1 a) can be subcloned into an appropriated vector, wherein the recombinant polypeptide is to be expressed in an organism being able for an expression, for example in bacteria. Thus, the expressed recombinant protein can be intra-peritoneally injected into a mice and the resulting specific antibody can be , for example, obtained from the mice serum being provided by intra-cardiac blood puncture. The amount of obtained specific antibody can be quantified using an ELISA, which is also described herein below. Further methods for the production of antibodies are well known in the art, see, e.g. Harlow and Lane, "Antibodies, A Laboratory Manual", CSH Press, Cold Spring Harbor, 1988.

The term "specifically binds", as used herein, refers to a binding reaction that is determinative of the presence of the Caviidae allergen and antibody in the presence of a heterogeneous population of proteins and other biologics. Also, the term "specifically binds" relates to, for example, for the distinction between the Cavp 1 (N-Terminus as provided by Fahlbusch loc. cit.) and the novel *Cavia porcellus* allergen 1 a gene as provided herein.

Thus, under designated assay conditions, the specified antibodies and allergens bind to one another and do not bind in a significant amount to other components present in a sample. Specific binding to a target analyte under such conditions may require a binding moiety that is selected for its specificity for a particular target analyte. A variety of immunoassay formats may be used to select antibodies specifically reactive with a particular antigen. For example, solid-phase ELISA immunoassays are routinely used to select monoclonal antibodies specifically immunoreactive with an analyte. See Shepherd and Dean (2000), Monoclonal Antibodies: A Practical Approach, Oxford University Press and/ or Howard and Bethell (2000) Basic Methods in Antibody Production and Characterization, Crc. Pr. Inc. for a description of immunoassay formats and conditions that can be used to determine specific immunoreactivity. Typically a specific or selective reaction will be at least twice background signal to noise and more typically more than 10 to 100 times greater than background. The person skilled in the art is in a position to provide for and generate specific binding molecules directed against the novel polypeptides. For specific binding-assays it can be readily employed to avoid undesired cross-reactivity, for example polyclonal antibodies can easily be purified and selected by known methods (see Shepherd and Dean, loc. cit.).

The term "purification", as used herein, refers to a series of processes intended to isolate a single type of protein from a complex mixture. Protein purification is vital for the characterisation of the function, structure and interactions of the protein of interest. The starting material, as a non-limiting example, can be a biological tissue or a microbial culture. The various steps in the purification process may free the protein from a matrix that confines it, separate the protein and non-protein parts of the mixture, and finally separate the desired protein from all other proteins. Separation steps exploit differences in protein size, physico-chemical properties and binding affinity. Exemplary purification methods are also shown in appended Example 1.

In accordance with the above and in relation with the embodiments of this invention, the present invention relates also to a pharmaceutical composition comprising the polynucleotide as shown in SEQ ID NO: 19 *(Cavia porcellus* allergen 1a), the vector, the polypeptide as shown in Seq ID No 2, 4, 6 and 20, respectively, or the antibody. The pharmaceutical composition may also comprise (functional) fragments of the polypeptides provided herein. Such (functional) fragments may, inter alia, be used in the modification of the immune system of a patient, like an allergenic patient. Moreover, the use of the pharmaceutical composition in active or passive immunisation, like hyposensiblisation or in medical intervention of an allergic reaction is also envisaged in the context of the present invention. Active immunization means in this context to the introduction of a foreign molecule into the body, which causes the body itself to generate immunity against the target. It clear for the skilled person that this immunity comes from the T cells and the B cells with their antibodies. Passive immunization relates to an immunization where pre-synthesized elements of the immune system are transferred to a person so that the body does not need to produce these elements itself. Artificial passive immunization is normally administered by injection and is used if there has been a recent outbreak of a particular disease.

The pharmaceutical composition will be formulated and dosed in a fashion consistent with good medical practice, taking into account the clinical condition of the individual patient, the site of delivery of the pharmaceutical composition, the method of administration, the scheduling of administration, and other factors known to practitioners. The "effective amount" of the pharmaceutical composition for purposes herein is thus determined by such considerations.

The skilled person knows that the effective amount of pharmaceutical composition administered to an individual will, inter alia, depend on the nature of the compound. For example, if said compound is a (poly)peptide or protein the total pharmaceutically effective amount of pharmaceutical composition administered parenterally per dose will be in the range of about 1 µg protein /kg/day to 10 mg protein /kg/day of patient body weight, although, as noted above, this will be subject to therapeutic discretion. More preferably, this dose is at least 0.01 mg protein /kg/day, and most preferably for humans between about 0.01 and 1 mg protein /kg/day. If given continuously, the pharmaceutical composition is typically administered at a dose rate of about 1 µg/kg/hour to about 50 µg/kg/hour, either by 1-4 injections per day or by continuous subcutaneous infusions, for example, using a mini-pump. An intravenous bag solution may also be employed. The length of treatment needed to observe changes and the interval following treatment for responses to occur appears to vary depending on the desired effect. The particular amounts may be determined by conventional tests which are well known to the person skilled in the art. Pharmaceutical compositions of the invention may be administered orally, parenterally, intracisternally, intraperitoneally, topically (as by powders, ointments, drops or transdermal patch), bucally, or as an oral or nasal spray.

Pharmaceutical compositions of the invention preferably comprise a pharmaceutically acceptable carrier. By "pharmaceutically acceptable carrier" is meant a non-toxic solid, semisolid or liquid filler, diluent, encapsulating material or formulation auxiliary of any type. The term "parenteral" as used herein refers to modes of administration which include intravenous, intramuscular, intraperitoneal, intrasternal, subcutaneous and intraarticular injection and infusion.

The pharmaceutical composition is also suitably administered by sustained release systems. Suitable examples of sustained-release compositions include semipermeable polymer matrices in the form of shaped articles, e.g., films, or mirocapsules. Sustained-release matrices include polylactides (U.S. Pat. No. 3,773,919, EP 58,481), copolymers of L-glutamic acid and gamma-ethyl-L-glutamate (Sidman, U. et al., Biopolymers 22:547-556 (1983)), poly (2-hydroxyethyl methacrylate) (R. Langer et al., J. Biomed. Mater. Res. 15:167-277 (1981), and R. Langer, Chem. Tech. 12:98-105 (1982)), ethylene vinyl acetate (R. Langer et al., Id.) or poly-D-(-)-3-hydroxybutyric acid (EP 133,988). Sustained release pharmaceutical composition also include liposomally entrapped compound. Liposomes containing the pharmaceutical composition are prepared by methods known per se: DE 3,218,121; Epstein et al., Proc. Natl. Acad. Sci. (USA) 82:3688-3692 (1985); Hwang et al., Proc. Natl. Acad. Sci. (USA) 77:4030-4034 (1980); EP 52,322; EP 36,676; EP 88,046; EP 143,949; EP 142,641; Japanese Pat. Appl. 83-118008; U.S. Pat. Nos. 4,485,045 and 4,544,545; and EP 102,324. Ordinarily, the liposomes are of the small (about 200-800 Angstroms) unilamellar type in which the lipid content is greater than about 30 mol. percent cholesterol, the selected proportion being adjusted for the optimal therapy.

For parenteral administration, the pharmaceutical composition is formulated generally by mixing it at the desired degree of purity, in a unit dosage injectable form (solution, suspension, or emulsion), with a pharmaceutically acceptable carrier, i.e., one that is non-toxic to recipients at the dosages and concentrations employed and is compatible with other ingredients of the formulation.

Generally, the formulations are prepared by contacting the components of the pharmaceutical composition uniformly and intimately with liquid carriers or finely divided solid carriers or both. Then, if necessary, the product is shaped into the desired formulation. Preferably the carrier is a parenteral carrier, more preferably a solution that is isotonic with the blood of the recipient. Examples of such carrier vehicles include water, saline, Ringer's solution, and dextrose solution. Non aqueous vehicles such as fixed oils and ethyl oleate are also useful herein, as well as liposomes. The carrier suitably contains minor amounts of additives such as substances that enhance isotonicity and chemical stability. Such materials are non-toxic to recipients at the dosages and concentrations employed, and include buffers such as phosphate, citrate, succinate, acetic acid, and other organic acids or their salts; antioxidants such as ascorbic acid; low molecular weight (less than about ten residues) (poly)peptides, e.g., polyarginine or tripeptides; proteins, such as serum albumin, gelatin, or immunoglobulins; hydrophilic polymers such as polyvinylpyrrolidone; amino acids, such as glycine, glutamic acid, aspartic acid, or arginine; monosaccharides, disaccharides, and other carbohydrates including cellulose or its derivatives, glucose, manose, or dextrins; chelating agents such as EDTA; sugar alcohols such as mannitol or sorbitol; counterions such as sodium; and/or nonionic surfactants such as polysorbates, poloxamers, or PEG.

The components of the pharmaceutical composition to be used for therapeutic administration must be sterile. Sterility is readily accomplished by filtration through sterile filtration membranes (e.g., 0.2 micron membranes). Therapeutic components of the pharmaceutical composition generally are placed into a container having a sterile access port, for example, an intravenous solution bag or vial having a stopper pierceable by a hypodermic injection needle.

The components of the pharmaceutical composition ordinarily will be stored in unit or multi-dose containers, for example, sealed ampoules or vials, as an aqueous solution or as a lyophilized formulation for reconstitution. As an example of a lyophilized formulation, 10-ml vials are filled with 5 ml of sterile-filtered 1% (w/v) aqueous solution, and the resulting mixture is lyophilized. The infusion solution is prepared by reconstituting the lyophilized compound(s) using bacteriostatic Water-for-Injection.

The term "medical intervention" as herein described, refers to any examination, treatment, or other act having preventive, diagnostic therapeutic or rehabilitative aims and which is carried out by a physician or other health care provider (WHO).

The term "allergic reaction", as used herein, refers to the hypersensitive response of the immune system of an allergic individual to a substance by an immunoglobulin E (IgE) mediated reaction in the organism. Such allergic reactions are also known in the art as type I hypersensitivity reactions. The term allergic reaction or allergic reactions are used broadly to refer to such reactions and to diseases or symptoms associated with such reactions including allergic rhinitis, allergic asthma, anaphylaxis, wheal and flare reaction, eczema, urticaria and dermatitis.

The term "hyposensibilization", as used herein, refers to a medical treatment that affects the natural course of an allergic disease, and that may cure allergy. The treatment is by injecting gradually increasing doses of the allergen to which the patient is allergic. By increasing the dosage very gradually, the immune system may then get the opportunity to "learn" to react correctly when exposed to the allergen in question. The treatment seems to work by inducing the formation of "blocking antibodies" of the IgG4 subclass, which prevent the allergen from triggering an allergic reaction. The injection programme usually carries on for a period of three to five years. In the initial phase, the dosage is gradually increased from a very low initial value. Typically, the total increase of dosage may by a factor of 1:10000.

In accordance with the above and in relation with the embodiments of this invention, the present invention relates also to the use of the polynucleotide, the polypeptide or the antibody for the preparation of a pharmaceutical composition for use in hyposensibilization or in medical intervention of an allergic reaction. The pharmaceutical composition is particularly useful in the medical intervention of allergies against Caviidae.

In a further embodiment, the present invention relates to the use of said polynucleotide, the said polypeptide or the said antibody in diagnosis of allergic reaction or for the determination of a subject prone to an allergic reaction. Furthermore, the above-mentioned components can be also used for the preparation of a diagnostic composition for the diagnosis of allergic reaction or for the determination of a subject prone to an allergic reaction. The subject is preferably a human subject.

The compounds of the present invention (polypeptides and fragments thereof, polynucleotides, vectors, host cells, primers etc.) may also be comprised in a diagnostic composition.

The term "diagnostic composition" as herein described, refers to one of the aforementioned compounds which is prepared to be used for diagnostic purposes. It is to be understood that depending on the nature of the diagnostic agent, i.e. depending on whether a polynucleotide, a polypeptide, an antibody or an oligonucleotide is used, the diagnostic composition may comprise additional agents such as agents which allow hybridization, antibody binding or detection. Such additional agents are well known to those skilled in the art.

The term "diagnosis" as used herein means identification of pathological condition and features. For the purpose of the invention, a diagnosis is to identify the allergenicity to a Caviidae allergen(s) of an individual. One of the known diagnosis method to be used in accordance with the present invention is the skin test which can be performed by placing a small amount of a suspected allergen on or below the skin to see if a reaction develops. However, there are different types of skin tests. The skin prick test can be applied by placing a drop of a solution containing a possible allergen on the skin, and a series of scratches or needle pricks allows the solution to enter the skin. If the skin develops a red, raised itchy area, it usually means that the person is allergic to that allergen indicating a positive reaction. In the intradermal test a small amount of the allergen solution is injected into the skin. An intradermal allergy test may be done when a substance does not cause a reaction in the skin prick test but is still suspected as an allergen for that person. The intradermal test is more sensitive than the skin prick test but is more often positive in people who do not have symptoms to that allergen (false-positive test results). For a skin patch test, the allergen solution is placed on a pad that is taped to the skin for 24 to 72 hours. This test can be used to detect a skin allergy, e.g. contact dermatitis.

Another known method to diagnose allergenicity is the allergy blood test which aims at the identification of substances in the blood, e.g. antibodies. Blood tests are not as sensitive as skin tests but are often used for people who are not able to have skin tests. A common type of blood test used is the enzyme-linked immunosorbent assay (ELISA, EIA). It measures the blood level of a type of antibody (called immunoglobulin E or G (IgE/IgG)) that the body may make in response to certain allergens. Other lab testing methods, such as radioallergosorbent testing (RAST), an immunoassay capture test (ImmunoCAP, UniCAP, or Pharmacia CAP), a cytometry beads array (CBA) or a microarray, preferably a protein microarray, may be used to provide more information. Protein microarrays (also protein binding microarray, biochip, proteinchip) are measurement devices used in biomedical applications to determine the presence and/or amount (referred to as quantitation) of polypeptides/ proteins or fragments thereof in biological samples, e.g. blood. The protein microarray provides a multiplex approach to identify protein-protein interactions or to identify the targets of biologically active small molecules. In this context, the polypeptides/ proteins or fragments thereof can be externally synthesised, purified and attached to the array. Alternatively they can be synthesised in-situ and directly attached to the array. In the present invention, the array can be a piece of glass or silicon on which different molecules of polypeptides/ proteins or fragments thereof have been affixed at separate locations in an ordered manner thus forming a microscopic array. The protein microarray of the present invention can also be used as an antibody microarray, where antibodies are spotted onto the protein chip and are used as capture molecules to detect proteins in biological samples, e.g. blood.

The appended examples document how the novel polypeptides can be used in diagnostic setting. For example, the quantification of specific IgE antibodies to (r)Cavia porcellus allergen 1a, (r)Cav p 2, (r)Cav p 3 and/or (r)Cav p 6. Purified recombinant *Cavia porcellus* allergen 1 a, Cav p 2, Cav p 3 and Cav p 6 are coated overnight at 4°C at a concentration of 5 µg/ml in PBS to microtiter plates. Plates are blocked with blocking buffer (3% bovine serum albumin (BSA, Sigma) in TBS/0.05% Tween-20 for *Cavia porcellus* allergen 1a and 2% cold water fish gelatin (CWFG, Sigma) for Cav p 2, 3 and 6) for 30 min at room temperature. Patient sera can be serially diluted into blocking buffer and may be added to the microtiter plates for 2 hours. Binding of IgE is measured by incubation, for example, for 1 hour with biotin-labeled antihuman IgE antibodies (1/2000); Southern Biotechnology, Birmingham, Ala) followed by an incubation for 30 min with alkaline phosphatase-labeled streptavidine (1/1000; BD Biosciences Pharmingen, Erembodegem, Belgium). As detection means herein, p-Nitrophenyl phosphate can be added as substrate, and plates were left in the dark before OD was read at 405 nm. The cutoff may be determined as the mean of the negative controls plus 3 SD. The serum of a patient with a known titer of specific IgE to cat serum albumin determined by Phadia CAP-System (Phadia, Uppsala, Sweden) are serially diluted to generate a standard curve on which all determinations of specific IgE were determined. Appended Example 6 provides for illustrative data in this respect.

Furthermore, nucleic acids, polypeptides/ proteins, antibodies of the present invention can also be spotted onto solid carriers, like chips.

Taking advantage of the herein provided means and methods a person skilled in the art is easily in the position to diagnose an allergic reaction to a Caviidae allergen or to determine a subject prone to an allergic reaction to Caviidae allergen.

In a further embodiment, the present invention relates to an *in vitro* method for determining allergenicity comprising the steps of (a) exposing a sample to the defined polypeptide(s) or antibody; and (b) determining immunological reaction or the formation of an immunological complex to the exposure of step (a).

In this *in vitro* method, said immunological reaction to be assayed may comprise an increased titer of immunglobulines, preferably IgE, IgG4 or IgG1, activation of basophiles, an increased histamine release, leukotriene release, any mediator specifically released by an immune cell in an IgE, IgG4 or IgG1 mediated allergic reaction or whereby said formation of immunological complex is the formation of an antibody-antigen complex between either said polypeptide and an antibody or the above-mentioned antibody (like IgE, IgG4 or !gG1) and a corresponding antigen. An increased titer of immunglobulines and/or an increased histamine release is indicative for an immunological or allergic reaction. It is preferred that the immunological or allergic reaction is a reaction to Caviidae and a preferred immunoglobuline is IgE, IgG4 or IgG1.

The term "formation of an immunological complex" as used herein refers to the formation of an antibody-antigen complex between either the polypeptide provided herein and an antibody or the antibody and a corresponding antigen. Said immunological or allergic reaction can be a reaction to polypeptides/ proteins of Caviidae.

The term "allergenicity" as herein described, refers to the property of an individual of being allergenic.

In a preferred embodiment, the sample to be used in accordance with the present invention may, for example, be a biological sample, a medical sample or an environmental sample. The environmental sample(s) may be any sample from air or dust or from home or working place, such as carpet, tissue or filter samples. For instance, the biological or medical sample comprises cell(s) or tissue(s) taken, e. g. by the attending physician, from an individual. Such a biological sample may also be a blood sample, a serum sample and the like. But also skin sample or sputum and mucosal samples may be used. Exemplarily, but not limiting, the biological or medical sample to be analysed in context of the present invention may or may be derived from a patient or a subject suspected to suffer from or being prone to suffer from an allergy to (a) Caviidae allergen(s), e.g. skin, respiratory system or mucosa. Exemplarily, but not limiting, the biological or medical sample to be analysed in context of the present invention is or is derived from blood, plasma, white blood cells, urine, semen, sputum, cerebrospinal fluid, lymph or lymphatic tissues or cells, amniotic fluid, hair, hair and/or follicles, stem cells (embryonal, neuronal, and/or others) or primary or immortalized cell lines (lymphocytes, macrophages, or cell lines). Alternatively, a sample obtained, for example, from an animal of the Caviidae family may be used in the assessment of the allergenic potential of said animal, e.g. by binding of the herein described antibodies directed against the novel peptides/polypeptides of this invention to the Caviidae allergen.

The use of the herein provided Caviidae allergens/antigens in combination is envisaged in the means and methods of the present invention, wherein the combination of the novel Caviidae allergens of the present invention is particularly preferred. However, Cav p 3, Cav p 6 and/or *Cavia porcellus* allergen 1 a may also be used by itself or in combination with known guinea pig allergens like Cav p 1 or Cav p 2 (Fahlbusch (2002) loc.cit; Fahlbusch (2003) loc.cit). The explanations and definitions given herein above in context of the Caviidae allergens of the present invention, in particular in context of identifying variant sequences of the nucleic acid molecules/ polypeptides of the present invention also apply, mutatis mutandis, to known guinea pig allergens (e.g. Cav p 1 or Cav p 2) to be used in accordance with the present invention. The (recombinant) polypeptides may be applied e.g. for the immunization (immunotheapy) by oneself or in combination with each other, e.g. (r)Cav p 1 with (r)Cav p 2, (r)Cav p 1 with (r)Cav p 3 , (r)Cav p 1 with (r)Cav p 6, (r)Cav p 2 with (r)Cav p 3, (r)Cav p 2 with (r)Cav p 6, *(r)Cavia porcellus* allergen 1 a with (r)Cav p 1, (r)*Cavia porcellus* allergen 1a with (r)Cav p 2, (r)*Cavia porcellus* allergen 1a with (r)Cav p 3, *(r)Cavia porcellus* allergen 1a with (r)Cav p 6, *Cavia porcellus* allergen 1a with (r)Cav p 1, (r)Cav p 2 and (r)Cav p 3, *(r)Cavia porcellus* allergen 1 a with (r)Cav p 1 and (r)Cav p 2 or (r)Cav p 3, (r)Cav p 3 with (r)Cav p 6 or (r)Cav p 2 with (r)Cav p 3 and (r)Cav p 6. As shown in appended Example 6 and Example 8, recombinant polypeptides ((r)Cav p 2, (r)Cav p 3, (r)Cav p6, (r)*Cavia porcellus* allergen 1a) can be used for the immunization of mice sera to screen tissues and secretion products.

A kit comprising said vector, said recombinant host cell or the antibody as abov-mentioned. Said kit may also comprise primers of the present invention. The polypeptides (or fragments thereof) of the present invention may also be comprised in kits. The kits may be useful in diagnostic settings as well as in medical interventions. This kit can be used in diagnosis of allergic reaction or for the determination of a subject prone to an allergic reaction. The kit may comprise further components, like means of detection (like secondary antibodies, labeled antibodies). It may comprise negative or positive control samples. The kit (to be prepared in context) or the methods and uses of the invention may further comprise or be provided with (an) instruction manual(s), for example how carry out the diagnostic assays and methods provided herein, like the detection methods for allergic responses towards the antigens/allergens provided herein . For example, said instruction manual(s) may guide the skilled person (how) to use the polypeptides in the detection of immunoglobulin titers (like IgE-, IgG- titers) in patient samples, in accordance with the present invention. Particularly, said instruction manual(s) may comprise guidance to use or apply the herein provided methods or uses. The kit (to be prepared in context) may further comprise substances/chemicals and/or equipment suitable/required for carrying out the methods and uses of this invention. For example, such substances/chemicals and/or equipment are solvents, diluents and/or buffers for stabilizing and/or storing (a) compound(s) required for the antigens/allergens provided herein or the polynucleotides encoding the same or specific antibody molecules directed against the herein disclosed novel antigens/allergens.

In a further embodiment, the present invention relates to a method for identifying molecules which are capable of interacting with the said polypeptide of comprising (a) producing cells which express said polypeptide; (b) contacting the polypeptide produced in step (a) with a test sample potentially containing said molecule; and (c) identifying among these molecules the molecules which are capable of interacting with said polypeptide.

The term "identifying molecules", as used herein, refers to a method for identifying a molecule compound, which may be performed by using the cell culture medium or the supernatant thereof into which the polypeptide has been secreted. Also, the polypeptide may be purified to a degree which is necessary by conventional techniques in order to obtain significant results in said method. In a preferred embodiment, the polypeptide may be used in an immobilized form, i.e. directly or indirectly via the linkage over suitable intermediate molecules (e.g. peptide linkers, antibodies and the like) attached to a solid support.

The term "contacting the polypeptide with a test sample", as used herein, refers to a method of evaluating a compound for the ability to interact with one of the defined polypeptide. The method includes: contacting the compound with the polypeptide; and evaluating the ability of the compound to interact with, e. g., to bind or form a complex with, the defined polypeptides as shown in Seq ID Nos 2, 4, 6 and 20. This method can be performed *in vitro,* e. g., in a cell free system, or *in vivo,* e. g., in a two-hybrid interaction trap assay. This method can be used to identify naturally-occurring molecules that interact with the above-mentioned polypeptides. It can also be used to find natural or synthetic inhibitors of the above-mentioned polypeptides.

In particular embodiments of the invention assays are used wherein the polypeptide is immobilized on a solid support. A variety of appropriate solid supports is known to persons of skill in the art. Exemplary supports may include beads, such as resin beads, slides, glass and plastic wells, test tubes, and plates.

The attachment of the polypeptide to a solid support may facilitate performing the steps of the methods of the present invention, including, for example, contacting the polypeptide with a compound. Attachment to a solid support will also facilitate washing the polypeptide between steps. To a person of skill in the art, it will be apparent that any of the above described methods may comprise washing steps between, for example, the contact of the polypeptide with the compound and contact with a noxious substance. Further, washing after contacting the polypeptide with the compound may also be performed before determining, for example, the degree of fragmentation or binding or stability of the polypeptide. The present invention also encompasses embodiments comprising at least one or multiple washing steps between at least one or more of the steps of the above-mentioned methods.

The present invention is further described by reference to the following non-limiting figures and examples.

The Figures show:
- **Figure 1:**: Detection of IgE reactive proteins in sub-maxillary gland extract by immunoblot.
- **Figure 2:**: Detection of IgE reactive proteins in harderian gland extract by immunoblot.
Remark: Numbers of patient sera do not correspond to the numbering used in Figures 13 and 15.
- **Figure 3:**: 2D-gel of harderian gland extract stained with Coomassie Blue.
- **Figure 4:**: Cav p 2 cDNA nucleotide sequence and deduced amino acid sequence. In the DNA sequence (SEQ ID NO: 5), the corresponding start codon is at nt 53-55 and stop codon at nt 563-565 is underlined, cDNA coding for signalpeptide is in italic (nt 53 to 100), cDNA coding for mature Cav p 2 is at nt 101-565; in the amino acid sequence (SEQ ID NO: 6), the signal peptide is marked up in italic, the determined N-terminal sequence is underlined.
- **Figure 5:**: Analysis of purified (r)Cav p 2 (lane 1), (r)Cav p 3(lane 2) and (r)Cav p 6 (lane 3) by SDS_PAGE and silver staining under denaturing (+) and non-denaturing (-Mercaptoethanol) conditions.
- **Figure 6:**: Detection of Cav p 2 in different guinea pig protein extracts by immunoblot using a polyclonal mouse anti-(r)Cav p 2 serum.
- **Figure 7:**: 2D gel of submaxillary gland extract stained with Coomassie Blue. Identification of Cav p 3 by N-terminal sequencing
- **Figure 8:**: Cav p 3 cDNA nucleotide sequence and deduced amino acid sequence. In the DNA sequence (SEQ ID NO: 1), the corresponding start codon is at nt 59-61 and stop codon at nt 569-571 is underlined, cDNA coding for signalpeptide is in italic (nt 59 to 103), cDNA coding for mature Cav p 3 is at nt 104-571; in the amino acid sequence (SEQ ID NO: 2), the signal peptide is marked up in italic, the determined N-terminal sequence is underlined.
- **Figure 9:**: Detection of Cav p 3 in different guinea pig protein extracts by immunoblot using a polyclonal mouse anti-rCav p 3 serum.
- **Figure 10:**: Cav p 6 cDNA nucleotide sequence and deduced amino acid sequence. Amino acids marked up bold represent putative isoforms which have been detected by sequencing different cDNA clones.
In the DNA sequence (SEQ ID NO: 3), the corresponding start codon is at nt 92-94 and stop codon at nt 635-637 is underlined, cDNA coding for signalpeptide is in italic (nt 92 to 148), cDNA coding for mature Cav p 6 is at nt 149-637; in the amino acid sequence (SEQ ID NO: 4), the signal peptide is marked up in italic, the determined N-terminal sequence is underlined.

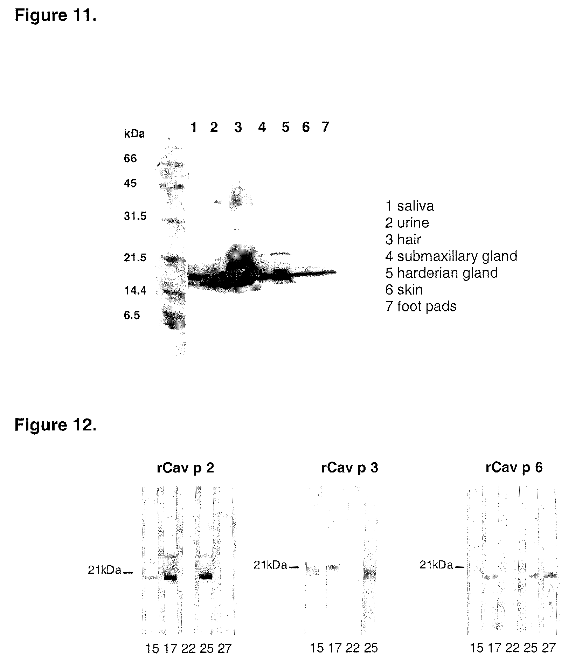

- **Figure 11:**: Detection of Cav p 6 in different guinea pig protein extracts by immunoblot using a polyclonal mouse anti-rCav p 6 serum.
- **Figure 12:**: (r)Cav p 2, (r)Cav p 3 and (r)Cav p 6 are recognized by patient IgE antibodies in immunoblot No 15, 17, 22, 25 and 27 are individual sera of patients allergic to guinea pig.

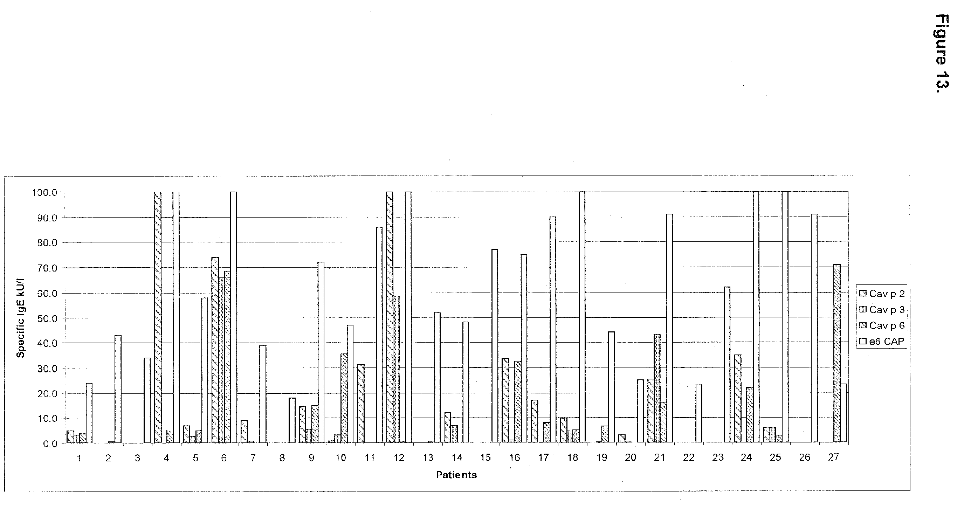

- **Figure 13:**: IgE reactivity to rCav p 2, rCav p 3 and rCav p 6 assayed by ELISA in 27 patients. Values are compared to ImmunoCAP e6 (Phadia, Uppsala, Sweden).
Remark: Values are cut at 100 kU/I to compare to CAP results.

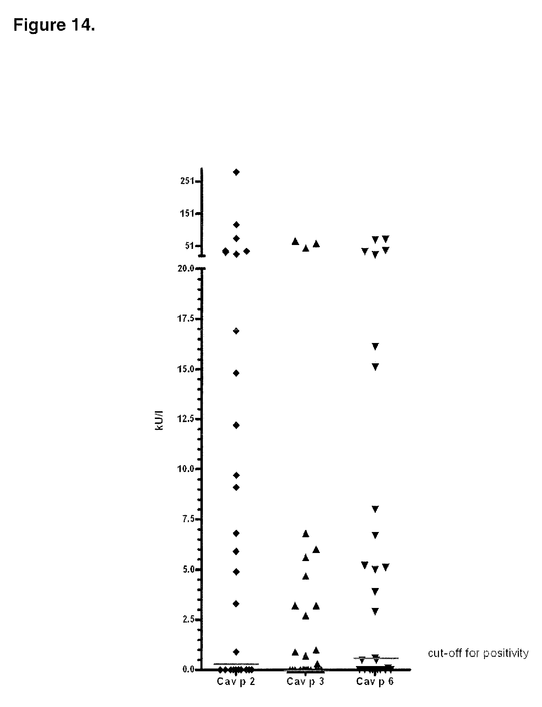

- **Figure 14:**: IgE reactivity to (r)Cav p 2, (r)Cav p 3 and (r)Cav p 6 assayed by ELISA in 27 patients.

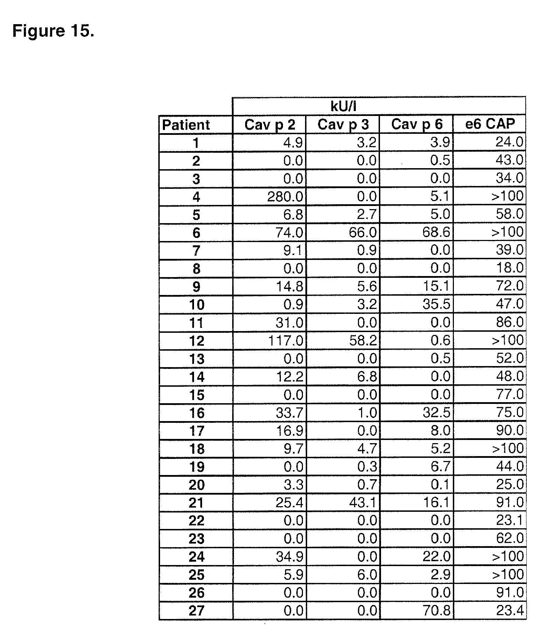

- **Figure 15:**: IgE reactivity of guinea pig allergic patients to recombinant allergens (r)Cav p 2, (r)Cav p 3 and (r)Cav p 6 in comparison to ImmunoCAP e6.

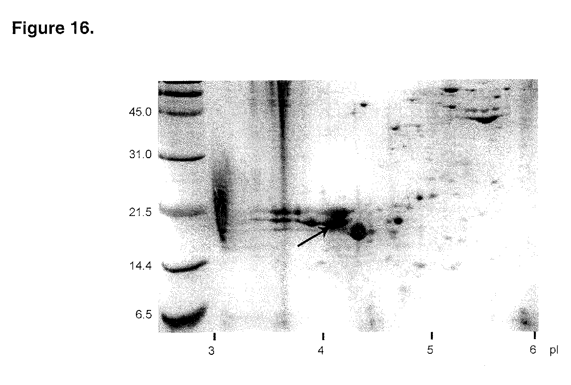

- **Figure 16:**: Twodimensional SDS-PAGE gel (20%) of harderian gland extract stained with Coomassie Blue.

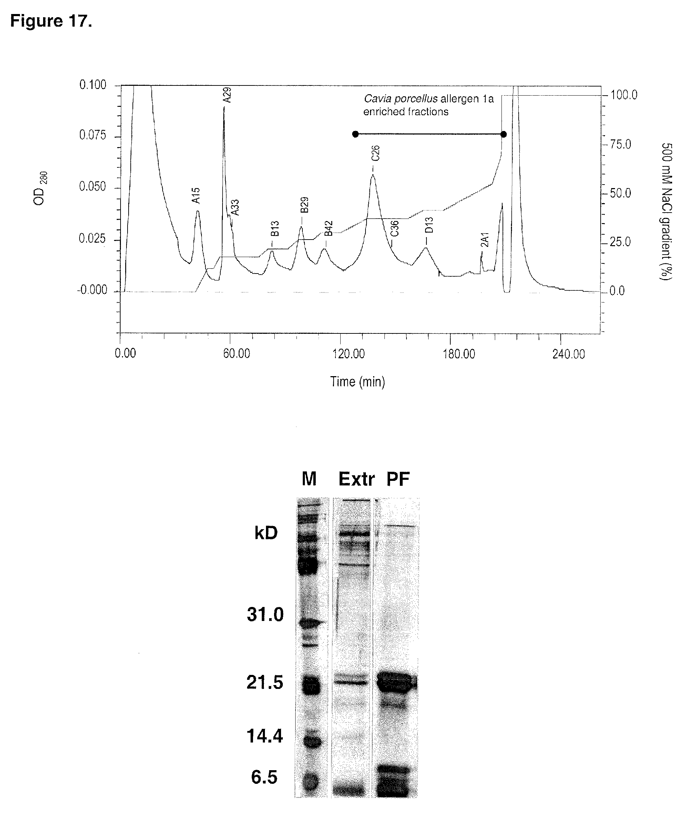

- **Figure 17:**: Separation of harderian gland extract by anion exchange chromatography on Resource Q. Harderian gland extract and pooled enriched fractions are analysed by silver stained 15% SDS-PAGE. (M, molecular weight marker; Extr, harderian gland extract; PF, Cavia porcellus allergen 1a enriched fractions).

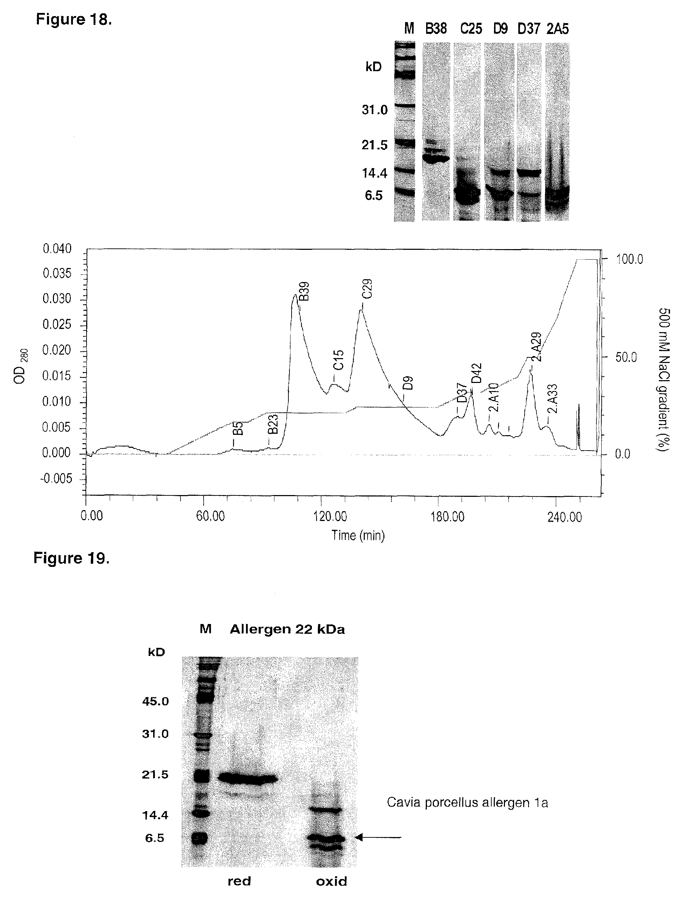

- **Figure 18:**: Isolation of *Cavia porcellus* allergen 1a by anion exchange chromatography on Mono Q. Analysis of individual fractions by silver stained 15 % SDS-PAGE under denaturing conditions (M, molecular weight marker; B38-2A5, individual eluted fractions).
- **Figure 19:**: Analysis of the 22 kD allergen under reducing (red) and non-reducing conditions (oxid). Fractions containing the 22 kD allergen isolated from harderian gland by anion exchange chromatography were analysed by 15 % SDS-PAGE followed by silver staining.
- **Figure 20:**: *Cavia porcellus* allergen 1 a cDNA nucleotide sequence and deduced amino acid sequence. In the DNA sequence (SEQ ID NO: 19), the corresponding start codon is at nt 55-57 and stop codon at nt 553-555 is underlined, cDNA coding for signalpeptide is in italic (nt 55 to 108), cDNA coding for mature *Cavia porcellus* allergen 1 a is at nt 109-555; in the amino acid sequence (SEQ ID NO: 20), the signal peptide is marked up in italic, the determined N-terminal sequence is underlined.

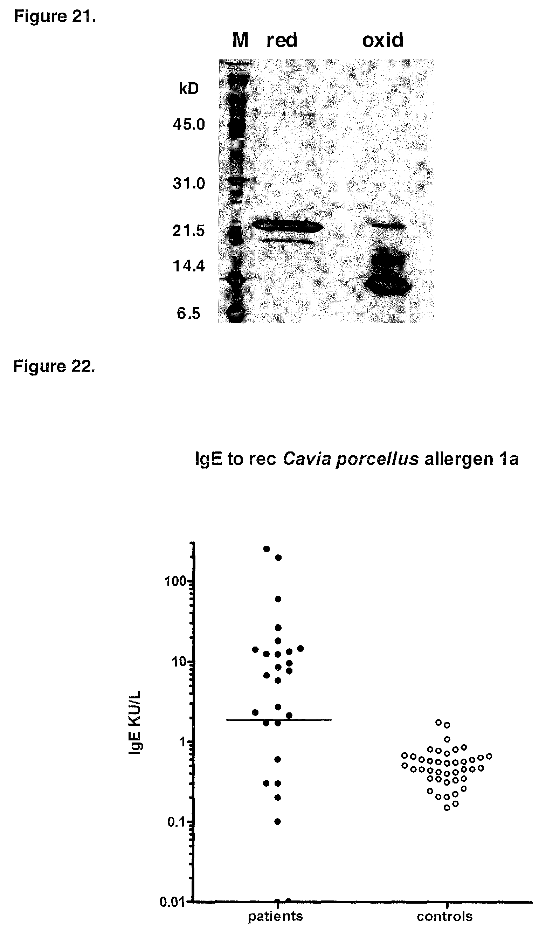

- **Figure 21:**: Analysis of r*Cavia porcellus* allergen 1 a by 15% SDS-PAGE and silver staining. Migration under reducing (red) and non-reducing conditions (oxid). M, molecular weight marker.
- **Figure 22:**: IgE binding to *Cavia porcellus* allergen 1 a analysed by ELISA. Specific IgE were determined in a group of 27 patients and 41 controls. Controls are a group of 19 IgE neg patients as well as 22 IgE positive patients without allergy to mammals. The line marks the cutoff for positivity as calculated from the average of the IgE neg group plus 3 SD.

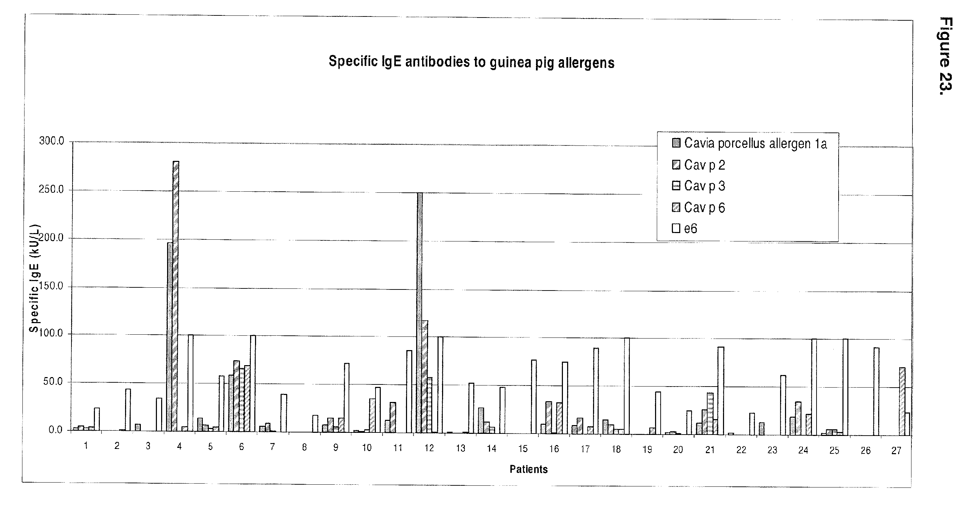

- **Figure 23:**: IgE reactivity to recombinant *Cavia porcellus* allergen 1 a, Cav p 2, Cav p 3 and Cav p 6 assayed by ELISA in 27 patients. Values are compared to ImmunoCAP e6 (Phadia, Uppsala, Sweden).

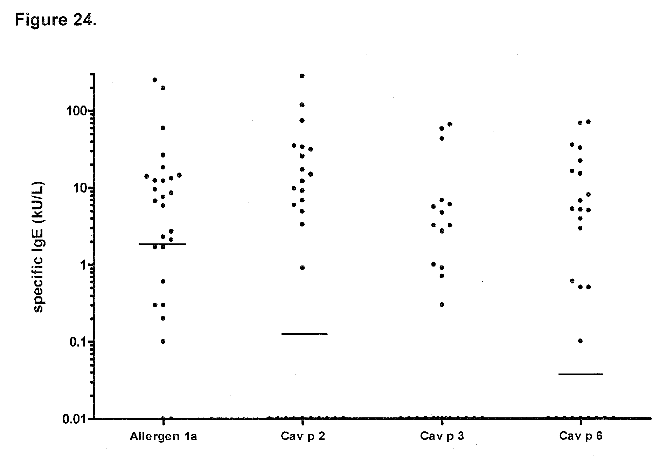

- **Figure 24:**: Specific IgE to recombinant *Cavia porcellus* allergen 1 a, Cav p 2, Cav p 3 and Cav p 6 assayed by ELISA. Specific IgE were determined in 27 guinea pig allergic patients. Lines mark the cutoff for positivity which was determined in 19 IgE negative controls by calculating the average of the IgE neg group plus 3 SD.

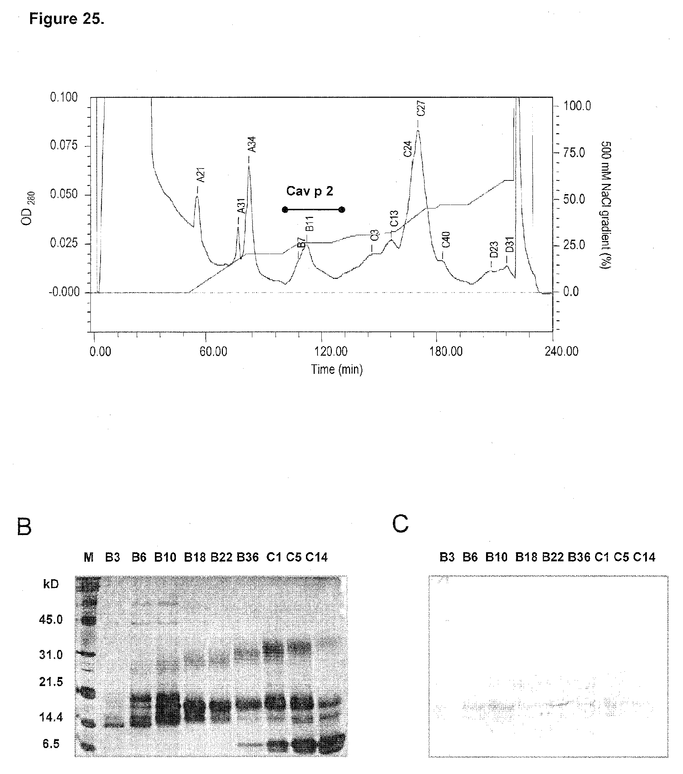

- **Figure 25:**: Purification of Cav p 2 from hair protein extract. A) Separation of soluble hair proteins by anion exchange chromatography. B) Analysis of individual fractions by 15% SDS-PAGE gel followed by silver staining. The gel was run under non-reducing conditions. C) Analysis of individual eluted fractions by immunoblot. A polyclonal mouse anti-Cav p 2 serum was used for detection of Cav p 2.

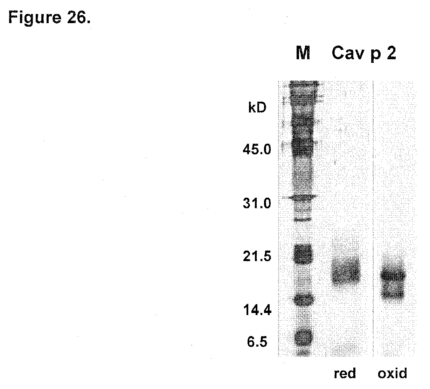

- **Figure 26:**: Analysis of purified Cav p 2 by 15% SDS-PAGE followed by silver staining. Migration under reducing (red) and non-reducing (oxid) conditions. M, molecular weight marker.

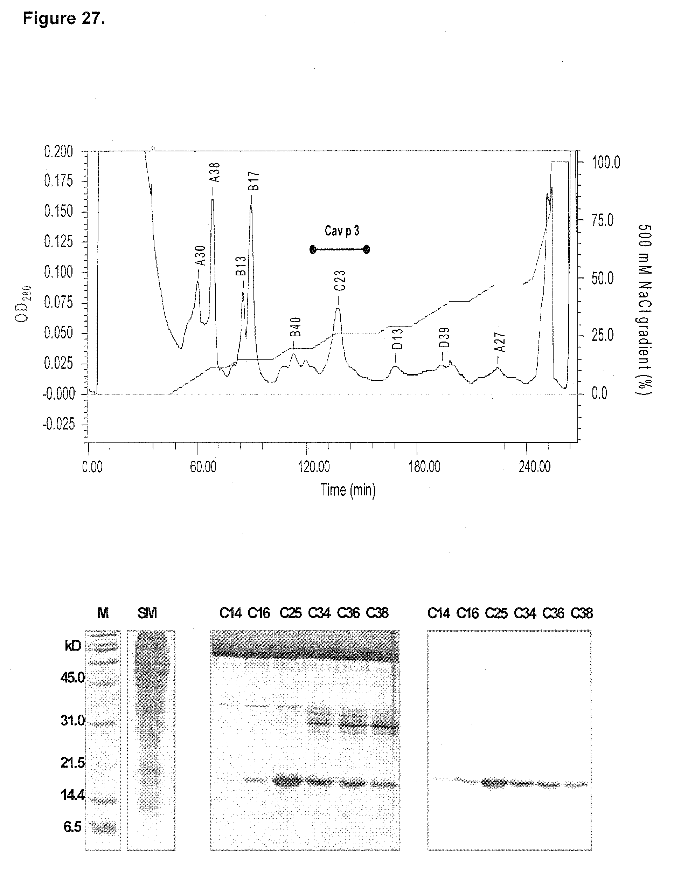

- **Figure 27:**: Purification of Cav p 3 from submaxillary gland extract. A) Separation of soluble submaxillary gland proteins by anion exchange chromatography. B) Analysis of submaxillary gland extract by 15% SDS-PAGE before loading on the column. The gel was run under reducing conditions and stained with Coomassie Blue. M, molecular weight marker, SM, submaxillary gland extract. C) Analysis of individual fractions by 15% SDS-PAGE followed by silver staining. The gel was run under non-reducing conditions. D) Analysis of individual eluted fractions by immunoblot. A polyclonal mouse anti-Cav p 3 serum was used for detection of Cav p 3.

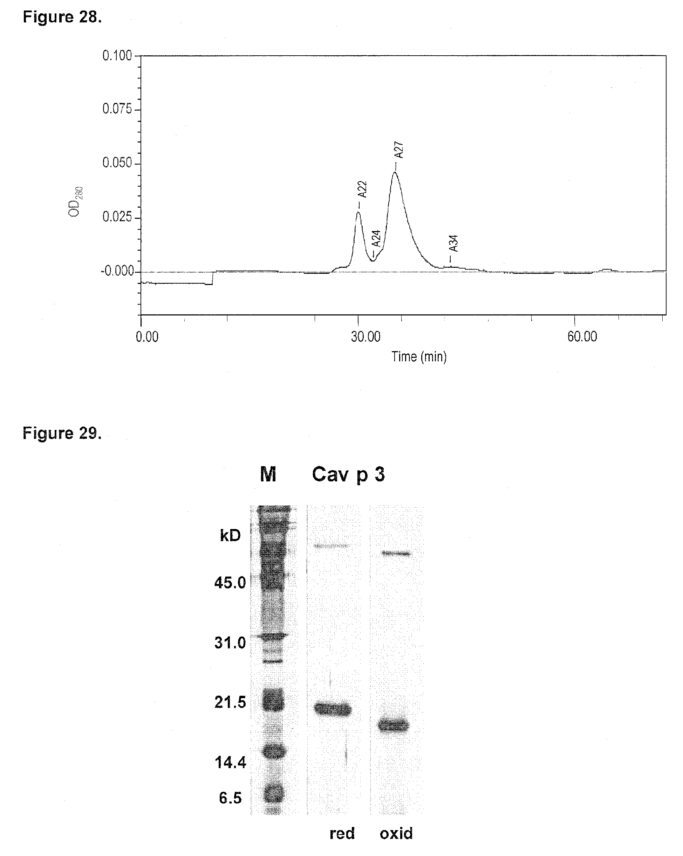

- **Figure 28:**: Purification of Cav p 3 by gel filtration.
Cav p 3 enriched fractions obtained by anion exchange chromatography were pooled and purified by gel filtration.
- **Figure 29:**: Analysis of purified Cav p 3 by 15% SDS-PAGE followed by silver staining. Migration under reducing (red) and non-reducing (oxid) conditions. M, molecular weight marker.

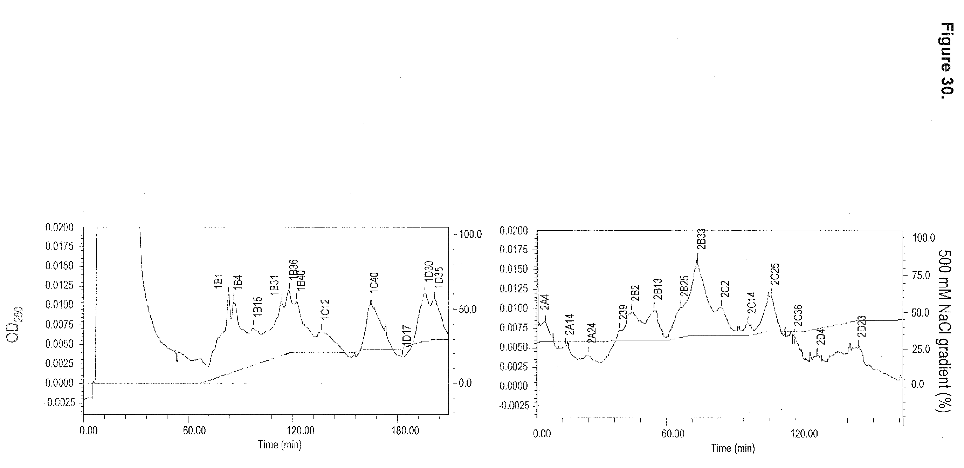

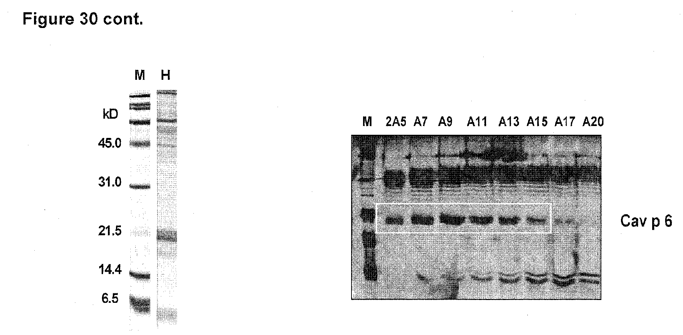

- **Figure 30:**: Purification of Cav p 6 by anion exchange chromatography.
A) Separation of soluble harderian gland proteins by anion exchange chromatography. B) Analysis of harderian gland extract by 15% SDS-PAGE before loading on the column. The gel was run under reducing conditions and stained with Coomassie Blue. M, molecular weight marker, H, harderian gland extract. C) Analysis of individual fractions by 15% SDS-PAGE gel followed by silver staining. The gel was run under non-reducing conditions.

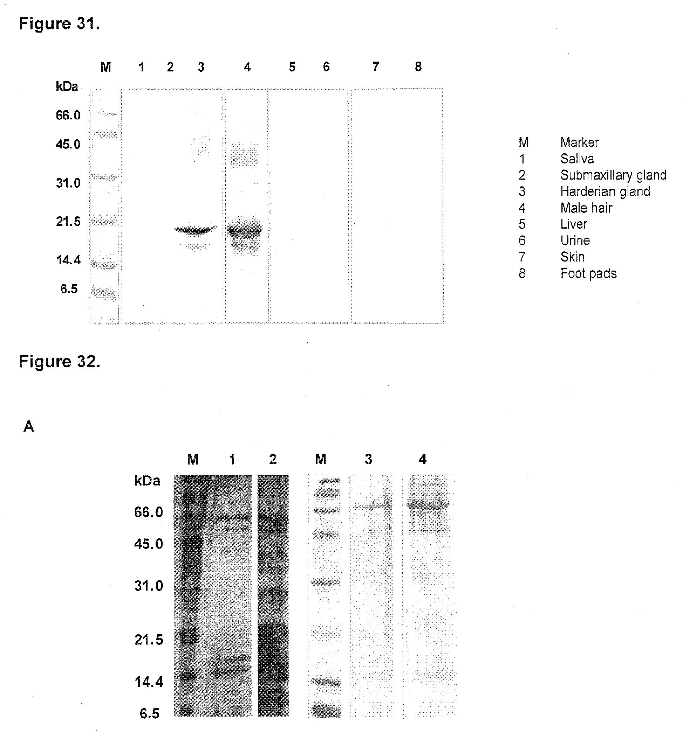

- **Figure 31:**: Detection of *Cavia porcellus* allergen 1a in different guinea pig protein extracts by immunoblot using an anti *-Cavia porcellus* allergen 1a serum.

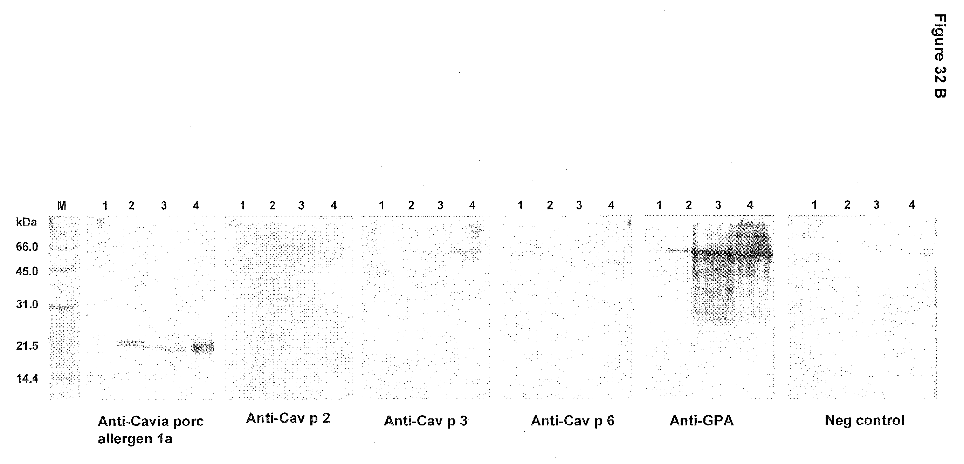

- **Figure 32:**: Analysis of skin prick test solutions from different suppliers by SDS-PAGE and immunoblot. A) Prick test solutions from 4 suppliers were analysed by 15% SDS-PAGE followed by silver (500 ng of samples 1 and 2) or Coomassie staining (12 µg of samples 3 and 4); B) Detection of *Cavia porcellus* allergen 1 a in prick test solutions using a polyclonal mouse serum raised against *Cavia porcellus* allergen 1 a, Cav p 2, Cav p 3, Cav p 6 and GPA. M, molecular weight marker; 1-4, prick test solutions from 4 different suppliers.

The following Examples illustrate the invention:

### Example 1: Material and methods

### Source materials and extract preparation of soluble proteins:

All extracts were derived from male Dunkin Hartley guinea pigs which were killed by intra-cardiac injection of barbiturate solution.

Unwashed hair was clipped off using scissors, cut into pieces of 2 mm and shaken at 4°C overnight with Phosphate buffered saline containing a mixture of protease inhibitors (*Protease Inhibitor Cocktail Tablets,* Roche, Mannheim, Germany). The suspension was centrifuged for 30 min at 12 000g at 4°C to remove hair and solid particles. The supernatant was concentrated with an Ultrafree concentrator (cut off 5000 MW, Millipore, Bedford, MA) and frozen at -20 °C.

After hair clipping, the entire skin (extraneous fat was excised) was collected. The skin was cut into 3mm pieces and degreased by shaking overnight in diethyl ether (1:10, v:v) at 4°C, dried at room temperature and extracted.

All solid source materials was cut into 3mm pieces, frozen in liquid nitrogen, grinded with mortar and pestle to fine powder and suspended in Single Detergent Lysis buffer (50mM TRIS-HCI pH 8, 150mM NaCI, 1% Triton X-100, protease inhibitors). The extract was centrifuged for 30min at 12 000xg at 4°C to remove the insoluble particles. The supernatant was concentrated with an Ultrafree concentrator (cut off 5000 MW, Millipore).

Serum was collected by intra-cardiac blood puncture. Urine was drawn directly out the bladder with a needle and syringe.

Salivation was induced with pilocarpine nitrate salt (Sigma, St. Louis, MO) (1ml of a 4% solution in the mouth) before anesthesia with a formulation of ketamine and domitor (subcutaneously injection).

### Patient sera

Sera of patients with allergy to guinea pigs were selected for this study. Selection was based on clinical history, rhinitis and/or asthma, positive skin prick test results to guinea-pig commercial allergen extracts and positive specific IgE results (> 17 kU/I) to Caviidae (e6) using CAP system (Phadia, Uppsala, Sweden). Sera of 19 IgE negative patients were collected as controls for specific IgE determination in ELISA. Another control group consisted of 22 IgE positive patients allergic to pollen and/or mite with total IgE ranging from 16 to 2051 kU/L.

### SDS-PAGE and allergen detection by specific IgE (human) or IgG (mouse) antibodies in immunoblot

The different extracts and purified proteins were studied by SDS-PAGE gels under reducing or non-reducing conditions. Proteins were detected with Coomassie stain (*GelCode Blue Stain reagent,* Pierce, Rockford, IL) or silver stain (*SiverSNAP stain kit II,* Pierce). For immunoblotting, proteins were transferred onto a PVDF membrane with TRIS-HCI-glycine buffer (25mM TRIS-HCI, 192mM glycine, 20% methanol) in semi-dry conditions (14V, 900mM, 35 min). To reduce non-specific binding, blotted membranes were incubated in blocking buffer (TRIS-HCl buffered saline/ tween-20 0.05%, 2% cold water fish gelatine (Sigma), for 2 hours.

PVDF membranes were incubated overnight with patient sera diluted one-tenth in blocking buffer or mouse anti-sera (1/5000) raised against guinea pig allergens. Control strips were incubated with buffer alone. Strips were washed three times with TBS/Tween-20 0.05%. Bound IgE were detected with anti-human IgE antibody (1:1 000) labelled with alkaline phosphatase (Sigma). Bound mouse IgG were detected with anti-mouse IgG antibody (1:10 000) labelled with alkaline phosphatase (Sigma). Blots were developed by addition of nitro blue tetrazolium/5-bromo-4-chloro-3-indolyl-phosphate (NBT/BCIP) (Promega, Madison, WI).

### 2D-gels

2D-gels were performed according to the conditions described by the manufacturer (Bio-Rad, Hercules, CA). The first dimension isoelectro-focusing (IEF) was carried out on immobilized pH gradient (IPG) strips, pH 3-10 or 3-6. Extracts (150-450µg of soluble proteins) were precipitated by TCA/acetone and resuspended in rehydratation buffer containing 8M urea, 0.5% CHAPS, 10mM DTT, 0.2% Bio-Lytes 3-10.

Proteins were separated at 4000V and 50mA for 6 hours according to their pl. After a first incubation for 15 min in equilibration buffer (375mM TRIS-HCI-HCI pH8.8, 6M urea, 2%SDS, 20% glycerol and 130mM DTT) and a second equilibration (15 min) with iodoacetamide in the SDS equilibration buffer (without DTT), the strips were loaded onto a SDS-PAGE for separating the proteins according to their molecular weight. The 2D-gels were stained with GelCode Blue Stain reagent (Pierce) or blotted onto PVDF membrane (Millipore) in order to perform an immunoblotting or N-terminal sequencing.

### Purification and analysis of guinea pig hair proteins by anion exchange chromatography

15 mg of soluble hair proteins were dialysed into 20mM TRIS-HCI , pH 8 (buffer A) and were loaded onto a RESOURCE Q column (1ml, GE Healthcare, Buckinghamshire, UK). Unbound material was removed by washing with buffer A. Bound proteins were eluted by a linear gradient of 0 - 0.5 M NaCl in 20mM Tris-HCl, pH8 (buffer B). Eluted fractions were first analysed by SDS-PAGE followed by silver staining, than by immunoblotting with patient serum. IgE reactive bands were analysed by N-terminal sequencing.

### Purification of Cavia porcellus allergen 1a by chromatography

Harderian gland proteins were extracted as described above and dialyzed with 20mM TRIS-HCI, pH 8. 2x25 mg of extract was loaded onto a RESOURCE Q column (1ml, GE Healthcare, Buckinghamshire, UK). Bound proteins were eluted by a linear gradient of 0-500 mM NaCl in 20mM TRIS-HCI, pH8. Eluted fractions were first analysed by SDS-PAGE followed by silver staining. Fractions containing *Cavia porcellus* allergen 1a were pooled and dialyzed with 20 mM Piperazine, pH 5.5 (Sigma). A total of 1.8 mg enriched *Cavia porcellus* allergen 1a were further separated by ion exchange chromatography (Mono Q 1 ml, GE Healthcare, Buckinghamshire, UK) using a gradient of 0-500 mM NaCl in 20 mM Piperazine, pH 5.5. Individual fractions were selected for N-terminal sequencing.

### Amino acid sequencing

The N-terminal amino acid sequences of purified proteins were determined by automated Edman degradation on PROCISE 49X HT Protein sequencer (Applied Biosystems, Foster City, CA). Liquid samples were desalted and adsorbed onto a PVDF membrane by the ProSorb column. Alternatively proteins were separated by SDS-PAGE and transferred onto PVDF membranes by semi-dry transfer using CAPS-buffer (CAPS 10mM, MeOH 20%). Proteins were stained on the membrane with Gelcode® Blue (Pierce) overnight. After destaining with methanol 50%, acetic acid 7%, the membrane was washed for 5-6h in ultrapure water and dried. Bands of interest were cut out using a clean scalpel and stored at -20°C until sequencing.

### RNA extraction

RNA in tissues was stabilized in *RNA Stabilization Reagent* (Qiagen, Hilden, Germany). After 24h at 4°C, the reagent was removed and samples were stored at-80°C.

A total of 200 mg of tissue was used for RNA extractions. For disruption, the glands were thawed, cut in fine pieces, ground with a mortar and pestle. The powder was suspended in the lysis buffer from the *RNeasy lipid tissue midi kit* (Qiagen). The suspension was homogenized by passing through a needle of 20-gauge at least 10 times. The RNA extractions were performed according to the protocol and materials provided by the *RNeasy lipid tissue midi kit* (Qiagen). The quality of mRNA was checked by formaldehyde agarose gel 1%.

### Cloning of cDNA coding for Cav p 2, Cav p 3 and Cav p 6

The cDNA were obtained according to the conditions and materials of *BD SMART RACE cDNA amplification kit* (BD Biosciences). 3' and 5' RACE was performed with sub-maxillary and harderian gland total RNA.

Using the degenerated primers, the PCR products were obtained after a touch down from 66°C to 58°C for 12 cycles for Cav p 2, from 60°C to 54°C for Cav p 3 and from 62°C to 57°C for Cav p 6. These first cycles were followed by 25 cycles with annealing temperatures of 58°C, 53°C and 57°C respectively. The PCR products were analysed by 1.5% agarose gel.

PCR products corresponding to the expected molecular weight were cloned into pCR2.1-topo for sequence analysis. Sequences obtained allowed to design specific reverse primers for amplification of the 5'end. After final sequence analysis, specific primers were used for cloning the open reading frame into the expression vector pQE60 (Qiagen). DNA sequence was confirmed again by sequencing.

### Cloning of cDNA coding for Cavia porcellus allergen 1a

The cDNA was obtained according to the conditions and materials of *BD SMART RACE cDNA amplification kit* (Clontech-Takara, St-Germain-en-Laye, France). 3' and 5' RACE was performed with harderian gland total RNA.

12 forward and 8 reverse degenerate primers (table 1 in example 9) were designed on the N-terminal amino acid sequence obtained from *Cavia porcellus* allergen 1 a. Using all possible combinations of these degenerate primers, PCR products of the expected size of 95 bp were obtained with primers Cavp-MQ2 and Cavp-MQ7rev. The 95 bp DNA fragment was cloned into pCR2.1-topo (Invitrogen, Merelbeke, Belgium) for sequence analysis. Sequences obtained allowed to design specific forward and reverse primers for amplification of the 3' and 5'end (table 1 in example 9). After final sequence analysis, specific primers were used for cloning the open reading frame into the expression vector pQE60 (Qiagen). DNA sequence was confirmed again by double strand DNA sequencing.

### DNA sequencing

The DNA sequencing was performed by the method of Sanger using a LI-COR 4300 DNA analyser (LI-COR Biosciences, Lincoln, Nebraska).

### Expression and purification of recombinant Cavia porcellus allergen 1a, Cav p 2, Cav p 3 and Cav p 6

cDNAs coding for mature proteins *Cavia porcellus* allergen 1 a, Cav p 2, Cav p 3 and Cav p 6 were subcloned into pQE-60 (Qiagen) and were expressed in *E.coli* M15 and Rosettagami cells as recombinant proteins with a C-terminal 6 Histidine tag. The recombinant proteins were purified by affinity chromatography (HisTrap™ HP, GE Healthcare) under native conditions according to the instructions of the manufacturer. Recombinant proteins were eluted by imidazol gradient in 20 mM NaH₂PO₄, 500 mM NaCl pH 8 and dialyzed with PBS. The purity of the recombinant proteins was analysed by SDS-PAGE. Identity was confirmed by N-terminal sequencing.

### Mice immunisation and polyclonal serum against (r)Cav p 2

The recombinant Cav p 2 protein was mixed with aluminium hydroxide. Two mice were vaccinated by intra-peritoneal injections with 20µg of coupled (r)Cav p 2 /injection. The mouse immunization was performed following a program of 38 days (first injection = 0 day, first boost = 14^{th} day, second boost = 28^{th} day, harvest at day 38). The sera from both mice were obtained by intra-cardiac blood puncture, incubation of blood for 30 min at room temperature and centrifugation.

### Mice immunisation and polyclonal serum against (r)Cavia porcellus allergen 1a, (r)Cav p 3, (r)Cav p 6

50ug of purified recombinant proteins were suspended in adjuvant (complete Freund's for (r)Cav p 3 and aluminium hydroxyde for (r)Cav p 6 and for (r)*Cavia porcellus* allergen 1 a). Three mice were vaccinated intra-peritoneally. 3 boosts at 3 weeks of interval were administered using 50 ug of the same proteins in adjuvant (incomplete Freund's for (r)Cav p 3 and aluminium hydroxyde for (r)Cav p 6 and (r) *Cavia porcellus* allergen 1 a). Sera were harvested 3 to 10 days after the last boost by intra-cardiac blood puncture. Blood was incubated for 30 min at room temperature and centrifuged.

### Quantification of specific IgE antibodies to (r)Cavia porcellus allergen 1a, (r)Cav p 2, (r)Cav p 3 and (r)Cav p 6

Purified recombinant *Cavia porcellus* allergen 1 a, Cav p 2, Cav p 3 and Cav p 6 were coated overnight at 4°C at a concentration of 5 µg/ml in PBS to microtiter plates. Plates were blocked with blocking buffer (3% bovine serum albumin (BSA, Sigma) in TBS/0.05% Tween-20 for *Cavia porcellus* allergen 1 a and 2% cold water fish gelatin (CWFG, Sigma) for Cav p 2, 3 and 6) for 30 min at room temperature. Patient sera were serially diluted into blocking buffer and were added to the microtiter plates for 2 hours. Binding of IgE was measured by incubation for 1 hour with biotin-labeled antihuman IgE antibodies (1/2000); Southern Biotechnology, Birmingham, Ala) followed by an incubation for 30 min with alkaline phosphatase-labeled streptavidine (1/1000; BD Biosciences Pharmingen, Erembodegem, Belgium). p-Nitrophenyl phosphate was added as substrate, and plates were left in the dark before OD was read at 405 nm. The cutoff was determined as the mean of the negative controls plus 3 SD. The serum of a patient with a known titer of specific IgE to cat serum albumin determined by Phadia CAP-System (Phadia, Uppsala, Sweden) was serially diluted to generate a standard curve on which all determinations of specific IgE were determined.

### Example 2: Screening of different tissues and secretions from guinea pig.

Screening of different tissues and secretions from guinea pig showed IgE binding proteins ranging from 6 kDa to 66 kDa. We could confirm that hair, saliva and urine are the most important allergen sources (Swanson (1984), Am Rev Respir Dis 129, 844-849; Walls (1985), Clinical Allergy 15, 241-251; Walls (1985), Clinical Allergy 15, 535-546).

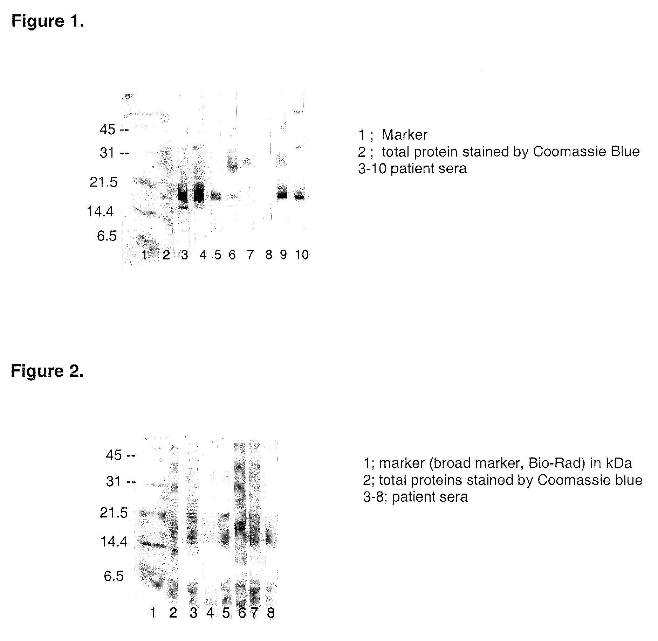

In order to characterize the allergens, hair proteins were purified using anion exchange chromatography. IgE immunoreactive fractions were further analysed by N-terminal amino acid sequencing. In a next step, protein extracts of different tissues were prepared and they were screened for allergen expression by IgE immunoblot. The submaxillary gland as well as the harderian gland were found to contain allergens in the 5-66 kDa range (Figure 1 and 2).

### Example 3: Characterization, cloning and expression of Cav p 2.

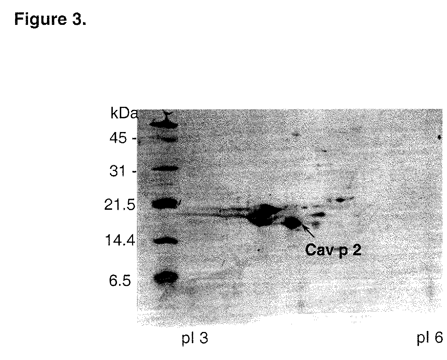

Harderian gland proteins were analysed by 2D gel electrophoresis. IgE immunoreactive spots were isolated for N-terminal aa sequencing (Figure 3).

The N-terminal sequence of the protein spot at 17 kD corresponds to the N-terminal sequence published by Fahlbusch (2003), Allergy 58, 629-634 for Cav p 2.

### DSIDYSKVPGNWRTI

Backtranslation of the N-terminal aa sequence allowed to design degenerate primers Cav p 2 a-d:
Seq ID No:7: Cav p 2a: 5' AAR GTN CCN GGN AAC TGG CG 3'
Seq ID No:8: Cav p 2b: 5' AAR GTN CCN GGN AAT TGG CG 3'
Seq ID No:9: Cav p 2c: 5' AAR GTN CCN GGN AAC TGG AG 3'
Seq ID No:10: Cav p 2d: 5' AAR GTN CCN GGN AAT TGG AG 3'

A degenerate primer of SEQ ID No: 10 may also comprise a shorter sequence starting from AAR to TGG with 18 nucleotides. However, the skilled person is in a position to modify and amend primers and primer sequences as shown in SEQ ID No: 10, for example, by elongation and/or shortening of the sequences or distinct nucleotide exchanges. A particular primer to be used are e.g. SEQ ID NOS: 33 - 40 (Cavp-MQ1 rev to Cavp-MQ8rev).

Such an exchange needs to lead to a complementary strand sequence that is still capable to specifically hybridize under conditions disclosed herein to its corresponding sequence on the corresponding complementary strand.

Oligo Cavp 2b yielded a 800 bp product from submaxillary gland extract. Sequence analysis of the clones obtained identified the cDNA as Cav p 2. A specific reverse primer (Seq ID No:11: Cavp2rev GTGTTTTCACCAGCGTAGTCCACAG) was designed to amplify the 5'end using 5'RACE. The combination of the 5' and 3' amplifications revealed an open reading frame of 512 nucleotides. A hydropathy plot of the deduced aa sequence revealed a major hydrophobic region from aa 1 to 16. This region corresponds to a secretory signal peptide with a predicted cleavage site corresponding to the site determined by N-terminal sequencing. Cleavage of the signal peptide results in a 154 amino acid mature protein with a predicted molecular weight of 17.1 kDa and an isoelectric point of 4.27. No asparagin-linked glycosylation sites were detected. The official name 'Allergenic lipocalin from Cavia porcellus Cav p 2.0101' has been assigned to the protein by the I.U.I.S. allergen nomenclature sub-committee.

The Cav p 2 protein sequence (Figure 4) was aligned to the protein databases. Sequence alignments clearly identify the protein as lipocalin. It has an identity of 30-42 % to different odorant binding proteins from mouse and rat, 41% to hamster aphrodisin and to a lesser extent (25-30%) to different mammalian urinary proteins.

A cloning experiment using the specific Cav p 2 primers and RNA from the harderian gland yielded a number of clones identical to Cav p 2 cloned from submaxillary gland. A screening of a high number of clones showed some sequence diversity (up to 2% on aa level), possibly pointing out to the existence of different isoforms as already discussed by Fahlbusch (2003), Allergy 58, 629-634.

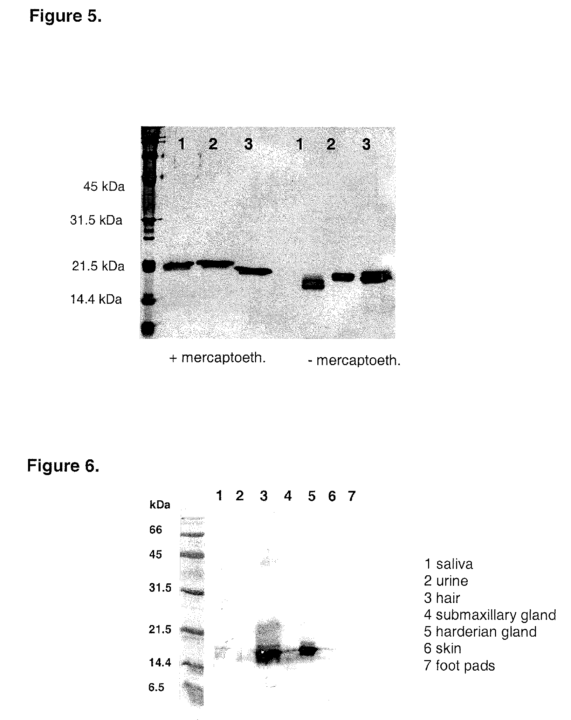

The cDNA coding for Cav p 2 was inserted into pQE-60 and Cav p 2 was expressed in *E.coli* M15 and Rosettagami cells with a C-terminal 6His tag (Figure 5, lane 1).

The serum of a mouse immunized with (r)Cav p 2 was used to screen several tissues and secretion products. This serum recognized strong protein bands in hair and harderian gland extracts. Reactivity to saliva and urine is weak in unconcentrated samples. Faint bands are detected in sub-maxillary gland and skin (Figure 6).

### Example 4: Characterization, cloning and expression of Cav p 3.

Submaxillary gland extract was used for IgE immunoblotting. An immunoreactive band at 19 kDa was isolated for N-terminal sequencing. The same extract was analysed by 2D electrophoresis and an immunoreactive spot at a pl of 5.2 was isolated and the N-terminal aa determined (Figure 7).

N-terminal sequence analysis showed the following results:

| | |
|---|---|
| 2D-gel : | **G Q T L D L P S E I N G Q W/V/G R/G T/A** |
| SDS-PAGE (1D): | **G/H Q T L D L P S E I N G Q W/H/V H T** |
| Published sequence Cav p 1: | **S E I N G D W N TIALSAD** |

The N-terminal sequence may also comprise:

| | |
|---|---|
| 2D-gel: | **G Q T L D P S E I N G Q W/V/G R/G T/A** |
| SDS-PAGE (1D): | **G/H Q T L D P S E I N G Q W/H/V H T** |
| Published sequence Cav p 1: | **S E I N G D W N TIALSAD** |

The following primers are used (amino acid D was changed into Q):
Seq ID No:12: Cav p 1e : 5' GAR ATH AAY GGN CAG TGG AAC AC 3'
Seq ID No:13: Cav p 1 f : 5' GAR ATH AAY GGN CAG TGG AAT AC 3'
Seq ID No:14: Cav p 1g : 5' GAR ATH AAY GGN CAA TGG AAC AC 3'
Seq ID No:15: Cav p 1 h : 5' GAR ATH AAY GGN CAA TGG AAT AC 3'

Oligo Cav p 1h amplified bands in the 700 to 900 bp range from submaxillary gland extract. Sequence analysis of the clones obtained identified the cDNA as Cav p 3 (Figure 8). A specific reverse primer (Seq ID NO:16: Cav p 3rev GCC CTT TTC CCC AGC CAC CTC CTG C) was designed to amplify the 5'end using 5'RACE. The combination of the 5' and 3' amplifications revealed an open reading frame of 512 nucleotides. A hydropathy plot of the deduced aa sequence showed a major hydrophobic region from aa 1 to 15. This region corresponds to a secretory signal peptide with a predicted cleavage site corresponding to the site determined by N-terminal sequencing. Cleavage of the signal peptide results in a 155 amino acid mature protein with a predicted molecular weight of 17.5 kDa and an isoelectric point of 5.15. No asparagin-linked glycosylation sites were detected.

The translated cDNA sequence clearly shows that Cav p 3 is different from the protein called Cav p 1 by Fahlbusch (2002), Allergy 57, 417-422; Fahlbusch (2003), Allergy 58, 629-634. The N-terminus as well as 5 aa are different from the published 15 aa. The official name 'Allergenic lipocalin from Cavia porcellus Cav p 3.0101' has been assigned to the protein by the I.U.I.S. allergen nomenclature sub-committee.

| | |
|---|---|
| Cav p 3 | HQTLDPSEINGQWHTISIAAD |
| Cav p 1 | SEINGDWNTIALSAD |

The Cav p 3 protein sequence was aligned to the protein databases. Sequence alignments clearly identify the protein as lipocalin. It has an identity of 46 % to a rat odorant binding protein, 43 % to hamster aphrodisin, 42 % to a lacrimal protein of hamster, 40 % to the cattle allergen Bos d 2 and 30-45 % identity to other mammalian odorant binding proteins, 20-30 % to rodent urinary proteins.

The cDNA coding for Cav p 3 was inserted into pQE-60 and Cav p 3 was expressed in *E.coli* Rosettagami cells with a C-terminal 6His tag (Figure 5, lane 2).

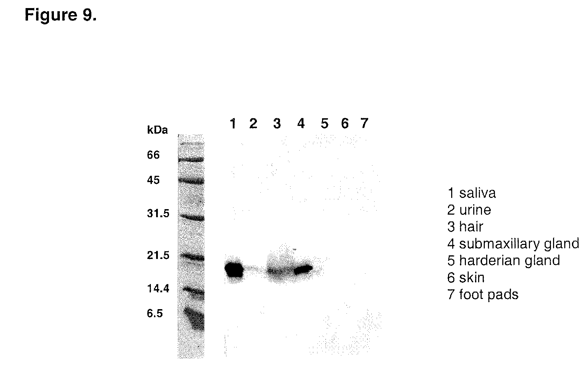

The serum of a mouse immunized with (r)Cav p 3 was used to screen several tissues and secretion products (Figure 9). This serum recognized a strong band at 18-19 kDa in saliva, hair, and submaxillary gland. Cav p 3 is not expressed in the harderian gland, in the skin and in foot pads.

### Example 5: Characterization, cloning and expression of Cav p 6.

A guinea pig hair extract was purified by anion exchange chromatography. Fractions were analysed by immunoblotting with patient sera and mouse anti-Cav p 2 and anti-Cav p 3 sera. An IgE reactive band at about 19 kDa was detected in several fractions negative for Cav p 2 and Cav p 3. This band was subjected to N-terminal sequencing.

| | |
|---|---|
| hair extract | DEVLYGNFDAEKISG |
| harderian gland extract | DEVVRGNFDAEKISGN |

The same protein could be identified in harderian gland extract, however with 2 aa differences. As the protein sequence obtained from hair was ambiguous in several positions, these differences might be due to contaminants.

Protein alignments with the database showed high similarities between our N-terminus and Fel d 4, Equ c 1, alpha-uteroglobulin of the dog, a mouse urinary protein and a salivary lipocalin of the pig. DNA sequences of these proteins were also highly conserved. A degenerated primer Cav p 6 was designed which took into account the protein sequence of Cav p 6 and the conserved DNA sequences of the homologous proteins.
Seq ID NO:17: Cav p 6c: GAR AAG ATT TCR GGV AAY TGG TAT

Oligo Cav p 6 amplified a band at about 750 bp from harderian gland extract. Sequence analysis of the clones obtained identified the cDNA as Cav p 6 (Figure 10). A specific reverse primer (Seq ID NO:18: Cav p 6_5RACE-2 CTG GTT CTC GGC CAT AGA GCT C) was designed to amplify the 5'end using 5'RACE. The combination of the 5' and 3' amplifications revealed an open reading frame of 545 nucleotides. A hydropathy plot of the deduced aa sequence revealed a major hydrophobic region from aa 1 to 15. This region corresponds to a secretory signal peptide with 3 different possible predicted cleavage sites at aa positions 16, 17 or 19. Position 19 corresponds to the site determined by N-terminal sequencing. Cleavage of the signal peptide results in a 162 amino acid mature protein with a predicted molecular weight of 18.5 kDa and an isoelectric point of 4.67. No asparagin-linked glycosylation sites were detected.

The Cav p 6 protein sequence was aligned to the protein databases. Sequence alignments clearly identify the protein as lipocalin. It has an identity of 51 % to rat alpha-uteroglobulin, 49 % to Equ c 1, 42% to Fel d 4 and 45 - 50 % to rodent urinary proteins. The high identity to Fel d 4 and Equ c 1 is not surprising as already the N-terminal sequence aligned on these proteins.

A total of 77 clones arising from 7 independent PCR's and 2 reverse transcriptions have been sequenced. Different clones with single mutations as well as a clone having 6 mutations have been isolated. 4 aa substitutions were present in different RT-PCR clonings, suggesting the existence of at least 5 different Cav p 6 isoforms. A "K to E" substitution at position 47 of the mature protein has been repeatedly found in different clones from independent RT-PCR's.

The cDNA coding for Cav p 6 was inserted into pQE-60 and Cav p 6 was expressed in *E.coli* Rosettagami cells with a C-terminal 6His tag (Figure 5, lane 3). Under non-denaturing conditions, rCav p 6 migrates as a double band.

The serum of a mouse immunized with (r)Cav p 6 was used to screen several tissues and secretion products (Figure 11). This serum detected a strong band at about 18 kDa in saliva, urine, hair, submaxillary gland, skin and foot pads. Bands of 18 and 25 kDa were detected in the harderian gland.

### Example 6: Allergenicity of (r)Cav p 2, (r)Cav p 3 and (r)Cav p 6.

Recombinant proteins were expressed in Rosettagami and purified by affinity chromatography. Protein purity was assessed by N-terminal sequencing and by SDS-PAGE followed by silver staining (Figure 5). Purified (r)Cav p 2, (r)Cav p 3 and (r)Cav p 6 were tested for IgE binding in immunoblot using sera of patients allergic to guinea pig. The recombinant proteins were shown to be immunoreactive in several patients (Figure 12).

In immunoblot, proteins are subjected to SDS-PAGE and their conformation does not reflect their native structure. Therefore, a series of 27 patients with allergy to guinea pig was analysed by ELISA for IgE reactivity to (r)Cav p 2, (r)Cav p 3 and (r)Cav p 6 (Figure 15).

Figures 13 and 14 show the graphical display of the results of the 27 patients. 19 out of 27 patients (70.4%) had IgE antibodies to one or several of the 3 recombinant proteins. For a number of patients, the sum of the specific IgE values to Cav p 2, Cav p 3 and Cav p 6 corresponds to the e6 CAP value. For others, and especially the ones negative for Cav p 2, 3 or 6, the value is below the CAP value, suggesting that at least one other important IgE reactive protein is still missing for a 100% sensitivity of the clinical test.

IgE profiles differ individually. 63% of the patients react to (r)Cav p 2, 52% react to (r)Cav p 3 and 63% react to (r)Cav p 6. 33% of the patients react to all 3 allergens. Some patients react only or predominantly to one allergen (Cav p 2: patients 4, 7, 11; Cav p 6: patients 10, 19, 27). All patients, which were negative for the 3 allergens, were tested for IgE reactivity to guinea pig serum albumin (GPA). One of them was clearly positive (no 13), two others were weakly positive (No 3, 26).

GPA is commercially available. It could be added to the other proteins for diagnosis as recombinant or native molecule. It is known from our studies with cat allergic patients and from other studies (Hilger (1997), Allergy 52,179-187; Spitzauer (1995), J Allergy Clin Immunol 96, 951-959; Goubran Botros (1996), Immunology 88(3), 340-347) that about 22 to 35 % of patients allergic to mammalians react to serum albumins. Most of these recognize also other allergens of these animals, but in rare cases, a monosensitization to serum albumin can occur. Serum albumin is known as a cross-reactive mammalian allergen. Cat allergic patients with IgE reactivity to cat serum albumin will probably also react to GPA and this will result in a positive e6 ImmunoCAP without the patient being truely allergic to guinea pig. The use of the recombinant allergens Cav p 2, Cav p 3 and Cav p 6 will help to distinguish these patients from truely guinea pig allergic patients.

The first commercial microarray has been introduced in the clinic for IgE diagnosis. Large scale epidemiological studies are still needed to evaluate the diagnostic sensitivity and specificity of each of the recombinants used in this system. However for a number of allergens, it has become clear that a single recombinant will not be able to replace the natural extract. For each allergenic source, we will have to rely on several purified native or recombinant molecules.

For other allergens (e.g. birch and grass pollen) it has been shown that individual patients present different IgE reactivity profiles. Diagnosis at the molecular level allows the prediction of cross-reactivities and allows the orientation of the patient to avoidance of specific allergen sources. Another goal of the component-resolved diagnosis is to enable a patient-tailored immunotherapy.

Today, diagnosis of allergy to guinea pig is done with whole extracts. These however can not be used in microarray systems. Purified natural or recombinant molecules have to be used in these high throughput systems. The next years will bring a general change from whole extracts to pure molecules.

### Example 7: Protein alignments of Cav p 2, Cav p 3 and Cav p 6.

Protein sequences of the 3 allergens were aligned to analyse aa identities:

| | |
|---|---|
| Cav p 2 | |
| Cav p 3 | |
| identity 42.86% | similarity 53.90% |
| Cav p 2 | |
| Cav p 6 | |
| | |
| identity 23.03% | similarity 34.21% |
| Cav p 3 | |
| Cav p 6 | |
| identity 21.57% | similarity 31.37% |

Cav p 2 and Cav p 3 share the highest number of amino acid identities. The GXW motif, signature of the lipocalin family, is present near the N-terminus of all three proteins.

### Example 8: Protein purification and characterization of Cavia porcellus allergen 1a

Harderian gland proteins were analysed by 2D gel electrophoresis (Figure 16). IgE immunoreactive spots were isolated for N-terminal amino acid sequencing.

The N-terminal sequence of the 19 kD spot with a pl of 4.1 was S E/Q I N/S G D W N/DTIALSAD.

Further N-terminal sequence analyses were performed on proteins purified by anion exchange chromatography from hair and harderian gland. The result was always a mixture of two sequences at positions 2, 4 and 8.

In a first step, we designed degenerate forward primers from this amino acid sequence to amplify the corresponding cDNA by 3'RACE. Amplification experiments yielded bands in the 600-1000 bp range from the harderian gland. These amplificates were cloned and analysed by DNA sequencing. However all clones contained inserts representing cDNA unrelated to the allergen sequence.

In a second step, the N-terminal sequencing was extended up to aa position 23. aa 9-23 could be read without ambiguity and degenerate primers were designed on this sequence (ADNKEKIEEG). A number of 3'RACE were performed and amplified bands were cloned. None of the clones corresponded to the protein sequence determined by the N-terminal sequencing. Apparently the primers used in PCR had bound to unrelated mRNAs containing part of the sequence motif used for amplification.

The approach using 3'RACE was not successful and we decided to amplify a short internal cDNA fragment using 2 specific primers instead of using 1 specific and 1 oligo dT. For this purpose however we needed to sequence up to 40 residues of the allergen. The purification method was improved by adding a second chromatography step using a high resolution matrix.

*Cavia porcellus* allergen 1 a was purified from 50 mg harderian gland protein extract using 2 sequential anion exchange chromatographies. The extract was first loaded on a Resource Q column and *Cavia porcellus* allergen 1a was eluted by a salt gradient in a TRIS-buffer of pH 8.0 (Figure 17). Fractions containing *Cavia porcellus* allergen 1a were pooled, dialysed with Piperazine pH 5.5 and 1.8 mg of protein was loaded on a Mono Q column. The elution profile showed a number of peaks (Figure 18).

The allergen was found to migrate at a molecular weight of 22 kD under reducing conditions in 15 % SDS-gels. However, under non-reducing conditions, the 22 kD band splits into a 15 kD, a 6kD and a 7 kD band (Figure 19). These different forms eluted as a broad peak between 105 mM NaCl and 170 mM NaCl from the Mono Q column.

N-terminal sequencing of the different bands gave the following results:

| | |
|---|---|
| Fraction D9 7 kD protein: | S Q I S G D W D T I A L S A D |
| Fraction D3715 kD protein: | S E I N G D W N T I A L S A D |
| Fraction 2A5 7 kD protein | S Q I S G D W D T I A L S |
| Fraction C256+7 kD protein: | S Q/E I S/N G D W D/N T I A L S A D |

As the 6 kD protein could not isolated by chromatography, a mixture of the 6 and 7 kD proteins (fraction C25) was analysed by N-terminal sequencing. At 3 positions, there was a mixture of 2 amino acid peaks of high intensity. When substracting the 7kD sequence determined for fraction D9, the sequence of the 6 kD protein should be SEINGDWNTIALSAD. The N-terminal sequence of the 6 kD band seems therefore identical to the sequence of the 15 kD protein. The 15 first amino acids of the 15 kD protein are identical to the Cav p 1 allergen described by Fahlbusch et al. (2002). The 7 kD protein however is a new allergen called *Cavia porcellus* allergen 1 a. N-terminal amino acid sequencing was run for a maximum number of cycles and a large amino acid sequence could be determined, 'x' representing unresolved residues:
SQISGDWDTIALSADNKEKIEEGGPLxxYFRQIDDNADDS

The serum of a mouse immunized with recombinant *Cavia porcellus* allergen 1a (produced as in example 10) was used to screen a number of tissues and secretion products. Extracts were analysed by 15% SDS-PAGE under reducing conditions. Strong bands were detected at 19 kD in the harderian gland and in guinea pig hair extract. *Cavia porcellus* allergen 1 a was not detected in urine, saliva, submaxillary gland, liver or foot pads (Figure 31).

### Example 9: cDNA cloning of Cavia porcellus allergen 1a

All attempts to clone *Cavia porcellus* allergen 1 a using a specific forward primer and 3'RACE had failed. We choose to design another experimental setup. The long amino acid sequence obtained in example 8 was used to make degenerate forward (aa 5-11) and reverse (aa 30-36) primers in order to amplify a small cDNA fragment corresponding to the obtained N-terminal sequence. The primers were designed in a special way: the 5 last bases at the 3'end should not contain ambiguities and the overall sequence complexity should not exceed 32 possible sequences per primer (Table 1). Using all possible primer combinations, a total of 96 PCRs were performed and analysed by high resolution metaphor agarose gels for the presence of a 95bp band amplified specifically by the used primers.

Primers Cavp-MQ2 and Cavp-MQ7rev amplified a 95 bp DNA fragment by PCR which was cloned for sequence analysis. The translated sequence corresponded to the *Cavia porcellus* allergen 1 a amino acid sequence 5-36. A specific forward primer (Cavp-11_for) was designed from this sequence to obtain the 3'end of the cDNA. Analysis of the 3'end enabled the choice of a specific primer (Cavp-b_rev) for amplification of the 5'end by 5'RACE PCR.

**Table 1: Primers designed for amplification of a cDNA fragment coding for the N-terminus of Cavia porcellus allergen 1a**

| **SEQ ID No.:** | **Primer name** | **Primer sequence 5' -> 3'** |
|---|---|---|
| 21 | Cavp-MQ1 | GGN GAY TGG RAC ACR ATC GC |
| 22 | Cavp-MQ2 | GGN GAY TGG RAC ACR ATT GC |
| 23 | Cavp-MQ3 | GGN GAY TGG RAC ACR ATA GC |
| 24 | Cavp-MQ4 | GGN GAY TGG RAC ACY ATC GC |
| 25 | Cavp-MQ5 | GGN GAY TGG RAC ACY ATT GC |
| 26 | Cavp-MQ6 | GGN GAY TGG RAC ACY ATA GC |
| 27 | Cavp-MQ7 | GGN GAY TGG RAT ACR ATC GC |
| 28 | Cavp-MQ8 | GGN GAY TGG RAT ACR ATT GC |
| 29 | Cavp-MQ9 | GGN GAY TGG RAT ACR ATA GC |
| 30 | Cavp-MQ10 | GGN GAY TGG RAT ACY ATC GC |
| 31 | Cavp-MQ11 | GGN GAY TGG RAT ACY ATT GC |
| 32 | Cavp-MQ12 | GGN GAY TGG RAT ACY ATA GC |
| | | |
| 33 | Cavp-MQ1rev | TT RTC RTC DAT YTG TCT AAA |
| 34 | Cavp-MQ2rev | TT RTC RTC DAT YTG CCT AAA |
| 35 | Cavp-MQ3rev | TT RTC RTC DAT YTG TCT GAA |
| 36 | Cavp-MQ4rev | TT RTC RTC DAT YTG CCT GAA |
| 37 | Cavp-MQ5rev | TT RTC RTC DAT YTG RCG AAA |
| 38 | Cavp-MQ6rev | TT RTC RTC DAT YTG YCG AAA |
| 39 | Cavp-MQ7rev | TT RTC RTC DAT YTG RCG GAA |
| 40 | Cavp-MQ8rev | TT RTC RTC DAT YTG YCG GAA |
| | | |
| 41 | Cavp-11_for (3'RACE) | CTCTGTCTGC TGACAACAAA GAGRAGATCG AAGAGG |
| 42 | Cavp-b_rev (5'RACE) | CGTTTCTTCG GGTGTCAGAG AGTCTC |

Combination of the 5' and 3' cDNA ends revealed an open reading frame of 498 nucleotides (Figure 20). Sequence analysis software SignalSeq predicted a secretory signal peptide of 18 amino acids with a cleavage site corresponding to the site determined by N-terminal sequencing. The mature protein has a predicted molecular weight of 16.4 kD and an isoelectric point of 4.03. No asparagin-linked glycosylation sites were detected. The deduced amino acid sequence deviates at residues Cys⁵³ and Cys⁵⁷ from the protein sequence determined by Edman degradation. Cysteins can not be detected by this method and positions have been misinterpreted as aspartic acid.

*Cavia porcellus* allergen 1 a was aligned to protein databases. It is classified into the lipocalin family and more specifically to the odorant binding protein family. It has an identity of 45 % to hamster aphrodisin and identities to different mammalian odorant binding proteins ranging from 31 % to 39%. Identity to Bos d 2 (cattle dander allergen 2) is 36%.

### Example 10: Cavia porcellus allergen 1a: production of recombinant protein and determination of IgE reactivity

The cDNA coding for *Cavia porcellus* allergen 1 a was inserted into pQE-60 and the protein was expressed in *E.coli* Rosettagami cells with a C-terminal hexahistidine tag. Recombinant protein was purified by affinity chromatography. Purity was assessed by N-terminal sequencing and by SDS-PAGE followed by silver staining (Figure 21).

The theoretical molecular weight of recombinant *Cavia porcellus* allergen 1a including the hexahistidine tag is 17.584 kD. The apparent molecular weight in 15% SDS-PAGE under reducing conditions is higher. Running the protein sample under non-reducing conditions induces a mobility shift indicating that the 4 cysteins of the protein may form 1 or 2 disulfid bridges. A similar mobility shift is observed for the native protein (Figure 18, lane D9).

IgE reactivity of recombinant *Cavia porcellus* allergen 1 a was analysed by ELISA in a series of 27 guinea pig allergic patients and 19 IgE negative controls. A second control group consisted of 22 IgE positive patients without allergy to mammals, but with specific IgE to pollen or house dust mite (total IgE range 16-2051 kU/L).

The cutoff value 1.9 kU/L was calculated from the IgE negative patient group as the average plus 3 standard deviations. 18 out of 27 patients (67%) had specific IgE antibodies to recombinant *Cavia porcellus* allergen 1a (Figure 22).

### Example 11: IgE reactivity of (r)Cavia porcellus allergen 1a, (r)Cav p 2, (r)Cav p 3 and (r)Cav p 6.

A series of 27 patients with allergy to guinea pig was analysed by ELISA for IgE reactivity to (r)Cavia porcellus allergen 1 a, (r)Cav p 2, (r)Cav p 3 and (r)Cav p 6.

Figures 23 and 24 show the graphical display of the results of the 27 patients. 24 out of 27 patients (88.9%) had IgE antibodies to one or several of the 3 recombinant proteins. For a number of patients, the sum of the specific IgE values to *Cavia porcellus* allergen 1 a, Cav p 2, Cav p 3 and Cav p 6 corresponds to the e6 CAP value. For others, the value obtained by ELISA is below the CAP value. There are several possible explanations to this: the capacity of antigen binding of our ELISA plate is much lower than that of the CAP system and the binding sites on the ELISA plate might have been saturated; another possibility is that at least one other important IgE reactive protein might still be missing.

IgE profiles differ individually. 67% of the patients react to (r)*Cavia porcellus* allergen 1 a, 63% react to (r)Cav p 2, 52% react to (r)Cav p 3 and 63% react to (r)Cav p 6. 33% of the patients react to all 4 allergens. Some patients react only or predominantly to one allergen (*Cavia porcellus* allergen 1 a: patients 3, 4, 22, 23; Cav p 2: patients 4, 7, 11; Cav p 6: patients 10, 19, 27).

All patients, which were negative for the 4 allergens (no 8, 15 and 26), were tested for IgE reactivity to guinea pig serum albumin (GPA) in ELISA. Sera no 2 and 13 were included as they were only weakly reacting to one allergen. Sera no 8, 13 and 26 had specific IgE antibodies to GPA (1.7,11.3 and 1.4 kU/L), no 15 was weakly positive (0.8 kU/L). Serum no 26 has also IgE antibodies to cat and dog dander and to cat serum albumin (9.3 kU/L). It might be possible that the patient is primarily sensitized to cat and/or dog and has a cross-sensitization to guinea pig because of IgE cross-reactivity to GPA.

Serum albumin is known as a cross-reactive mammalian allergen. Cat allergic patients with IgE reactivity to cat serum albumin will probably also react to GPA and this will result in a positive e6 ImmunoCAP without the patient being truly allergic to guinea pig. The use of the recombinant allergens *Cavia porcellus* allergen 1a, Cav p 2, Cav p 3 and Cav p 6 will help to distinguish these patients from truely guinea pig.

Protein sequences of the 4 allergens were aligned to *Cavia porcellus* allergen 1a to analyse amino acid identities:

| | |
|---|---|
| Allergen | |
| Cav p 2 | |
| | |
| Identity 43.92% | similarity 52.70% |
| Allergen | |
| Cav p | |
| Identity: 45.95% | Similarity: 54.05% |
| Allergen | |
| Cav | |
| Identity: 28.76% | Similarity: 41.78% |

### Example 12: Protein purification and characterization of Cav p 2

### Purification of Cav p 2 from hair

Proteins were extracted from hair as described above and dialysed with 20 mM TRIS-HCl pH8 using Ultrafree concentrators with a cut off of 3000 Da (Millipore). The soluble proteins were filtered through a 0.22 µm filter and loaded onto a Resource Q column (1ml, GE Healthcare). Bound proteins were eluted by a linear gradient of 0-500 mM NaCl in 20 mM TRIS-HCl, pH8. Eluted fractions were analysed by SDS-PAGE followed by silver staining and immunoblotting with mouse anti-Cav p 2 serum. Fractions containing Cav p 2 were pooled and concentrated using Ultrafree concentrators with a cut off of 3000 Da (Millipore).

### Result

Cav p 2 is abundant on hair. The protein was purified by anion exchange chromatography using protein extract from hair. 2x 5 mg were separated by a 1 ml Resource Q column using a linear gradient of 0-500 mM NaCl in 20 mM TRIS-HCl, pH 8. Eluted fractions were analysed by SDS-PAGE followed by silver staining (Figure 25).

Fractions containing proteins of a molecular weight of 17 kD were immunoblotted using a mouse anti-Cav p 2 serum. Cav p 2 eluted within a broad peak at a NaCl concentration of 115-150 mM. A total of 0.6 mg Cav p 2 was obtained from 10.3 mg guinea pig hair proteins. This represents a final yield of 5.8% of soluble hair proteins.

Purified Cav p 2 was analysed by SDS-PAGE (Figure 26). The protein has an apparent molecular weight of 17 kDa under reducing conditions. Non-reducing conditions induce a mobility shift indicating that the 4 cysteins present in the molecule may form one or 2 disulfid bridges. Identity of the purified protein was confirmed by N-terminal sequencing and by immunoblotting using a mouse anti-Cav p 2 serum.

### Example 13: Protein purification and characterization of Cav p 3

### Purification of Cav p 3 from submaxillary gland extract

Submaxillary gland was extracted as described above and precipitated with methanol. 50 mg of precipitated extract were suspended in 20 mM TRIS-HCl pH8. The sample was centrifuged at high speed and the soluble proteins were filtered by a 0.22 µm filter and loaded onto a Resource Q column (1ml, GE Healthcare). Bound proteins were eluted by a linear gradient of 0-500 mM NaCl in 20 mM TRIS-HCl, pH8. Eluted fractions were analysed by SDS-PAGE followed by silver staining and immunoblotting with mouse anti-Cav p 3 serum.

Fractions containing Cav p 3 were pooled and concentrated using Ultrafree concentrators with a cut off of 3000 Da (Millipore). The concentrated sample was than loaded on a prepacked gelfiltration column (Superdex 75, 10/300 GL, GE Healthcare) and proteins were eluted in 50 mM KH₂PO₄, 150 mM NaCl, pH7. Fractions were analysed by SDS-PAGE followed by silver staining and fractions containing Cav p 3 were combined, concentrated and dialysed into PBS.

### Result

Cav p 3 was found to be abundant in the submaxillary gland. The protein was purified by anion exchange chromatography using protein extract from the submaxillary gland. A total of 50 mg of extract were separated by a 1 ml Resource Q column using a linear gradient of 0-500 mM NaCl in 20 mM TRIS-HCl, pH 8. Eluted fractions were analysed by SDS-PAGE followed by silver staining and immunoblot (Figure 27).

Fractions of all peaks were analysed by SDS-PAGE and fractions containing proteins of a molecular weight of 18-20 kD were immunoblotted using a mouse anti-Cav p 3 serum. Cav p 3 eluted as a single peak at a NaCl concentration of 105-130 mM. Analysis of the pooled fractions showed that Cav p 3 was contaminated with a 66 kD protein and Cav p 3 was further purified by gel filtration (Figure 28) using Superdex 75 resin.

The gel filtration could not totally separate the two proteins. Fractions A29 to B10 were pooled and analysed by SDS-PAGE (Figure 29). Silver staining showed that Cav p 3 is still contaminated by the 66 kD protein. The 66 kD protein was suspected to be guinea pig serum albumin. This could be confirmed by immunoblot using an anti-GPA mouse serum.

Under reducing conditions, Cav p 3 runs at an apparent molecular weight of 20 kD. Non-reducing conditions induce a mobility shift indicating that the 4 cysteins present in the molecule may form one or 2 disulfid bridges. Identity of Cav p 3 was confirmed by immunoblot using a anti-Cav p 3 mouse serum.

### Example 14: Protein purification and characterization of Cav p 6

### Purification of Cav p 6 from harderian gland extract

Harderian gland was extracted as described above and dialysed in 20 mM TRIS-HCl pH8. The sample was filtered by a 0.22 µm filter and 20 mg of protein were loaded onto a Resource Q column (1ml, GE Healthcare). Bound proteins were eluted by a linear gradient of 0-500 mM NaCl in 20 mM TRIS-HCl, pH8. Fractions were analysed by 15% SDS-PAGE followed by silver staining.

### Result

Cav p 6 was identified on hair and in harderian gland extract. Cav p 6 was purified from the harderian gland by anion exchange chromatography. A total of 20 mg of extract were separated by a 1 ml Resource Q column using a linear gradient of 0-500 mM NaCl in 20 mM TRIS-HCl, pH 8. Eluted fractions were analysed by SDS-PAGE followed by silver staining and immunoblot (Figure 30).

Cav p 6 had initially been identified in a purification of hair extract by N-terminal sequencing. The IgE reactive protein eluted directly after Cav p 2 in a NaCl gradient. Therefore individual fractions were analysed with mouse anti-Cav p 2 serum. Cav p 2 was found to be present in fractions 1C30 to 1D35. The dominant 20kD band in fraction 2A5 was analysed by N-terminal sequencing and identified as Cav p 6. N-terminal sequence: DEVVRGNFDAEKISGN

Cav p 6 is present in fractions 2A4 through 2A15. It elutes as a small peak at a concentration of 150 mM NaCl.

### Example 15: Detection of guinea pig allergens in commercial skin prick test solutions

The protein content of skin prick test solutions from 4 suppliers was quantified. Protein quantity was highly variable ranging from 0.13 mg/ml to 1.6 mg/ml. A sample of each prick test solution was analysed by 15 % SDS-PAGE gel. Due to low protein concentrations, samples 1 and 2 were stained with silver, samples 3 and 4 with Coomassie (Figure 32 A).

Polyclonal mouse sera raised against *Cavia porcellus* allergen 1 a, Cav p 2, Cav p 3, Cav p 6 and GPA were used for detection of allergens by immunoblot. GPA was detected in all 4 extracts. *Cavia porcellus* allergen 1 a was detected in extracts 2, 3 and 4. Cav p 6 was detected in extracts 2 and 4. Weak bands were visualized by anti-Cav p 2 and anti-Cavp 3 sera in extract 4.

These results highlight the value of purified and/or recombinant molecules. Antibodies can be raised against single components and used for standardization of whole extracts. Single molecules can be added to extracts to compensate for insufficient allergen quantity.

The protein content as well as the allergen content is highly variable in commercial skin prick test solutions. The set up of tools for standardization of animal extracts is mandatory to improve the quality of those extracts.

The present application refers to the following nucleotide and amino acid sequences:
SEQ ID No. 1:
   Nucleotide sequence encoding Caviidae allergen Cav p 3 (cDNA).
SEQ ID No. 2:
   Amino acid sequence of the Caviidae allergen Cav p 3.
SEQ ID No. 3:
   Nucleotide sequence encoding Caviidae allergen Cav p 6 (cDNA).
SEQ ID No. 4:
   Amino acid sequence of the Caviidae allergen Cav p 6.
SEQ ID No. 5:
   Nucleotide sequence encoding Caviidae allergen Cav p 2 (cDNA).
SEQ ID No. 6:
   Amino acid sequence of the Caviidae allergen Cav p 2. (Accession Number of Swissprot: P83508)
SEQ ID No. 7:
   Nucleotide sequence of degenerate Cav p 2a primer used for amplification of the Cav p 2 gene.
SEQ ID No. 8:
   Nucleotide sequence of degenerate Cav p 2b primer used for amplification of the Cav p 2 gene.
SEQ ID No. 9:
   Nucleotide sequence of degenerate Cav p 2c primer used for amplification of the Cav p 2 gene.
SEQ ID No. 10:
   Nucleotide sequence of degenerate Cav p 2d primer used for amplification of the Cav p 2 gene.
SEQ ID No. 11:
   Nucleotide sequence of degenerate Cav p 2rev primer used for amplification of the 5'end of the Cav p 2 gene.
SEQ ID No. 12:
   Nucleotide sequence of degenerate Cav p 1 e primer used for amplification of the Cav p3 gene.
SEQ ID No. 13:
   Nucleotide sequence of degenerate Cav p 1f primer used for amplification of the Cav p3 gene.
SEQ ID No. 14:
   Nucleotide sequence of degenerate Cav p 1g primer used for amplification of the Cav p 3 gene.
SEQ ID No. 15:
   Nucleotide sequence of degenerate Cav p 1 h primer used for amplification of the Cav p 3 gene.
SEQ ID No. 16:
   Nucleotide sequence of degenerate Cav p 3 rev primer used for amplification of the Cav p 3 gene.
SEQ ID No. 17:
   Nucleotide sequence of degenerate Cav p 6 primer used for amplification of the Cav p 6 gene.
SEQ ID No. 18:
   Nucleotide sequence of degenerate Cav p 6_5RACE2 primer used for amplification of the 5'end of the Cav p 6 gene.
SEQ ID No. 19:
   Nucleotide sequence encoding *Cavia porcellus* allergen 1 a (cDNA).
SEQ ID No. 20:
   Amino acid sequence of the *Cavia porcellus* allergen 1 a.
SEQ ID No. 21:
   Nucleotide sequence of degenerate Cavp-MQ1 primer used for amplification of the *Cavia porcellus* allergen 1 a gene.
SEQ ID No. 22:
   Nucleotide sequence of degenerate Cavp-MQ2 primer used for amplification of the *Cavia porcellus* allergen 1 a gene.
SEQ ID No. 23:
   Nucleotide sequence of degenerate Cavp-MQ3 primer used for amplification of the *Cavia porcellus* allergen 1 a gene.
SEQ ID No. 24:
   Nucleotide sequence of degenerate Cavp-MQ4 primer used for amplification of the *Cavia porcellus* allergen 1 a gene.
SEQ ID No. 25:
   Nucleotide sequence of degenerate Cavp-MQ5 primer used for amplification of the *Cavia porcellus* allergen 1 a gene.
SEQ ID No. 26:
   Nucleotide sequence of degenerate Cavp-MQ6 primer used for amplification of the *Cavia porcellus* allergen 1 a gene.
SEQ ID No. 27:
   Nucleotide sequence of degenerate Cavp-MQ7 primer used for amplification of the *Cavia porcellus* allergen 1 a gene.
SEQ ID No. 28:
   Nucleotide sequence of degenerate Cavp-MQ8 primer used for amplification of the *Cavia porcellus* allergen 1 a gene.
SEQ ID No. 29:
   Nucleotide sequence of degenerate Cavp-MQ9 primer used for amplification of the *Cavia porcellus* allergen 1 a gene.
SEQ ID No. 30:
   Nucleotide sequence of degenerate Cavp-MQ10 primer used for amplification of the *Cavia porcellus* allergen 1 a gene.
SEQ ID No. 31:
   Nucleotide sequence of degenerate Cavp-MQ11 primer used for amplification of the *Cavia porcellus* allergen 1 a gene.
SEQ ID No. 32:
   Nucleotide sequence of degenerate Cavp-MQ12 primer used for amplification of the *Cavia porcellus* allergen 1 a gene.
SEQ ID No. 33:
   Nucleotide sequence of degenerate Cavp-MQ1 rev primer used for amplification of the *Cavia porcellus* allergen 1 a gene.
SEQ ID No. 34:
   Nucleotide sequence of degenerate Cavp-MQ2rev primer used for amplification of the *Cavia porcellus* allergen 1 a gene.
SEQ ID No. 35:
   Nucleotide sequence of degenerate Cavp-MQ3rev primer used for amplification of the *Cavia porcellus* allergen 1 a gene.
SEQ ID No. 36:
   Nucleotide sequence of degenerate Cavp-MQ4rev primer used for amplification of the *Cavia porcellus* allergen 1 a gene.
SEQ ID No. 37:
   Nucleotide sequence of degenerate Cavp-MQ5rev primer used for amplification of the *Cavia porcellus* allergen 1 a gene.
SEQ ID No. 38:
   Nucleotide sequence of degenerate Cavp-MQ6rev primer used for amplification of the *Cavia porcellus* allergen 1 a gene.
SEQ ID No. 39:
   Nucleotide sequence of degenerate Cavp-MQ7rev primer used for amplification of the *Cavia porcellus* allergen 1 a gene.
SEQ ID No. 40:
   Nucleotide sequence of degenerate Cavp-MQ8rev primer used for amplification of the *Cavia porcellus* allergen 1 a gene.
SEQ ID No. 41:
   Nucleotide sequence of degenerate Cavp-11_for (3'RACE) primer used for amplification of the *Cavia porcellus* allergen 1a gene.
SEQ ID No. 42:
   Nucleotide sequence of degenerate Cavp-b_rev (5'RACE) primer used for amplification of the *Cavia porcellus* allergen 1a gene.

## Claims

1. A nucleic acid molecule encoding a Caviidae allergen comprising a polynucleotide selected from the group consisting of:
(a) a polynucleotide sequence as shown in SEQ ID NO:19;
(b) a polynucleotide sequence encoding a polypeptide as shown in SEQ ID NO:20;
(c) a polynucleotide sequence which has at least 80% identity to the polynucleotides as defined in (a) or (b) encoding a Caviidae allergen;
(d) a polynucleotide sequence encoding a polypeptide which has at least 85% identity to the polypeptide as shown in SEQ ID NO:20; and
(e) a polynucleotide sequence being degenerate as a result of the genetic code to the nucleotide sequence as defined in any one of (a) to (d).

2. A vector comprising a nucleic acid molecule according to claim 1.

3. A recombinant host cell comprising a nucleic acid molecule according to claim 1, or a vector according to claim 2.

4. A method for producing a polypeptide, comprising culturing a recombinant host cell according to claim 3 under such conditions that the polypeptide is expressed, and recovering the polypeptide.

5. A polypeptide encoded by a nucleic acid molecule according to claim 1, or obtainable by the process of claim 4.

6. A polypeptide according to claim 5, in combination with a further polypeptide encoded by a further nucleic acid molecule encoding a Caviidae allergen comprising a polynucleotide selected from the group consisting of:
a) a polynucleotide sequence as shown in SEQ ID NO:5;
(b) a polynucleotide sequence encoding a polypeptide as shown in SEQ ID NO:6;
(c) a polynucleotide sequence which has at least 80% identity to the polynucleotides as defined in (a) or (b) encoding a Caviidae allergen;
(d) a polynucleotide sequence encoding a polypeptide which has at least 85% identity to the polypeptide as shown in SEQ ID NO:6.

7. An antibody that specifically binds to the polypeptide of SEQ ID N0:20.

8. Use of an antibody according to claim 7, for purification of the polypeptide according to claim 5.

9. Pharmaceutical composition comprising the nucleic acid molecule of claim 1, the vector of claim 2, the polypeptides of claim 5, or 6, or the antibody of claim 7.

10. Pharmaceutical composition according to claim 9, for use in hyposensibilisation or in medical intervention of an allergic reaction.

11. The nucleic acid molecule of claim 1, the vector of claim 2, the polypeptides of claims 5, or 6, or the antibody of claim 7 for use in hyposensibilisation or in medical intervention of an allergic reaction.

12. Use of the nucleic acid molecule of claim 1, the vector of claim 2, the polypeptides of claims 5, or 6, or the antibody of claim 7, for the preparation of a pharmaceutical composition for hyposensibilisation or medical intervention of an allergic reaction.

13. The nucleic acid molecule of claim 1, the vector of claim 2, the polypeptides of claims 5, or 6, or the antibody of claim 7 for use in diagnosis of an allergic reaction or for the determination of a subject prone to an allergic reaction.

14. Use of nucleic acid molecule of claim 1, the vector of claim 2, the polypeptides of claims 5, or 6, or the antibody of claim 7 for the preparation of a diagnostic composition for the diagnosis of allergic reaction or for the determination of a subject prone to an allergic reaction.

15. An in vitro method for determining allergenicity comprising the steps of:
(a) exposing a sample to the polypeptides according to claims 5, or 6 or the antibody of claim 7; and
(b) determining immunological reaction or the formation of an immunological complex to the exposure of step (a).

16. The method of claim 15, wherein said sample is selected from the group, consisting of a biological sample, a medical sample or an environmental sample.

17. The method of claim 15 or 16, wherein said immunological reaction comprises an increased titre of immunglobulins, an increased histamine release, leukotriene release or wherein said formation of an immunological complex is the formation of an antibody-antigen complex between either the polypeptides of claims 5, or 6, and an antibody or the antibody of claim 7 and a corresponding antigen.

18. The method of claim 17 wherein an increased titre of immunglobulins and/or an increased histamine release is indicative for an immunological or allergic reaction.

19. The method of claim 17 or 18 wherein said immunoglobulin is IgE.

20. The method of claim 18 or 19, wherein said immunological or allergic reaction is a reaction to Caviidae.

21. A method for identifying molecules which are capable of interacting with the polypeptides of claims 5, or 6, comprising:
(a) producing cells which express said polypeptide;
(b) contacting the polypeptide produced in step (a) with a test sample potentially containing said molecule; and
(c) identifying among these molecules the molecules which are capable of interacting with said polypeptide.

## Patentansprüche

1. Nucleinsäuremolekül, codierend ein Caviidae-Allergen, umfassend ein Polynucleotid, ausgewählt aus der Gruppe, bestehend aus:
a) einer Polynucleotidsequenz, wie angegeben in SEQ ID NO:19;
b) einer Polynucleotidsequenz, codierend ein Polypeptid, wie angegeben in SEQ ID NO:20;
c) einer Polynucleotidsequenz, welche mindestens 80% Identität mit den Polynucleotiden, wie definiert in (a) oder (b), hat, codierend ein Caviidae-Allergen;
d) einer Polynucleotidsequenz, codierend ein Polypeptid, welches mindestens 85% Identität mit dem Polypeptid, wie angegeben in SEQ ID NO:20, hat; und
e) einer Polynucleotidsequenz, die als Ergebnis des genetischen Codes zu der Nucleotidsequenz, wie definiert in einem von (a) bis (d), degeneriert ist.

2. Vektor, umfassend ein Nucleinsäuremolekül gemäß Anspruch 1.

3. Rekombinante Wirtszelle, umfassend ein Nucleinsäuremolekül gemäß Anspruch 1 oder einen Vektor gemäß Anspruch 2.

4. Verfahren zum Erzeugen eines Polypeptids, umfassend Kultivieren einer rekombinanten Wirtszelle gemäß Anspruch 3 unter derartigen Bedingungen, dass das Polypeptid exprimiert wird, und Gewinnen des Polypeptids.

5. Polypeptid, codiert durch ein Nucleinsäuremolekül gemäß Anspruch 1 oder erhältlich durch das Verfahren nach Anspruch 4.

6. Polypeptid gemäß Anspruch 5 in Kombination mit einem weiteren Polypeptid, codiert durch ein weiteres Nucleinsäuremolekül, codierend ein Caviidae-Allergen, umfassend ein Polynucleotid, ausgewählt aus der Gruppe, bestehend aus:
a) einer Polynucleotidsequenz, wie angegeben in SEQ ID NO:5;
b) einer Polynucleotidsequenz, codierend ein Polypeptid, wie angegeben in SEQ ID NO:6;
c) einer Polynucleotidsequenz, welche mindestens 80% Identität mit den Polynucleotiden, wie definiert in (a) oder (b), hat, codierend ein Caviidae-Allergen;
d) einer Polynucleotidsequenz, codierend ein Polypeptid, welches mindestens 85% Identität mit dem Polypeptid, wie angegeben in SEQ ID NO:6, hat.

7. Antikörper, der spezifisch an das Polypeptid von SEQ ID NO:20 bindet.

8. Verwendung eines Antikörpers gemäß Anspruch 7 zur Reinigung des Polypeptids gemäß Anspruch 5.

9. Pharmazeutische Zusammensetzung, umfassend das Nucleinsäuremolekül nach Anspmch 1, den Vektor nach Anspruch 2, die Polypeptide nach Anspmch 5 oder 6 oder den Antikörper nach Anspruch 7.

10. Pharmazeutische Zusammensetzung gemäß Anspruch 9 zur Verwendung bei der Hyposensibilisierung oder bei medizinischer Intervention bei einer allergischen Reaktion.

11. Nucleinsäuremolekül nach Anspruch 1, Vektor nach Anspruch 2, Polypeptide nach den Ansprüchen 5 oder 6 oder Antikörper nach Anspruch 7 zur Verwendung bei der Hyposensibilisierung oder bei medizinischer Intervention bei einer allergischen Reaktion.

12. Verwendung des Nucleinsäuremoleküls nach Anspruch 1, des Vektors nach Anspruch 2, der Polypeptide nach den Ansprüchen 5 oder 6 oder des Antikörpers nach Anspruch 7 für die Herstellung einer pharmazeutischen Zusammensetzung zur Hyposensibilisierung oder für eine medizinische Intervention bei einer allergischen Reaktion.

13. Nucleinsäuremolekül nach Anspruch 1, Vektor nach Anspruch 2, Polypeptide nach den Ansprüchen 5 oder 6 oder Antikörper nach Anspruch 7 zur Verwendung bei der Diagnose einer allergischen Reaktion oder für die Ermittlung eines Subjekts, anfällig für eine allergische Reaktion.

14. Verwendung des Nucleinsäuremoleküls nach Anspruch 1, des Vektors nach Anspruch 2, der Polypeptide nach den Ansprüchen 5 oder 6 oder des Antikörpers nach Anspruch 7 für die Herstellung einer diagnostischen Zusammensetzung für die Diagnose einer allergischen Reaktion oder für die Ermittlung eines Subjekts, anfällig für eine allergische Reaktion.

15. In-vitro-Verfahren zum Vermitteln von Allergenität, umfassend die Schritte:
a) Einwirken der Polypeptide gemäß den Ansprüchen 5 oder 6 oder des Antikörpers nach Anspruch 7 auf eine Probe; und
b) Ermitteln einer immunologischen Reaktion oder der Bildung eines immunologischen Komplexes, auf die Einwirkung von Schritt (a).

16. Verfahren nach Anspruch 15, wobei die Probe aus der Gruppe, bestehend aus einer biologischen Probe, einer medizinischen Probe oder einer Umweltprobe, ausgewählt ist.

17. Verfahren nach Anspruch 15 oder 16, wobei die immunologische Reaktion einen erhöhten Titer von Immunglobulinen, eine erhöhte Histaminfreisetzung, Leukotrienfreisetzung umfasst oder wobei die Bildung eines immunologischen Komplexes die Bildung eines Antikörper-Antigen-Komplexes zwischen entweder den Polypeptiden nach den Ansprüchen 5 oder 6 und einem Antikörper oder dem Antikörper nach Anspruch 7 und einem entsprechenden Antigen ist.

18. Verfahren nach Anspruch 17, wobei ein erhöhter Titer von Immunglobulinen und/oder eine erhöhte Histaminfreisetzung ein Anzeichen für eine immunologische oder allergische Reaktion ist.

19. Verfahren nach Anspruch 17 oder 18, wobei das Immunglobulin IgE ist.

20. Verfahren nach Anspruch 18 oder 19, wobei die immunologische oder allergische Reaktion eine Reaktion auf Caviidae ist.

21. Verfahren zum Identifizieren von Molekülen, welche zum Wechselwirken mit den Polypeptiden nach den Ansprüchen 5 oder 6 fähig sind, umfassend:
(a) Erzeugen von Zellen, welche das Polypeptid exprimieren;
(b) Inkontaktbringen des Polypeptids, erzeugt in Schritt (a), mit einer Testprobe, potentiell enthaltend das Molekül; und
(c) Identifizieren unter diesen Molekülen der Moleküle, welche zum Wechselwirken mit dem Polypeptid fähig sind.

## Revendications

1. Molécule d'acide nucléique codant pour un allergène de Caviidae comprenant un polynucléotide choisi dans le groupe constitué de:
(a) une séquence de polynucléotide telle que montrée dans la SEQ ID NO:19;
(b) une séquence de polynucléotide codant pour un polypeptide tel que montré dans la SEQ ID NO:20;
(c) une séquence de polynucléotide qui possède au moins 80% d'identité avec les polynucléotides tels que définis dans (a) ou (b) codant pour un allergène de Caviidae;
(d) une séquence de polynucléotide codant pour un polypeptide qui possède au moins 85% d'identité avec le polypeptide tel que montré dans la SEQ ID NO:20; et
(e) une séquence de polynucléotide qui est dégénérée en conséquence du code génétique par rapport à la séquence de polynucléotide telle que définie dans l'un quelconque de (a) à (d).

2. Vecteur comprenant une molécule d'acide nucléique selon la revendication 1.

3. Cellule hôte recombinante comprenant une molécule d'acide nucléique selon la revendication 1 ou un vecteur selon la revendication 2.

4. Procédé pour la production d'un polypeptide, comprenant la culture d'une cellule hôte recombinante selon la revendication 3, dans des conditions telles que le polypeptide est exprimé, et la récupération du polypeptide.

5. Polypeptide codé par une molécule d'acide nucléique selon la revendication 1 ou pouvant être obtenu par le procédé selon la revendication 4.

6. Polypeptide selon la revendication 5, en combinaison avec un autre polypeptide codé par une autre molécule d'acide nucléique codant pour un allergène de Caviidae comprenant un polynucléotide choisi dans le groupe constitué de:
(a) une séquence de polynucléotide telle que montrée dans la SEQ ID NO:5;
(b) une séquence de polynucléotide codant pour un polypeptide tel que montré dans la SEQ ID NO:6;
(c) une séquence de polynucléotide qui possède au moins 80% d'identité avec les polynucléotides tels que définis dans (a) ou (b) codant pour un allergène de Caviidae;
(d) une séquence de polynucléotide codant pour un polypeptide qui possède au moins 85% d'identité avec le polypeptide tel que montré dans la SEQ ID NO:6.

7. Anticorps qui se lie spécifiquement au polypeptide de la SEQ ID NO:20.

8. Utilisation d'un anticorps selon la revendication 7, pour une purification du polypeptide selon la revendication 5.

9. Composition pharmaceutique comprenant la molécule d'acide nucléique selon la revendication 1, le vecteur selon la revendication 2, les polypeptides selon la revendication 5, ou 6, ou l'anticorps selon la revendication 7.

10. Composition pharmaceutique selon la revendication 9, pour une utilisation dans une hyposensibilisation ou dans une intervention médicale dans le cas d'une réaction allergique.

11. Molécule d'acide nucléique selon la revendication 1, vecteur selon la revendication 2, polypeptides selon la revendication 5, ou 6, ou anticorps selon la revendication 7 pour une utilisation dans une hyposensibilisation ou dans une intervention médicale dans le cas d'une réaction allergique.

12. Utilisation de la molécule d'acide nucléique selon la revendication 1, du vecteur selon la revendication 2, des polypeptides selon la revendication 5, ou 6, ou de l'anticorps selon la revendication 7, pour la préparation d'une composition pharmaceutique pour une hyposensibilisation ou une intervention médicale dans le cas d'une réaction allergique.

13. Molécule d'acide nucléique selon la revendication 1, vecteur selon la revendication 2, polypeptides selon la revendication 5, ou 6, ou anticorps selon la revendication 7 pour une utilisation dans le diagnostic d'une réaction allergique ou pour la détermination d'un sujet prédisposé à une réaction allergique.

14. Utilisation de la molécule d'acide nucléique selon la revendication 1, du vecteur selon la revendication 2, des polypeptides selon la revendication 5, ou 6, ou de l'anticorps selon la revendication 7, pour la préparation d'une composition diagnostique pour le diagnostic d'une réaction allergique ou pour la détermination d'un sujet prédisposé à une réaction allergique.

15. Méthode in vitro pour la détermination d'une allergénicité comprenant les étapes:
(a) d'exposition d'un échantillon aux polypeptides selon la revendication 5, ou 6, ou à l'anticorps selon la revendication 7; et
(b) de détermination d'une réaction immunologique ou de la formation d'un complexe immunologique à l'exposition de l'étape (a).

16. Méthode selon la revendication 15, dans laquelle ledit échantillon est choisi dans le groupe constitué d'un échantillon biologique, d'un échantillon médical ou d'un échantillon environnemental.

17. Méthode selon la revendication 15 ou 16, dans laquelle ladite réaction imnnmologique comprend un titre augmenté d'immunoglobulines, une libération d'histamine accrue, une libération de leucotriène ou dans laquelle ladite formation d'un complexe immunologique est la formation d'un complexe anticorps-antigène entre l'un ou l'autre des polypeptides selon la revendication 5, ou 6, et un anticorps ou l'anticorps selon la revendication 7 et un antigène correspondant.

18. Méthode selon la revendication 17, dans laquelle un titre augmenté d'immunoglobulines et/ou une libération d'histamine accrue est une indication pour une réaction immunologique ou allergique.

19. Méthode selon la revendication 17 ou 18, dans laquelle ladite immunoglobuline est IgE.

20. Méthode selon la revendication 18 ou 19, dans laquelle ladite réaction immunologique ou allergique est une réaction à Caviidae.

21. Méthode pour l'identification de molécules qui sont capables d'interagir avec les polypeptides selon la revendication 5, ou 6, comprenant:
(a) la production de cellules qui expriment ledit polypeptide;
(b) la mise en contact du polypeptide produit dans l'étape (a) avec un échantillon d'essai contenant potentiellement ladite molécule; et
(c) l'identification parmi ces molécules des molécules qui sont capables d'interagir avec ledit polypeptide.


### SEQUENCE LISTING

<110> Laboratory of Immunogenetics and Allergology CRP-Sante
<120> Novel Caviidae allergens and uses thereof
<130> P2267 PCT S3
<160> 42
<170> PatentIn version 3.4
<210> 1
   <211> 595
   <212> DNA
   <213> Guinea pig - Cav p3 (cDNA)
<400> 1
   acgcggcgga gctcccgtct cctctggact tctgaagagg aaaggcagct cccacaagat      60
   gcagattctc ctgcttgctc ttacaatcgg gctcgcgtac gcccatcaga ccttggaccc     120
   ttcggagatc aatggccaat ggcataccat ctccatagct gctgacaatg tggagaagat     180
   cggagagggt ggacccctga gaggctattt ccacaatcta cattgttatg atgggtgcaa     240
   aaacatcggc ctcacatttt atgtcaagtt ggatggcaac tgccaaaggt ttgatgtgct     300
   tggtgcaaag caggaagact cagatgttta cgtcgctcag tactcgggca caaaccattt     360
   tgaagtgatt ggtaagaagg aggatgccat agcattctat aaccacaata cagatgagac     420
   ggggaaggaa acaaaaatga tcgtagttgt tgcaagaaga gacagcctca cagaggagga     480
   acagcagaag ctgcaggagg tggctgggga aaagggcatt ccaaaggata acatccgcta     540
   cttcagagaa agagatactt gtgcacagta aagatgttgc ttgataggct gtttg          595
<210> 2
   <211> 170
   <212> PRT
   <213> Guinea pig - Cav p3 (Protein)
<400> 2
   Met Gln Ile Leu Leu Leu Ala Leu Thr Ile Gly Leu Ala Tyr Ala His
   1               5                   10                  15
   Gln Thr Leu Asp Pro Ser Glu Ile Asn Gly Gln Trp His Thr Ile Ser
               20                  25                  30
   Ile Ala Ala Asp Asn Val Glu Lys Ile Gly Glu Gly Gly Pro Leu Arg
           35                  40                  45
   Gly Tyr Phe His Asn Leu His Cys Tyr Asp Gly Cys Lys Asn Ile Gly
       50                  55                  60
   Leu Thr Phe Tyr Val Lys Leu Asp Gly Asn Cys Gln Arg Phe Asp Val
   65                  70                  75                  80
   Leu Gly Ala Lys Gln Glu Asp Ser Asp Val Tyr Val Ala Gln Tyr Ser
                   85                  90                  95
   Gly Thr Asn His Phe Glu Val Ile Gly Lys Lys Glu Asp Ala Ile Ala
               100                 105                 110
   Phe Tyr Asn His Asn Thr Asp Glu Thr Gly Lys Glu Thr Lys Met Ile
           115                 120                 125
   Val Val Val Ala Arg Arg Asp Ser Leu Thr Glu Glu Glu Gln Gln Lys
       130                 135                 140
   Leu Gln Glu Val Ala Gly Glu Lys Gly Ile Pro Lys Asp Asn Ile Arg
   145                 150                 155                 160
   Tyr Phe Arg Glu Arg Asp Thr Cys Ala Gln
                   165                 170
<210> 3
   <211> 926
   <212> DNA
   <213> Guinea pig - cDNA of Cav p6
<400> 3
   ggggtataaa gccagaggdt ctgaggagag agccattgct ggggaaaggc tcatccaggg      60
   agctgtggga gacatcttat ccactgccaa gatgaagctg cagctgctct gtctggggct     120
   gatactgttg tgcacccaag gggaaggcga tgaggttgtg agaggcaact ttgatgcgga     180
   aaagatttca gggaattggt atactgttaa agaggcctcc gacaaaaggg aaacaataga     240
   ggaaggtggt agcatgaggg tgtttgtgga aagcattgaa cccgtgaagg actctgcctt     300
   atcctttaaa ttctgggcac atgaaaatgg agaatgtaag caaatcagtc tcatttgcaa     360
   cagagtagca gaaggtgtat atgctgttga atatgatgga tacaatgttt ttcgcgtagc     420
   tgagacagac tataaaaact atgcaatatt tcaactcagg aatttcaagg ctgagggaag     480
   ctttcaactc ctggagctct atggccgaga accagatgtg agtggagaaa tcaagacaag     540
   gtttgaagac ttttgtcata aaaatggaat tggtgaggga aacataatag acatgaccac     600
   cgttgatcgc tgtctccagg ccagaggtga aaagtaacct aagcctccag tgctagtgag     660
   tgcttccctg cctggattct gcagcatctc ttcttcaact ctgccacctc atgcaagttc     720
   agcggcctga gaaccatcgt gctcacatat ggaagtgtca tcccctgcat ctccagwtct     780
   tctckkttaa tygtctgagg atcaatttgc ctagaagcaa agctttcctc tagatttctc     840
   ttgattccca tagctatgga aactgtatac caaataaaat ctttctctac tgttcaataa     900
   aaaaaaaaaa aaaaaaaaaa aaaaaa                                          926
<210> 4
   <211> 181
   <212> PRT
   <213> Guinea pig - Protein Cav p6
<400> 4
   Met Lys Leu Gln Leu Leu Cys Leu Gly Leu Ile Leu Leu Cys Thr Gln
   1               5                   10                  15
   Gly Glu Gly Asp Glu Val Val Arg Gly Asn Phe Asp Ala Glu Lys Ile
               20                  25                  30
   Ser Gly Asn Trp Tyr Thr Val Lys Glu Ala Ser Asp Lys Arg Glu Thr
           35                  40                  45
   Ile Glu Glu Gly Gly Ser Met Arg Val Phe Val Glu Ser Ile Glu Pro
       50                  55                  60
   Val Lys Asp Ser Ala Leu Ser Phe Lys Phe Trp Ala His Glu Asn Gly
   65                  70                  75                  80
   Glu Cys Lys Gln Ile Ser Leu Ile Cys Asn Arg Val Ala Glu Gly Val
                   85                  90                  95
   Tyr Ala Val Glu Tyr Asp Gly Tyr Asn Val Phe Arg Val Ala Glu Thr
               100                 105                 110
   Asp Tyr Lys Asn Tyr Ala Ile Phe Gln Leu Arg Asn Phe Lys Ala Glu
           115                 120                 125
   Gly Ser Phe Gln Leu Leu Glu Leu Tyr Gly Arg Glu Pro Asp Val Ser
       130                 135                 140
   Gly Glu Ile Lys Thr Arg Phe Glu Asp Phe Cys His Lys Asn Gly Ile
   145                 150                 155                 160
   Gly Glu Gly Asn Ile Ile Asp Met Thr Thr Val Asp Arg Cys Leu Gln
                   165                 170                 175
   Ala Arg Gly Glu Lys
               180
<210> 5
   <211> 712
   <212> DNA
   <213> Guinea pig - cDNA of Cav p2
<400> 5
   cctcagcagc tcacctgtcc tgcactcctg ccagaggaaa ggccgctcca gcatgatgca     60
   gattctcctc ctcgcgcttg ctgtcagcct ggcgtgtgct gactcaatcg actattcaaa    120
   ggtccccggg aattggcgca ccatcgccat agcagctgat catgtagaga agattgaagt    180
   gaatggggaa ctgagagcct atttccgtca ggtggactgt acagagggat gcgacaagat     240
   atccattacg ttttacacaa ataccgatgg ggtgtgtaca gagcacacag ttgttggcgc     300
   aagaaatggt gaaaatgatg tttacactgt ggactacgct ggtgaaaaca ctttccaaat     360
   tctttgcaac agtgatgatg ccttcgtaat aggtagtgtc aacacggatc aaaatggaca     420
   gacaacaaaa gaagttgcaa tcgctgccaa aagaaacttc ctgacacccg aacaagaaca     480
   gaagtttcag aaggcggttc agaatgcggg tatccccctg gaaaacatca gatacgtcat     540
   cgagacagac acctgcccag attaaagctc tcacttggat gaaaacaagc aatgtgtact     600
   gacgagagtc ttctccacga tgtccgaatc atcgatgtgt ggatccaatc atctgtgctt     660
   tccaattgta ctttcttctt tcctatgaaa tcatcagcca ataggctgat ct             712
<210> 6
   <211> 170
   <212> PRT
   <213> Guinea pig - Cav p2
<400> 6
   Met Met Gln Ile Leu Leu Leu Ala Leu Ala Val Ser Leu Ala Cys Ala
   1               5                   10                  15
   Asp Ser Ile Asp Tyr Ser Lys Val Pro Gly Asn Trp Arg Thr Ile Ala
               20                  25                  30
   Ile Ala Ala Asp His Val Glu Lys Ile Glu Val Asn Gly Glu Leu Arg
           35                  40                  45
   Ala Tyr Phe Arg Gln Val Asp Cys Thr Glu Gly Cys Asp Lys Ile Ser
       50                  55                  60
   Ile Thr Phe Tyr Thr Asn Thr Asp Gly Val Cys Thr Glu His Thr Val
   65                  70                  75                  80
   Val Gly Ala Arg Asn Gly Glu Asn Asp Val Tyr Thr Val Asp Tyr Ala
                   85                  90                  95
   Gly Glu Asn Thr Phe Gln Ile Leu Cys Asn Ser Asp Asp Ala Phe Val
               100                 105                 110
   Ile Gly Ser Val Asn Thr Asp Gln Asn Gly Gln Thr Thr Lys Glu Val
           115                 120                 125
   Ala Ile Ala Ala Lys Arg Asn Phe Leu Thr Pro Glu Gln Glu Gln Lys
       130                 135                 140
   Phe Gln Lys Ala Val Gln Asn Ala Gly Ile Pro Leu Glu Asn Ile Arg
   145                 150                 155                 160
   Tyr Val Ile Glu Thr Asp Thr Cys Pro Asp
                   165                 170
<210> 7
   <211> 20
   <212> DNA
   <213> Artificial - Cav p2a
<220>
   <221> source
   <223> /note="description of artificial sequence:primer"
<220>
   <221> misc_feature
   <222> (3)..(3)
   <223> r is g or a
<220>
   <221> misc_feature
   <222> (6)..(6)
   <223> n is a, c, g, or t
<220>
   <221> misc_feature
   <222> (9)..(9)
   <223> n is a, c, g, or t
<220>
   <221> misc_feature
   <222> (12)..(12)
   <223> n is a, c, g, or t
<400> 7
   aargtnccng gnaactggcg 20
<210> 8
   <211> 20
   <212> DNA
   <213> Artificial - Cav p2b
<220>
   <221> source
   <223> /note="description of artificial sequence:primer"
<220>
   <221> misc_feature
   <222> (3)..(3)
   <223> r is g or a
<220>
   <221> misc_feature
   <222> (6)..(6)
   <223> n is a, c, g, or t
<220>
   <221> misc_feature
   <222> (9)..(9)
   <223> n is a, c, g, or t
<220>
   <221> misc_feature
   <222> (12)..(12)
   <223> n is a, c, g, or t
<400> 8
   aargtnccng gnaattggcg 20
<210> 9
   <211> 20
   <212> DNA
   <213> Artificial - Cav p2c
<220>
   <221> source
   <223> /note="description of artificial sequence:primer"
<220>
   <221> misc_feature
   <222> (3)..(3)
   <223> r is g or a
<220>
   <221> misc_feature
   <222> (6)..(6)
   <223> n is a, c, g, or t
<220>
   <221> misc_feature
   <222> (9)..(9)
   <223> n is a, c, g, or t
<220>
   <221> misc_feature
   <222> (12)..(12)
   <223> n is a, c, g, or t
<400> 9
   aargtnccng gnaactggag 20
<210> 10
   <211> 18
   <212> DNA
   <213> Artificial - Cav p2d
<220>
   <221> source
   <223> /note="description of artificial sequence:primer"
<220>
   <221> misc_feature
   <222> (3)..(3)
   <223> r is g or a
<220>
   <221> misc_feature
   <222> (6)..(6)
   <223> n is a, c, g, or t
<220>
   <221> misc_feature
   <222> (9)..(9)
   <223> n is a, c, g, or t
<220>
   <221> misc_feature
   <222> (12)..(12)
   <223> n is a, c, g, or t
<400> 10
   aargtnccng gnaattggag 20
<210> 11
   <211> 25
   <212> DNA
   <213> Artificial - Cav p2rev
<220>
   <221> source
   <223> /note="description of artificial sequence:primer"
<400> 11
   gtgttttcac cagcgtagtc cacag 25
<210> 12
   <211> 23
   <212> DNA
   <213> Artificial - Cav p1e
<220>
   <221> source
   <223> /note="description of artificial sequence:primer"
<220>
   <221> misc_feature
   <222> (3)..(3)
   <223> r is g or a
<220>
   <221> misc_feature
   <222> (6)..(6)
   <223> h is a or c or t/u
<220>
   <221> misc_feature
   <222> (9)..(9)
   <223> y is t/u or c
<220>
   <221> misc_feature
   <222> (12)..(12)
   <223> n is a, c, g, or t
<400> 12
   garathaayg gncagtggaa cac 23
<210> 13
   <211> 23
   <212> DNA
   <213> Artificial - Cav p1f
<220>
   <221> source
   <223> /note="description of artificial sequence:primer"
<220>
   <221> misc_feature
   <222> (3)..(3)
   <223> r is g or a
<220>
   <221> misc_feature
   <222> (6)..(6)
   <223> h is a or c or t/u
<220>
   <221> misc_feature
   <222> (9)..(9)
   <223> y is t/u or c
<220>
   <221> misc_feature
   <222> (12)..(12)
   <223> n is a, c, g, or t
<400> 13
   garathaayg gncagtggaa tac 23
<210> 14
   <211> 23
   <212> DNA
   <213> Artificial - Cav p1g
<220>
   <221> source
   <223> /note="description of artificial sequence:primer"
<220>
   <221> misc_feature
   <222> (3)..(3)
   <223> r is g or a
<220>
   <221> misc_feature
   <222> (6)..(6)
   <223> h is a or c or t/u
<220>
   <221> misc_feature
   <222> (9)..(9)
   <223> y is t/u or c
<220>
   <221> misc_feature
   <222> (12)..(12)
   <223> n is a, c, g, or t
<400> 14
   garathaayg gncaatggaa cac 23
<210> 15
   <211> 23
   <212> DNA
   <213> Artificial - Cav p1h
<220>
   <221> source
   <223> /note="description of artificial sequence:primer"
<220>
   <221> misc_feature
   <222> (3)..(3)
   <223> r is g or a
<220>
   <221> misc_feature
   <222> (6)..(6)
   <223> h is a or c or t/u
<220>
   <221> misc_feature
   <222> (9)..(9)
   <223> y is t/u or c
<220>
   <221> misc_feature
   <222> (12)..(12)
   <223> n is a, c, g, or t
<400> 15
   garathaayg gncaatggaa tac 23
<210> 16
   <211> 25
   <212> DNA
   <213> Artificial - Cav p3rev
<220>
   <221> source
   <223> /note="description of artificial sequence:primer"
<400> 16
   gcccttttcc ccagccacct cctgc 25
<210> 17
   <211> 24
   <212> DNA
   <213> Artificial - Cav p6c
<220>
   <221> source
   <223> /note="description of artificial sequence:primer"
<220>
   <221> misc_feature
   <222> (3)..(3)
   <223> r is g or a
<220>
   <221> misc_feature
   <222> (15)..(15)
   <223> v is a or g or c
<220>
   <221> misc_feature
   <222> (18)..(18)
   <223> y is t/u or c
<400> 17
   garaagattt crggvaaytg gtat 24
<210> 18
   <211> 22
   <212> DNA
   <213> Artificial - Cav p6-5RACE-2
<220>
   <221> source
   <223> /note="description of artificial sequence:primer"
<400> 18
   ctggttctcg gccatagagc tc 22
<210> 19
   <211> 809
   <212> DNA
   <213> Guinea pig - cDNA of Cavia porcellus allergen 1a
<400> 19
   agccgctcac ctgcccttca cttctgccag aggaaaggcc gctccagctc catcatggtg      60
   cagattctcc tcctcgctct tgcagtgggt ctgtcatgtg ttgagtcttc acagatcagt     120
   ggagactggg acacaattgc tctgtctgct gacaacaaag agaagatcga agaggggggt      180
   cccctgagag tctatttccg tcaaatagac tgtaacgcgg attgctctga gattaccttc     240
   aggctttatg tcaaattaaa tggggagtgc aaagagagca cagtggttgc ttcacagagt     300
   ctaggcggtt tgtacactgt acaattcgcg ggccaaaaca ccttcgtaat tgttgataag     360
   caagaggata cgatcacatt ttttaacaca aacgtggatg agaacggact ggtgacacgg     420
   ggctatgtag tcgttggaaa aagagactct ctgacacccg aagaaacgct ctcctttgag     480
   gaggccaatg aagtaaaggg tattccccag gaaaacatag aatacctcgc tggaacagat     540
   gattgtccag agtgatgcta ttgattcgat ggcaacctgt ctggagtatt cacggtagca     600
   cttctccacg aattcttaat catgaacacc aagatccaac tttctttcct gttcagttgt     660
   actttttatt tggccaaaag tcctgaacat cttccctgtc cttcttttac atctctgcta     720
   caattcactc gaacttttgt atcccaatta attcaataaa ttgattgatg tcatctgaaa     780
   cctaaaaaaa aaaaaaaaaa aaaaaaaaa                                       809
<210> 20
   <211> 166
   <212> PRT
   <213> Guinea pig - protein Cavia porcellus allergen 1a
<400> 20
   Met Val Gln Ile Leu Leu Leu Ala Leu Ala Val Gly Leu Ser Cys Val
   1               5                   10                  15
   Glu Ser Ser Gln Ile Ser Gly Asp Trp Asp Thr Ile Ala Leu Ser Ala
               20                  25                  30
   Asp Asn Lys Glu Lys Ile Glu Glu Gly Gly Pro Leu Arg Val Tyr Phe
           35                  40                  45
   Arg Gln Ile Asp Cys Asn Ala Asp Cys Ser Glu Ile Thr Phe Arg Leu
       50                  55                  60
   Tyr Val Lys Leu Asn Gly Glu Cys Lys Glu Ser Thr Val Val Ala Ser
   65                  70                  75                  80
   Gln Ser Leu Gly Gly Leu Tyr Thr Val Gln Phe Ala Gly Gln Asn Thr
                   85                  90                  95
   Phe Val Ile Val Asp Lys Gln Glu Asp Thr Ile Thr Phe Phe Asn Thr
               100                 105                 110
   Asn Val Asp Glu Asn Gly Leu Val Thr Arg Gly Tyr Val Val Val Gly
           115                 120                 125
   Lys Arg Asp Ser Leu Thr Pro Glu Glu Thr Leu Ser Phe Glu Glu Ala
       130                 135                 140
   Asn Glu Val Lys Gly Ile Pro Gln Glu Asn Ile Glu Tyr Leu Ala Gly
   145                 150                 155                 160
   Thr Asp Asp Cys Pro Glu
                   165
<210> 21
   <211> 20
   <212> DNA
   <213> Artifical - Cavp-MQ1
<220>
   <221> source
   <223> /note="description of artificial sequence:primer"
<220>
   <221> misc_feature
   <222> (3)..(3)
   <223> n is a, c, g, or t
<400> 21
   ggngaytggr acacratcgc 20
<210> 22
   <211> 20
   <212> DNA
   <213> Artifical - Cavp-MQ2
<220>
   <221> source
   <223> /note="description of artificial sequence:primer"
<220>
   <221> misc_feature
   <222> (3)..(3)
   <223> n is a, c, g, or t
<400> 22
   ggngaytggr acacrattgc 20
<210> 23
   <211> 20
   <212> DNA
   <213> Artifical - Cavp-MQ3
<220>
   <221> source
   <223> /note="description of artificial sequence:primer"
<220>
   <221> misc_feature
   <222> (3)..(3)
   <223> n is a, c, g, or t
<400> 23
   ggngaytggr acacratagc 20
<210> 24
   <211> 20
   <212> DNA
   <213> Artifical - Cavp-MQ4
<220>
   <221> source
   <223> /note="description of artificial sequence:primer"
<220>
   <221> misc_feature
   <222> (3)..(3)
   <223> n is a, c, g, or t
<400> 24
   ggngaytggr acacyatcgc 20
<210> 25
   <211> 20
   <212> DNA
   <213> Artifical - Cavp-MQ5
<220>
   <221> source
   <223> /note="description of artificial sequence:primer"
<220>
   <221> misc_feature
   <222> (3)..(3)
   <223> n is a, c, g, or t
<400> 25
   ggngaytggr acacyattgc 20
<210> 26
   <211> 20
   <212> DNA
   <213> Artifical - Cavp-MQ6
<220>
   <221> source
   <223> /note="description of artificial sequence:primer"
<220>
   <221> misc_feature
   <222> (3)..(3)
   <223> n is a, c, g, or t
<400> 26
   ggngaytggr acacyatagc 20
<210> 27
   <211> 20
   <212> DNA
   <213> Artifical - Cavp-MQ7
<220>
   <221> source
   <223> /note="description of artificial sequence:primer"
<220>
   <221> misc_feature
   <222> (3)..(3)
   <223> n is a, c, g, or t
<400> 27
   ggngaytggr atacratcgc 20
<210> 28
   <211> 20
   <212> DNA
   <213> Artifical - Cavp-MQ8
<220>
   <221> source
   <223> /note="description of artificial sequence:primer"
<220>
   <221> misc_feature
   <222> (3)..(3)
   <223> n is a, c, g, or t
<400> 28
   ggngaytggr atacrattgc 20
<210> 29
   <211> 20
   <212> DNA
   <213> Artifical - Cavp-MQ9
<220>
   <221> source
   <223> /note="description of artificial sequence:primer"
<220>
   <221> misc_feature
   <222> (3)..(3)
   <223> n is a, c, g, or t
<400> 29
   ggngaytggr atacratagc 20
<210> 30
   <211> 20
   <212> DNA
   <213> Artifical - Cavp-MQ10
<220>
   <221> source
   <223> /note="description of artificial sequence:primer"
<220>
   <221> misc_feature
   <222> (3)..(3)
   <223> n is a, c, g, or t
<400> 30
   ggngaytggr atacyatcgc 20
<210> 31
   <211> 20
   <212> DNA
   <213> Artifical - Cavp-MQ11
<220>
   <221> source
   <223> /note="description of artificial sequence:primer"
<220>
   <221> misc_feature
   <222> (3)..(3)
   <223> n is a, c, g, or t
<400> 31
   ggngaytggr atacyattgc 20
<210> 32
   <211> 20
   <212> DNA
   <213> Artifical - Cavp-MQ12
<220>
   <221> source
   <223> /note="description of artificial sequence:primer"
<220>
   <221> misc_feature
   <222> (3)..(3)
   <223> n is a, c, g, or t
<400> 32
   ggngaytggr atacyatagc 20
<210> 33
   <211> 20
   <212> DNA
   <213> Artifical - Cavp-MQ1rev
<220>
   <221> source
   <223> /note="description of artificial sequence:primer"
<400> 33
   ttrtcrtcda tytgtctaaa 20
<210> 34
   <211> 20
   <212> DNA
   <213> Artifical - Cavp-MQ2rev
<220>
   <221> source
   <223> /note="description of artificial sequence:primer"
<400> 34
   ttrtcrtcda tytgcctaaa 20
<210> 35
   <211> 20
   <212> DNA
   <213> Artifical - Cavp-MQ3rev
<220>
   <221> source
   <223> /note="description of artificial sequence:primer"
<400> 35
   ttrtcrtcda tytgtctgaa 20
<210> 36
   <211> 20
   <212> DNA
   <213> Artifical - Cavp-MQ4rev
<220>
   <221> source
   <223> /note="description of artificial sequence:primer"
<400> 36
   ttrtcrtcda tytgcctgaa 20
<210> 37
   <211> 20
   <212> DNA
   <213> Artifical - Cavp-MQ5rev
<220>
   <221> source
   <223> /note="description of artificial sequence:primer"
<400> 37
   ttrtcrtcda tytgrcgaaa 20
<210> 38
   <211> 20
   <212> DNA
   <213> Artifical - Cavp-MQ6rev
<220>
   <221> source
   <223> /note="description of artificial sequence:primer"
<400> 38
   ttrtcrtcda tytgycgaaa 20
<210> 39
   <211> 20
   <212> DNA
   <213> Artifical - Cavp-MQ7rev
<220>
   <221> source
   <223> /note="description of artificial sequence:primer"
<400> 39
   ttrtcrtcda tytgrcggaa 20
<210> 40
   <211> 20
   <212> DNA
   <213> Artifical - Cavp-MQ8rev
<220>
   <221> source
   <223> /note="description of artificial sequence:primer"
<400> 40
   ttrtcrtcda tytgycggaa 20
<210> 41
   <211> 36
   <212> DNA
   <213> Artificial - Cavp-11_for (3'RACE)
<220>
   <221> source
   <223> /note="description of artificial sequence:primer"
<400> 41
   ctctgtctgc tgacaacaaa gagragatcg aagagg 36
<210> 42
   <211> 26
   <212> DNA
   <213> Artificial - Cavp-b_rev (5'RACE)
<220>
   <221> source
   <223> /note="description of artificial sequence:primer"
<400> 42
   cgtttcttcg ggtgtcagag agtctc 26